# EUROPEAN PATENT APPLICATION

(11) **EP 4 324 835 A1**
(43) Date of publication of application: **21.02.2024**
(21) Application number: 22798642.9
(22) Date of filing: 05.05.2022
(51) Int. Cl.: C07D 498/22, C07D 487/18, C07D 487/14, C07D 471/14, C07D 498/14, A61K 31/529, A61K 31/519, A61K 31/5383, A61P 3/10, A61P 25/00, A61P 9/10, A61P 13/12, A61P 7/10, A61P 25/04, A61P 25/28, A61P 25/16, A61P 21/00

(54) **PYRIMIDINONE DERIVATIVE AND PREPARATION METHOD THEREFOR, PHARMACEUTICAL COMPOSITION, AND USE**

(30) Priority: 07.05.2021 CN 202110494141
(71) Applicant: Shanghai Simr Biotechnology Co., Ltd., Shanghai 201318 (CN); Shanghai Simrd Biotechnology Co., Ltd., Shanghai 200120 (CN)
(72) Inventor: JIN, Yun, Shanghai 201318 (CN); PENG, Jun, Shanghai 201318 (CN)
(74) Representative: J A Kemp LLP
(86) International application number: PCT/CN2022/090984
(87) International publication number: WO 2022/233302

(57) **Abstract**

The present invention provides a compound as represented by general formula (I), a cis-trans isomer thereof, an enantiomer thereof, a diastereomer thereof, a racemate thereof, a solvate thereof, a hydrate thereof, or a pharmaceutically-acceptable salt thereof or a prodrug thereof, a preparation method therefor, a pharmaceutical composition containing the compound, and a use of the compound as an Lp-PLA₂ inhibitor, wherein R₁, R₂, Rₓ, R_{y}, R_{z}, Q, U, X, m, n, and A are defined in the description.

## Description

### TECHNICAL FIELD

The present invention relates to a novel pyrimidinone compound, a preparation method therefor, a pharmaceutical composition containing the compound and a use thereof in treatment of Lp-PLA₂-mediated diseases.

### BACKGROUND

Lipoprotein-associated phospholipase A₂ (Lp-PLA₂) is phospholipase A₂ participating in hydrolysis of lipoprotein lipid or phospholipid, and is also called platelet activating factor acetylhydrolase (PAF-AH). Lp-PLA₂ moves with low-density lipoprotein (LDL) and rapidly cleaves oxidized phosphatidylcholine molecules obtained from LDL oxidation. Lp-PLA₂ hydrolyzes sn-2 ester of oxidized phosphatidylcholine to obtain lipid-mediated lysophosphatidylcholine (lysoPC) and oxidized non-esterified fatty acid (NEFA). Literature reported that lysoPC and NEFA could cause inflammatory reactions, and thus Lp-PLA₂ mediated oxidative inflammatory reactions in vivo. (Zalewski A et al., Arterioscler. Thromb. Vasc.Biol., 25, 5, 923-31 (2005))

Literature (WO96/13484, WO96/19451, WO97/02242, WO97/12963, WO97/21675, WO97/21676, WO97/41098, WO97/41099, WO99/2442, WO00/10980, WO00/66566, WO00/66567, WO00/68208, WO01/60805, WO02/30904, WO02/30911, WO03/015786, WO03/016287, WO03/041712, WO03/042179, WO03/042206, WO03/042218, WO03/086400, WO03/87088, WO08/04886, US2008/0103156, US2008/0090851, US2008/0090852, WO08/048866, WO05/003118, WO06/063811, WO06/063813, WO2008/141176, WO2013013503A1, WO2013014185A1, WO2014114248A1, WO2014114694A1, WO2016011930A1, JP200188847US2008/0279846A1, US 2010/0239565A1, US 2008/0280829A1) described many Lp-PLA₂ inhibitors and/or their uses, treating diseases related to vascular endothelial dysfunction or related diseases, diseases related to lipid oxidation associated with Lp-PLA₂ activity (such as those related to the formation of lysophosphatidylcholine and oxidized free fatty acid), and diseases related to activated monocytes, macrophages or lymphocytes or increased involvement of monocytes, macrophages or lymphocytes. Examples of specific diseases include neurodegenerative diseases (such as Alzheimer's disease, Parkinson's disease, Huntington's disease, and vascular dementia), various neuropsychiatric diseases such as schizophrenia and autism, peripheral and cerebral atherosclerosis, stroke, metabolic bone diseases (such as bone marrow abnormalities), dyslipidemia, Paget's disease, type II diabetes, hypertension, angina pectoris, myocardial infarction, ischemia, reperfusion injury, metabolic syndrome, insulin resistance and hyperparathyroidism, diabetic complications (such as macular edema, diabetic retinopathy and posterior uveitis, diabetic ulcer and diabetic nephropathy), diabetic peripheral neuropathic pain, inflammatory pain, neuropathic pain, various types of cancer (such as prostate cancer, colon cancer, breast cancer, kidney cancer, lung cancer and ovarian cancer), macular edema, wound healing, male erectile dysfunction, rheumatoid arthritis, chronic obstructive pulmonary disease (COPD), sepsis, acute and chronic inflammation, psoriasis, and multiple sclerosis.

Scientific research results further prove that Lp-PLA₂ inhibitors can be used to treat atherosclerosis. Wilensky et al. demonstrated the effect of the Lp-PLA₂ inhibitors on atherosclerotic plaque components in diabetes and hypercholesterolemia pig models that accelerate coronary atherosclerosis (Wilensky et al., Nature Medicine, 10, 1015-1016 (2008)). Clinical studies also found that the Lp-PLA₂ inhibitors can stabilize atherosclerotic plaque in patients with atherosclerotic plaque and prevent the plaque from further development and rupture (Serruys et al., Circulation 118:1172-1182 (2008)).

Studies showed that high Lp-PLA₂ activity is associated with the risk of high dementia (including Alzheimer's disease (AD) and mixed dementia) (Van Oijen et al., Annals of Neurology, 59, 139 (2006); Fitzpatrick et al., Atherosclerosis 235:384-391 (2014)). High levels of oxidized LDL were observed in AD patients (Kassner et al., Current Alzheimer Research, 5, 358-366 (2008); Dildar et al., Alzheimer Dis Assoc Disord, 24, April-June (2010); Sinem et al., Current Alzheimer Research, 7, 463-469 (2010)).

In addition, US2008/0279846 described that Lp-PLA₂ inhibitors reduce blood-brain barrier leakage and brain amyloid protein (Abeta) loads, which can be used to treat diseases related to blood-brain barrier leakage, such as Alzheimer's disease and vascular dementia. In clinical studies, Lp-PLA₂ inhibitors have a significant effect on preventing further deterioration of cognitive functions in Alzheimer's patients (Maher-Edwards et al., Alzheimer's & Dementia: Translational Research & Clinical Interventions 1, 131-140 (2015)).

Neuroinflammation (including the release of various cytotoxic cytokines) is a common feature of all neurodegenerative diseases (including multiple sclerosis, amyotrophic lateral sclerosis, Parkinson's disease, Alzheimer's disease, etc.) (Perry, Acta Neuropathol, 120, 277-286 (2010)). Lp-PLA₂ inhibitors reduce the release of various cytokines by inhibiting lysoPC production (Shi et al., Atherosclerosis 191, 54-62 (2007)). Therefore, inhibiting Lp-PLA₂ is a potential treatment method for neurodegenerative diseases (including multiple sclerosis, amyotrophic lateral sclerosis, Parkinson's disease, etc.).

LysoPC is also involved in leukocyte activation, inducing cell apoptosis, and mediating endothelial cell dysfunction (Wilensky et al., Current Opinion in Lipidology, 20, 415-420, (2009)). Therefore, it is believed that Lp-PLA₂ inhibitors can be used to treat tissue damage related to diabetes by reducing the production of lysoPC. High Lp-PLA₂ activity is related to the high onset risk of diabetic retinopathy (Siddiqui et al., Diabetologia, 61, 1344-1353 (2018)). The Lp-PLA₂ inhibitors can inhibit the main pathological changes of retinopathy in diabetes rat models (Canning et al., P.N.A.S. 113, 7213-7218 (2016)). Clinical studies also showed that the Lp-PLA₂ inhibitors can improve retinal macular edema symptoms and vision in patients with diabetic retinopathy (Staurenghi et al., Ophthalmology 122, 990-996 (2015)). These studies demonstrate that the Lp-PLA₂ inhibitors can be used for diabetic retinopathy.

Studies showed that the Lp-PLA₂ activity in diabetes patients is higher than that in normal people (Serban et al. J. Cell. Mol. Med. 6:643-647, (2002); Garg et al., Indian J. Med. Res. 141:107-114 (2015)). As mentioned above, the Lp-PLA₂ activity mediates the oxidative inflammatory reaction, and it is speculated that inhibiting the Lp-PLA₂ activity can be used to treat various complications caused by the oxidative inflammatory reaction in the bodies of diabetes patients, such as diabetic nephropathy, diabetic peripheral neuropathy and diabetic skin ulceration.

Glaucoma and age-related macular degeneration (AMD) are retinal neurodegenerative diseases. Inflammation plays an important role in the pathogenesis of glaucoma and AMD (Buschini et al., Progress in Neurobiology, 95, 14-25 (2011); Tezel, Progress in Brain Research, vol. 173, ISSN0079-6123, Chapter 28). Therefore, Lp-PLA₂ inhibitors can provide potential therapeutic applications for glaucoma and AMD.

In patients with male erectile dysfunction, the activity of Lp-PLA₂ in the body is significantly higher than that of normal people, and it is believed that high Lp-PLA₂ activity can predict early male erectile dysfunction (Otunctemur et al., Andrologia 47:706-710 (2015)), suggesting that Lp-PLA₂ inhibitors can be used to treat male erectile dysfunction.

Lp-PLA₂ is highly expressed in prostate cancer tissue, and reducing Lp-PLA₂ can reduce prostate cell carcinogenesis and promote prostate cancer cell apoptosis in vitro experiments (Vainio et al., Oncotarget, 2:1176-1190 (2011)), suggesting that Lp-PLA₂ inhibitors can be used to treat prostate cancer.

### SUMMARY OF THE INVENTION

An object of the present invention is to provide compounds as represented by general formulas (I), (II), (III), (IV), (V) or (VI), cis-trans isomers thereof, enantiomers thereof, diastereomers thereof, racemates thereof, solvates thereof, hydrates thereof or pharmaceutically-acceptable salts and esters thereof.

Another object of the present invention is to provide preparation methods for the compounds as represented by general formulas (I), (II), (III), (IV), (V) or (VI).

Another object of the present invention is to provide uses of the compounds as represented by general formulas (I), (II), (III), (IV), (V) or (VI) as Lp-PLA₂ inhibitors, and thus applications in the manufacture of medications for preventing, treating or relieving diseases related to Lp-PLA₂ inhibition. The diseases are, for example, diabetic complications, neuroinflammation-related diseases or/and atherosclerosis, the diabetic complications are diabetic retinopathy/diabetic macular edema, diabetic nephropathy, diabetic neuropathy, diabetic peripheral neuropathic pain or/and diabetic foot, and the neuroinflammation-related diseases are Alzheimer's disease, multiple sclerosis, amyotrophic lateral sclerosis or/and Parkinson's disease.

Another object of the present invention is to provide a pharmaceutical composition, including one or more of effective treatment dosages of the compounds as represented by general formulas (I), (II), (III), (IV), (V) or (VI) or pharmaceutically-acceptable salts thereof, as well as pharmaceutically-acceptable vehicles and/or adjuvants.

Another object of the present invention is to provide a method for preventing, treating or relieving diseases related to Lp-PLA₂ inhibition, including administrating the compounds as represented by general formulas (I), (II), (III), (IV), (V) or (VI) or the pharmaceutically-acceptable salts thereof as described in the present invention or the composition as described in the present invention.

In a first aspect, provided are a compound as represented by formula I, a cis-trans isomer thereof, an enantiomer thereof, a diastereomer thereof, a racemate thereof, a solvate thereof, a hydrate thereof or a pharmaceutically-acceptable salt thereof or a prodrug thereof, 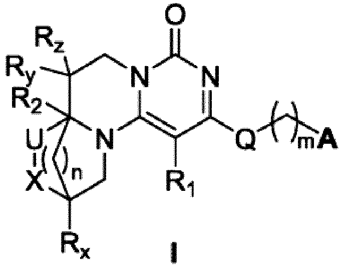 wherein
n is 0, 1 or 2, when n is 0, R₂ is H, methyl or ethyl, and when n is 1 or 2, R₂ is absent;
R₁ is H, halogen, cyano, amino, C₁₋₆ alkyl, C₁₋₆ alkoxy, C₃₋₈ cycloalkyl or 3- to 8-membered heterocyclyl, and R₁ can be substituted by one or more of the following substituents: halogen, cyano, C₁₋₆ alkoxy, C₃₋₈ cycloalkyl, 3- to 8-membered heterocyclyl or 3- to 8-membered heteroaryl;
m is 1 or 2;
Rₓ is H, halogen, hydroxyl, carboxyl, cyano, C₁₋₆ alkyl, C₁₋₆ alkoxy, C₃₋₈ cycloalkyl, 3-to 8-membered heterocyclyl, C₆₋₁₀ aryl, 3- to 8-membered heteroaryl, -C(O)NR_{b}R_{c}, or -S(O)₂NR_{b}R_{c}, and Rₓ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
R_{y} and R_{z} are independently H, halogen, cyano, hydroxyl, amino, -C(O)NR_{b}R_{c}, -S(O)₂NR_{b}R_{c}, C₁₋₆ alkyl, C₃₋₈ cycloalkyl, or 3- to 8-membered heterocyclyl, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl;
or R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ;
Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
Q is -O-, -S- or -NR_{b}-;
X is -O-, -CH₂-, -NR_{c}- or -OCH₂-;
R_{b} is H, C₁₋₆ alkyl, C₃₋₈ cycloalkyl or 3- to 8-membered heterocyclyl;
R_{c} is L, L-C(O)-, L-CH₂- or L-S(O)2-,
wherein L is H, C₁₋₆ alkyl, C₃₋₆ cycloalkyl, 3- to 8-membered heterocyclyl, aryl or 3- to 8-membered heteroaryl, and L can be substituted by one or more of the following groups: halogen, hydroxyl, C₁₋₆ alkoxy, cyano, C₃₋₈ cycloalkyl, 3- to 8-membered heterocyclyl, aryl or 3- to 8-membered heteroaryl;
U is -CH₂-, -C(O)- or absent, and U can be substituted by one or more of the following substituents: halogen, hydroxyl, C₁₋₆ alkyl, C₁₋₆ alkoxy, cyano, C₃₋₈ cycloalkyl, 3- to 8-membered heterocyclyl, aryl or 3- to 8-membered heteroaryl;
A is 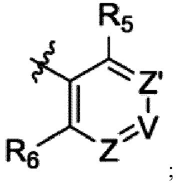
Z is N or CR₃;
Z' is N or CR₄;
R₃, R₄, R₅ and R₆ are independently H, CN, halogen, or C₁₋₃ haloalkyl;
V is N or CR₉, wherein R₉ is H, CN, halogen, C₁₋₃ alkyl, C₁₋₃ haloalkyl or -O-W; and
W is 5- or 6-membered heteroaryl or phenyl, and can be optionally substituted by one or more of the following substituents: halogen, cyano, C₁₋₆ alkyl, C₁₋₃ alkoxy, C₁₋₃ haloalkyl and C₁₋₃ haloalkoxy.

In a second aspect, provided are a compound as represented by formula II, a cis-trans isomer thereof, an enantiomer thereof, a diastereomer thereof, a racemate thereof, a solvate thereof, a hydrate thereof or a pharmaceutically-acceptable salt thereof or a prodrug thereof, 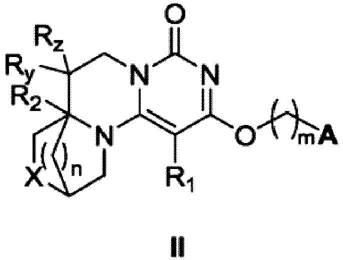 wherein
n is 0, 1 or 2, when n is 0, R₂ is H, methyl or ethyl, and when n is 1 or 2, R₂ is absent; and preferably, n is 0 or 1;
R₁ is H, halogen, cyano, C₁₋₆ alkyl or C₁₋₆ alkoxy; preferably, R₁ is H, halogen, cyano, C₁₋₃ alkyl or C₁₋₃ alkoxy; more preferably, R₁ is H, fluorine, chlorine, cyano, methyl, ethyl or methoxy; and most preferably, R₁ is H or methoxy;
m is 1 or 2; and preferably, m is 1;
X is -O-, -CH₂- or absent;
R_{y} and R_{z} are independently H, halogen, cyano, hydroxyl, amino, C₁₋₆ alkyl, C₃₋₈ cycloalkyl, or 3- to 8-membered heterocyclyl, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl; preferably, R_{y} and R_{z} are independently H, halogen, cyano, hydroxyl or amino, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl; and more preferably, R_{y} and R_{z} are independently H or halogen;
or R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ, Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
A is 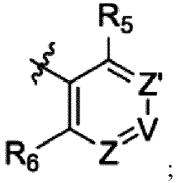
Z is N or CR₃;
Z' is N or CR₄;
R₃, R₄, R₅ and R₆ are independently H, CN, halogen, or C₁₋₃ haloalkyl;
V is N or CR₉, wherein R₉ is H, CN, halogen, C₁₋₃ alkyl, C₁₋₃ haloalkyl or -O-W;
W is 5- or 6-membered heteroaryl or phenyl, and can be optionally substituted by one or more of the following substituents: halogen, cyano, C₁₋₆ alkyl, C₁₋₃ alkoxy, C₁₋₃ haloalkyl and C₁₋₃ haloalkoxy;
preferably, A is 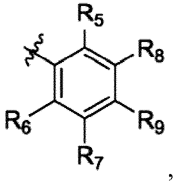
R₅, R₆, R₇, R₈ and R₉ are independently H, F or CN;
or A is 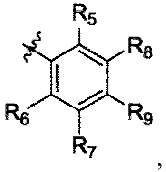
R₅, R₆, R₇ and R₈ are independently H, F or CN;
R₉ is -O-W; and
W is 5- or 6-membered heteroaryl or phenyl, and can be optionally substituted by one or more of the following substituents: C₁₋₃ haloalkyl, C₁₋₃ haloalkoxy, CN, halogen and C₁₋₆ alkyl.

More preferably, A is 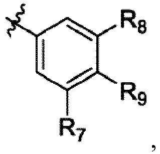
R₇ and R₈ are independently H, F or CN;
R₉ is -O-W;
W is pyridyl, pyrimidinyl, pyrazolyl, or phenyl, and can be optionally substituted by one or more substituents independently selected from: halogen, CN, CF₃, -OCF₃, CHF₂ and CH₃; and
most preferably, A is selected from the following groups:

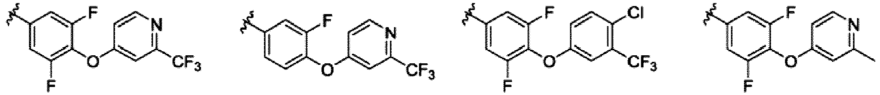

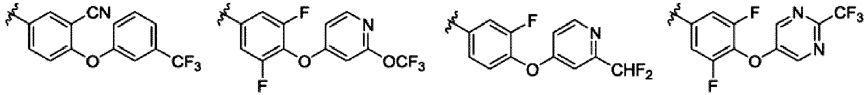

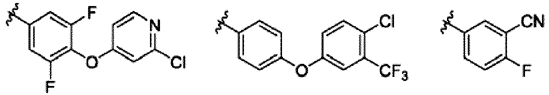

In a third aspect, provided are compounds as represented by formulas III to VI, cis-trans isomers thereof, enantiomers thereof, diastereomers thereof, racemates thereof, solvates thereof, hydrates thereof or pharmaceutically-acceptable salts thereof or prodrugs thereof 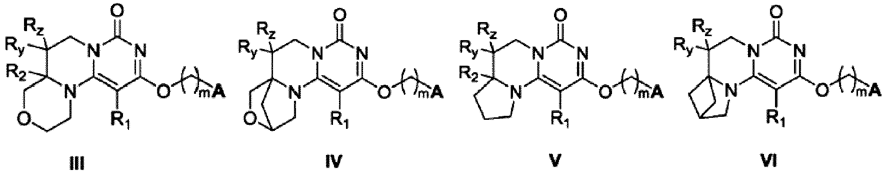 wherein
R₁ is H, halogen, cyano, C₁₋₆ alkyl or C₁₋₆ alkoxy; preferably, R₁ is H, halogen, cyano, C₁₋₃ alkyl or C₁₋₃ alkoxy; more preferably, R₁ is H, fluorine, chlorine, cyano, methyl, ethyl or methoxy; and most preferably, R₁ is H or methyl;
m is 1 or 2; and preferably, m is 1;
R_{y} and R_{z} are independently H, halogen, cyano, hydroxyl, amino, C₁₋₆ alkyl, C₃₋₈ cycloalkyl, or 3- to 8-membered heterocyclyl, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl; preferably, R_{y} and R_{z} are independently H, halogen, cyano, hydroxyl or amino, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl; and more preferably, R_{y} and R_{z} are independently H or halogen;
or R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ, Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
A is 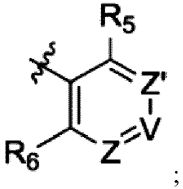
Z is N or CR₃;
Z' is N or CR₄;
R₃, R₄, R₅ and R₆ are independently H, CN, halogen, or C₁₋₃ haloalkyl;
V is N or CR₉, wherein R₉ is H, CN, halogen, C₁₋₃ alkyl, C₁₋₃ haloalkyl or -O-W;
W is 5- or 6-membered heteroaryl or phenyl, and can be optionally substituted by one or more of the following substituents: halogen, cyano, C₁₋₆ alkyl, C₁₋₃ alkoxy, C₁₋₃ haloalkyl and C₁₋₃ haloalkoxy;
preferably, A is 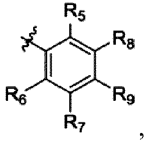
R₅, R₆, R₇, R₈ and R₉ are independently H, F or CN;
or A is 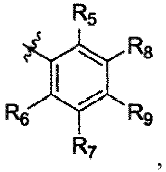
R₅, R₆, R₇ and R₈ are independently H, F or CN;
R₉ is -O-W; and
W is 5- or 6-membered heteroaryl or phenyl, and can be optionally substituted by one or more of the following substituents: C₁₋₃ haloalkyl, C₁₋₃ haloalkoxy, CN, halogen and C₁₋₆ alkyl.

More preferably, A is 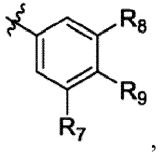
R₇ and R₈ are independently H, F or CN;
R₉ is -O-W;
W is pyridyl, pyrimidinyl, pyrazolyl, or phenyl, and can be optionally substituted by one or more substituents independently selected from: halogen, CN, CF₃, -OCF₃, CHF₂ and CH₃; and
most preferably, A is selected from the following groups:

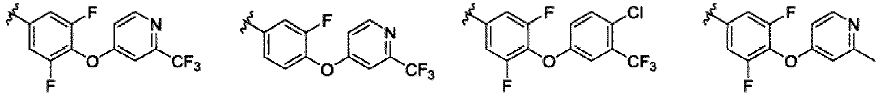

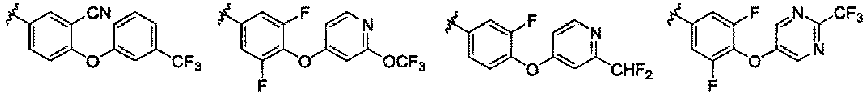

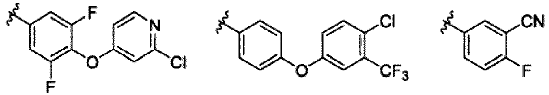

In a first preferred implementation, provided are a compound as represented by formula II, a cis-trans isomer thereof, an enantiomer thereof, a diastereomer thereof, a racemate thereof, a solvate thereof, a hydrate thereof or a pharmaceutically-acceptable salt thereof or a prodrug thereof, 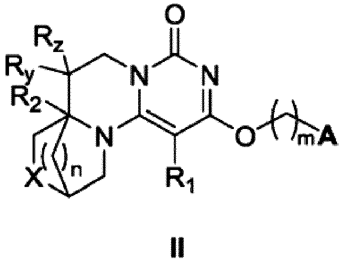 wherein
n is 0, 1 or 2, when n is 0, R₂ is H, methyl or ethyl, and when n is 1 or 2, R₂ is absent;
R₁ is H, halogen, cyano, C₁₋₆ alkyl or C₁₋₆ alkoxy;
m is 1 or 2;
X is -O-, -CH₂- or absent;
R_{y} and R_{z} are independently H, halogen, cyano, hydroxyl, amino, C₁₋₆ alkyl, C₃₋₈ cycloalkyl, or 3- to 8-membered heterocyclyl, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl;
or R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ;
Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
A is 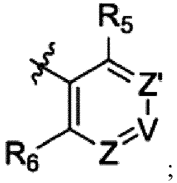
Z is N or CR₃;
Z' is N or CR₄;
R₃, R₄, R₅ and R₆ are independently H, CN, halogen, or C₁₋₃ haloalkyl;
V is N or CR₉, wherein R₉ is H, CN, halogen, C₁₋₃ alkyl, C₁₋₃ haloalkyl or -O-W; and
W is 5- or 6-membered heteroaryl or phenyl, and can be optionally substituted by one or more of the following substituents: halogen, cyano, C₁₋₆ alkyl, C₁₋₃ alkoxy, C₁₋₃ haloalkyl and C₁₋₃ haloalkoxy.

In another preferred implementation, as in the above first preferred implementation, n is 0, R₂ is H or methyl, and m is 1.

In another preferred implementation, as in the above first preferred implementation, n is 1 or 2, R₂ is absent, and m is 1.

In a second preferred implementation, provided are compounds as represented by formulas III to VI, cis-trans isomers thereof, enantiomers thereof, diastereomers thereof, racemates thereof, solvates thereof, hydrates thereof or pharmaceutically-acceptable salts thereof or prodrugs thereof, 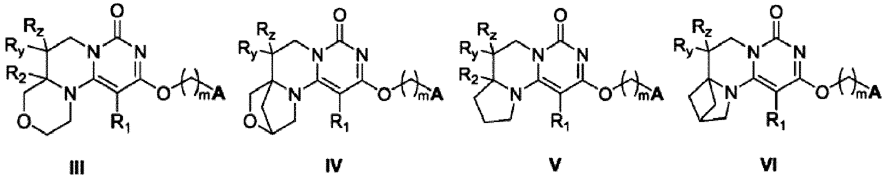
wherein R₁ is H, halogen, cyano, C₁₋₃ alkyl or C₁₋₆ alkoxy;
R₂ is H or methyl;
m is 1 or 2;
R_{y} and R_{z} are independently H, halogen, cyano, hydroxyl, amino, C₁₋₆ alkyl, C₃₋₈ cycloalkyl, or 3- to 8-membered heterocyclyl, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl;
or R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ;
Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
A is 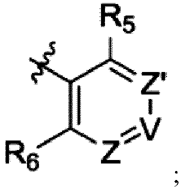
Z is N or CR₃;
Z' is N or CR₄;
R₃, R₄, R₅ and R₆ are independently H, CN, halogen, or C₁₋₃ haloalkyl;
V is N or CR₉, wherein R₉ is H, CN, halogen, C₁₋₃ alkyl, C₁₋₃ haloalkyl or -O-W; and
W is 5- or 6-membered heteroaryl or phenyl, and can be optionally substituted by one or more of the following substituents: halogen, cyano, C₁₋₆ alkyl, C₁₋₃ alkoxy, C₁₋₃ haloalkyl and C₁₋₃ haloalkoxy.

In another preferred implementation, as in the above first or second preferred implementation, m is 1, R_{y} and R_{z} are independently H, halogen, cyano, hydroxyl or amino, and R_{y} and R_{z} can be both substituted by one or more of the following substituents: halogen, hydroxyl, carboxyl, cyano, C₁₋₆ alkyl, C₆₋₁₀ aryl and 3- to 8-membered heteroaryl.

In another preferred implementation, as in the above first or second preferred implementation, m is 1, R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ; and Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl.

In another preferred implementation, as in the above first or second preferred implementation, A is 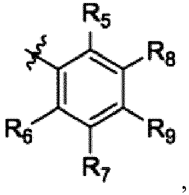 and
R₅, R₆, R₇, R₈ and R₉ are independently H, F or CN.

In another preferred implementation, as in the above first or second preferred implementation, A is 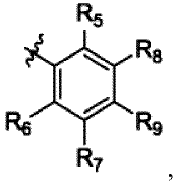
R₅, R₆, R₇ and R₈ are independently H, F or CN;
R₉ is -O-W; and
W is 5- or 6-membered heteroaryl or phenyl, and can be optionally substituted by one or more of the following substituents: C₁₋₃ haloalkyl, C₁₋₃ haloalkoxy, CN, halogen and C₁₋₆ alkyl.

In another preferred implementation, as in the above first or second preferred implementation, A is 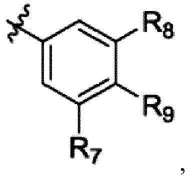
R₇ and R₈ are independently H, F or CN;
R₉ is -O-W; and
W is pyridyl, pyrimidinyl, pyrazolyl, or phenyl, and can be optionally substituted by one or more substituents independently selected from: halogen, CN, CF₃, -OCF₃, CHF₂ and CH₃.

In another preferred implementation, as in the above first or second preferred implementation, A is selected from the following groups:

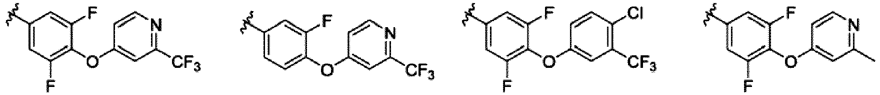

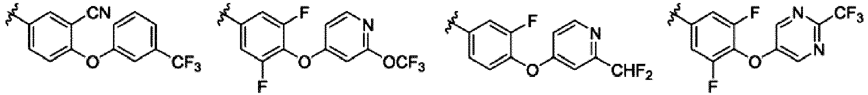

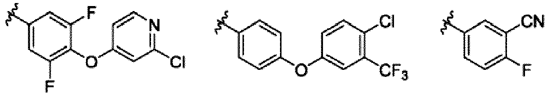

In one embodiment, the compound is any one of the following compounds:

| Example | Structure | Example | Structure |
|---|---|---|---|
| 1 | 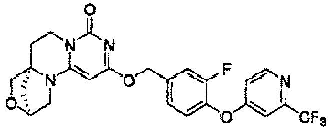 | 2 | 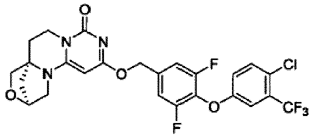 |
| 3 | 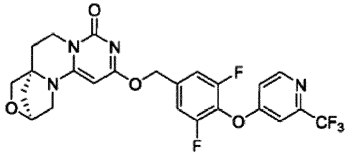 | 4 | 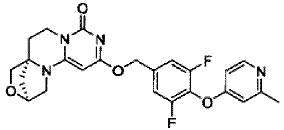 |
| 5 | 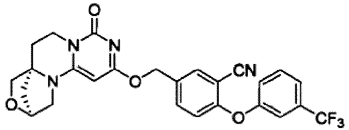 | 6 | 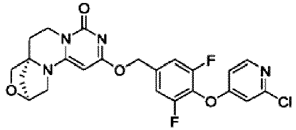 |
| 7 | 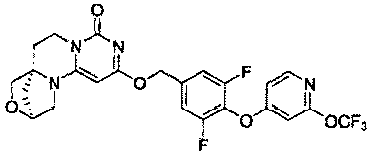 | 8 | 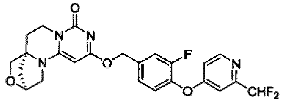 |
| 9 | 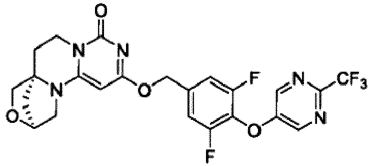 | 10 | 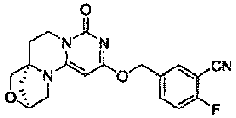 |
| 11 | 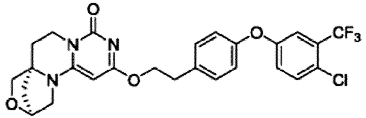 | 12 | 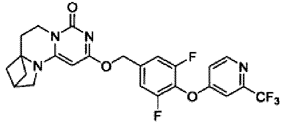 |
| 13 | 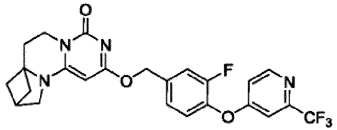 | 14 | 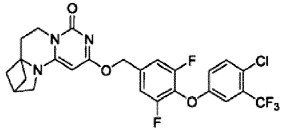 |
| 15 | 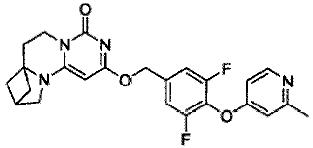 | 16 | 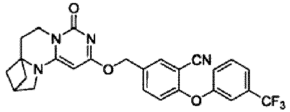 |
| 17 | 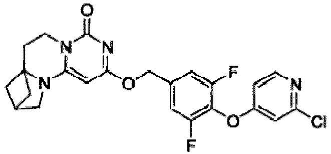 | 18 | 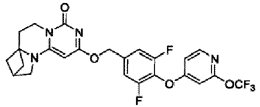 |
| 19 | 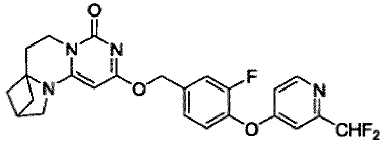 | 20 | 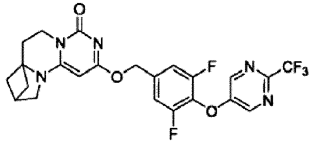 |
| 21 | 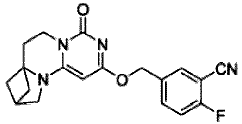 | 22 | 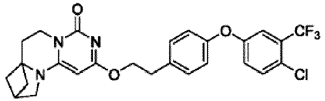 |
| 23 | 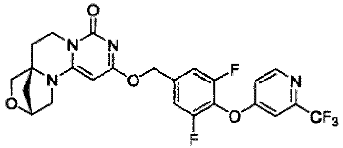 | 24 | 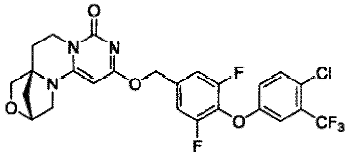 |
| 25 | 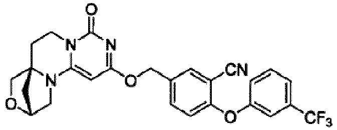 | 26 | 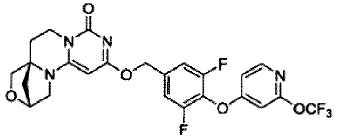 |
| 27 | 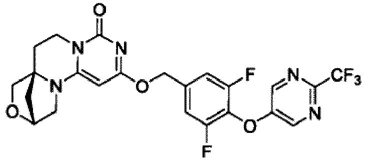 | 28 | 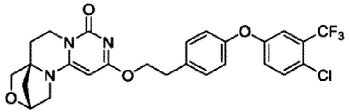 |
| 29 | 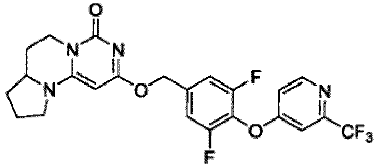 | 30 | 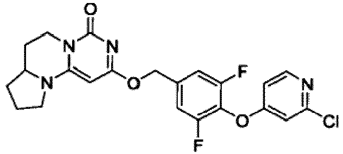 |
| 31 | 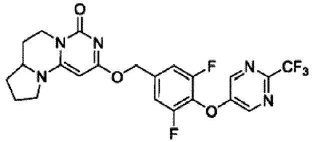 | 32 | 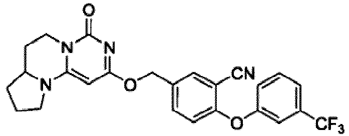 |
| 33 | 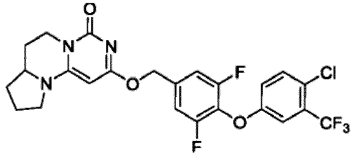 | 34 | 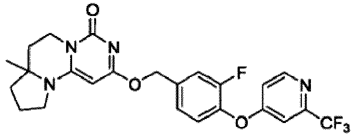 |
| 35 | 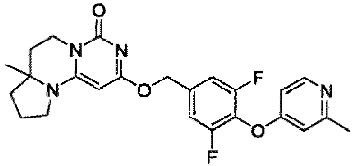 | 36 | 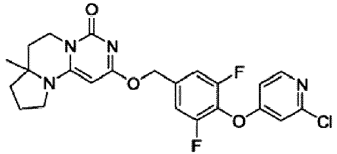 |
| 37 | 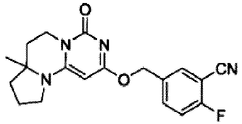 | 38 | 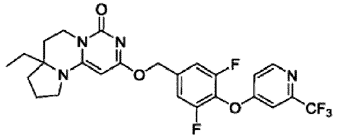 |
| 39 | 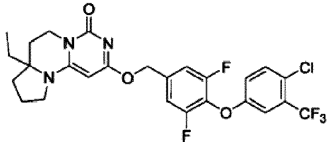 | 40 | 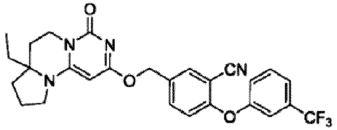 |
| 41 | 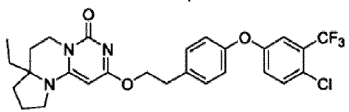 | 42 | 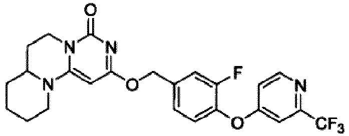 |
| 43 | 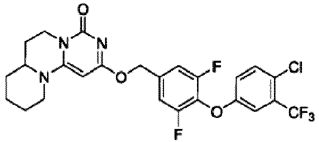 | 44 | 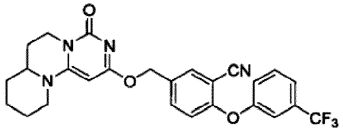 |
| 45 | 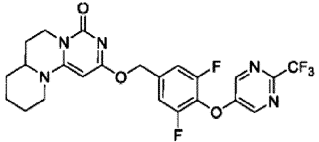 | 46 | 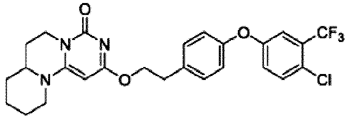 |
| 47 | 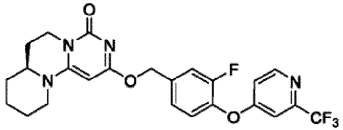 | 48 | 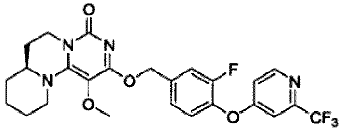 |
| 49 | 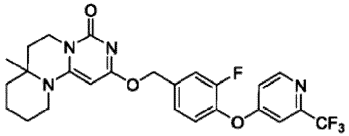 | 50 | 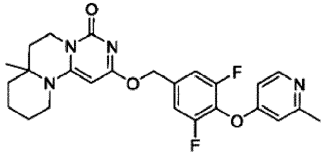 |
| 51 | 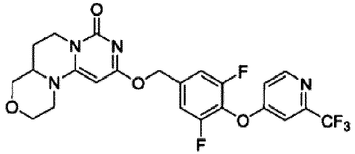 | 52 | 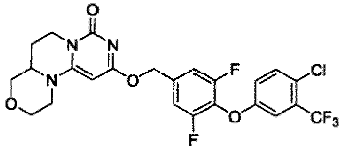 |
| 53 | 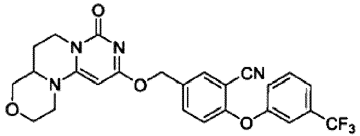 | 54 | 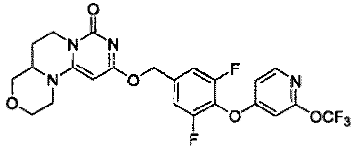 |
| 55 | 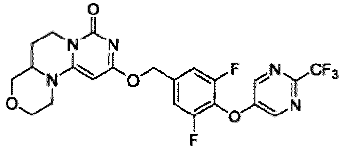 | 56 | 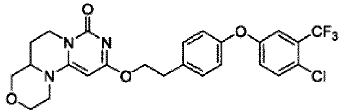 |
| 57 | 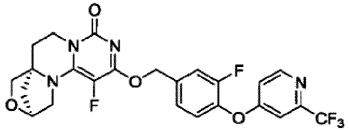 | 58 | 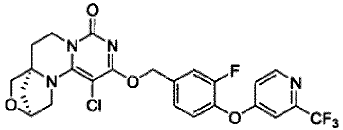 |
| 59 | 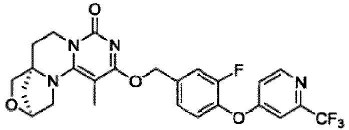 | 60 | 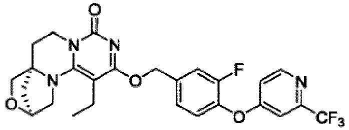 |
| 61 | 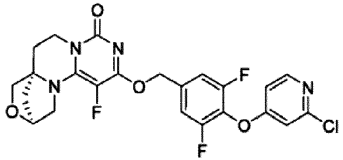 | 62 | 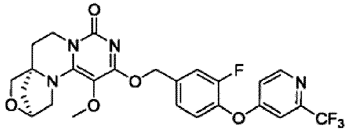 |
| 63 | 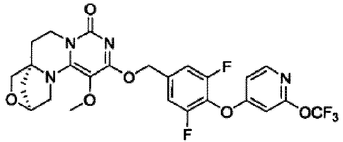 | 64 | 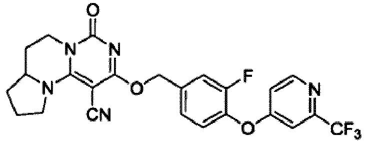 |

The compounds of the above formulas and their salts (such as the pharmaceutically-acceptable salts) can exist in the form of stereoisomers (e.g., containing one or more asymmetric carbon atoms). The individual stereoisomers (enantiomers and diastereomers) and their mixtures are included within the scope of the present invention.

The present invention further includes various deuterated forms of the compounds of the above formulas and their salts (such as the pharmaceutically-acceptable salts). The available hydrogen atoms connected to carbon atoms can be independently replaced by deuterium atoms. Those of ordinary skill in the art will understand how to synthesize the deuterated forms of the compounds of the above formulas and their salts (such as the pharmaceutically-acceptable salts). Commercially available deuterated raw materials can be used for the preparation of the compounds of the above formulas and their salts (such as the pharmaceutically-acceptable salts) in the deuterated form, or conventional techniques adopting deuterated reagents (such as lithium aluminum deuteride) can be used to synthesize these compounds.

In addition to the free base or free acid forms of the compounds described herein, the salt forms of the compounds are also within the scope of the present invention. The salts or pharmaceutically-acceptable salts of the compounds of the present invention can be prepared in situ during the final separation and purification process of the compounds, or by separately reacting the purified compounds in the form of a free acid or free base with a suitable base or acid. For a review of suitable pharmaceutical salts, see Berge et al., J. Pharm, Sci., 66,1-19,1977; P L Gould, International Journal of Pharmaceutics, 33 (1986), 201-217; and Bighley et al., Encyclopedia of Pharmaceutical Technology, Marcel Dekker Inc, New York 1996, Volume 13, pages 453-497.

The compounds, their salts (such as the pharmaceutically-acceptable salts), the deuterated forms, their solvates or hydrates described herein can exist in the form of one or more polymorphic substances. Therefore, in another aspect, the present invention provides polymorphic substances of the compounds and their salts (such as the pharmaceutically-acceptable salts) defined herein, or polymorphic substances of solvates or hydrates of the compounds or their salts (such as the pharmaceutically-acceptable salts) described herein.

The present invention further includes isotope-labeled compounds and salts, which are equivalent to the compounds of the above formulas or their salts, but one or more atoms are replaced by atoms with atomic masses or mass numbers different from those most commonly found in nature. Instances of isotopes that can be incorporated into compounds of the above formulas or their salts are isotopes of hydrogen, carbon, nitrogen, and deuterium. The compounds of the above formulas or their salts labeled with isotopes such as ³H, ¹¹C, ¹⁴C, and ¹⁸F can be used for medication and/or substrate tissue distribution testing. For example, isotopes ¹¹C and ¹⁸F can be used for PET (positron emission tomography). PET can be used for brain imaging. In one embodiment, the compounds of the above formulas or their salts are non-isotope-labeled.

Therefore, the compounds of the present invention include the compounds of the above formulas or their salts, such as their pharmaceutically-acceptable salts. Representative compounds of the present invention include the specific compounds.

The present invention further relates to a pharmaceutical composition containing the compound of the present invention and a pharmaceutically-acceptable excipient.

The present invention further relates to a method for treating or preventing diseases related to Lp-PLA₂ activity, including administrating a therapeutically effective amount of the compound of the present invention described herein to a subject in need. The diseases may be related to the following: the involvement of monocytes, macrophages, or lymphocytes increases the formation of lysophosphatidylcholine and oxidized free fatty acid and oxidation of lipid or endothelial dysfunction associated with Lp-PLA₂ activity.

The present invention also provides a method for treating or preventing diseases by inhibiting the Lp-PLA₂ activity. Exemplary diseases include but are not limited to: neurodegenerative diseases (such as Alzheimer's disease, Parkinson's disease, Huntington's disease, and vascular dementia), various neuropsychiatric diseases such as schizophrenia and autism, peripheral and cerebral atherosclerosis, stroke, metabolic bone diseases (such as bone marrow abnormalities), dyslipidemia, Paget's disease, type II diabetes, hypertension, angina pectoris, myocardial infarction, ischemia, reperfusion injury, metabolic syndrome, insulin resistance and hyperparathyroidism, diabetic complications (such as macular edema, diabetic retinopathy and posterior uveitis, diabetic ulcer and diabetic nephropathy), diabetic peripheral neuropathic pain, inflammatory pain, neuropathic pain, various cancers (such as prostate cancer, colon cancer, breast cancer, kidney cancer, lung cancer and ovarian cancer), macular edema, wound healing, male erectile dysfunction, rheumatoid arthritis, chronic obstructive pulmonary disease (COPD), sepsis, acute and chronic inflammation, psoriasis, and multiple sclerosis. The method includes administering a therapeutically effective amount of the compound of the present invention to a subject in need. The present invention is not intended to be limited to any specific stage of the diseases, such as early or late stages.

The present invention further provides a method for treating or preventing Alzheimer's disease. The method includes administrating a therapeutically effective amount of the compound of the present invention to a subject in need.

The present invention further provides a method for treating or preventing atherosclerosis. The method includes administrating a therapeutically effective amount of the compound of the present invention to a subject in need.

The present invention further provides a method for treating or preventing eye diseases by administrating the compound of the present invention. In some implementations, the present invention provides a method for treating macular edema, including administrating a therapeutically effective amount of the compound of the present invention to a subject in need. In some implementations, the macular edema is related to diabetic eye diseases (such as diabetic macular edema or diabetic retinopathy). In one implementation, the macular edema is related to posterior uveitis.

The present invention further provides a use of the compound of the present invention in the manufacture of medications for treating or preventing the diseases described herein.

The present invention further provides the compound of the present invention, which is used for treatment or prevention described herein.

### DETAILED DESCRIPTION

The term "and/or" used herein refers to any and all possible combinations that include one or more associated enumerated items. It can be further understood that the terms "comprising" and/or "including" used in the description indicate(s) the presence of the referred features, whole, steps, operations, elements, and/or components, but do not exclude the presence or addition of one or more other features, whole, steps, operations, elements, components, and/or combinations thereof.

Generally speaking, the naming used herein and the experimental operations for organic chemistry, pharmaceutical chemistry, and biology described herein are well-known in the art and widely adopted in the art. Unless otherwise defined, all technical and scientific terms used herein generally have the same meanings as those commonly understood by those of ordinary skill in the art to which the present disclosure belongs. In the case of multiple definitions of the terms used herein, unless otherwise specified, the definitions in this section shall prevail.

### Definitions

As used herein, unless otherwise stated, the term "disease" refers to any change in the state of the body or some organs, which disrupts or interferes with the execution of functions and/or causes symptoms (such as discomfort, dysfunction, adverse stress, or even death) in individuals who are ill or in contact with them.

As used herein, unless otherwise stated, "diabetic retinopathy" refers to the result of chronic progressive retinal microvascular leakage and obstruction caused by diabetes. "Diabetic macular edema" refers to retinal thickening or hard exudative deposition caused by the accumulation of extracellular fluid within the diameter range of an optic disc in the fovea of the macula caused by diabetes.

As used herein, unless otherwise stated, "neurodegenerative diseases" refer to different types of central nervous system disorders characterized by gradual and progressive losses of nerve tissue and/or nerve tissue functions. Neurodegenerative diseases are a type of neurological disease characterized by gradual and progressive losses and/or alteration of neural functions in nerve tissue, typically resulting in a decrease in neural function due to gradual and progressive losses of nerve tissue. In some implementations, the neurodegenerative diseases described herein include neurodegenerative diseases in which there is a defective blood-brain barrier (such as permeable blood-brain barrier). Examples of the neurodegenerative diseases with the defective blood-brain barrier include but are not limited to Alzheimer's disease, Huntington's disease, Parkinson's disease, and vascular dementia.

As used herein, unless otherwise stated, "vascular dementia", also known as "multiple infarct dementia", refers to a group of syndromes caused by different mechanisms that all lead to vascular damage in the brain. For example, the main subtypes of vascular dementia are vascular mild cognitive impairment, multiple infarct dementia, vascular dementia caused by major single infarcts (affecting the thalamus, anterior cerebral artery, parietal lobe, or cingulate gyrus), vascular dementia caused by hemorrhagic damage, small vessel diseases (including vascular dementia caused by lacunar damage and Binswanger disease), and mixed type dementia.

As used herein, unless otherwise stated, "neuropathic pain" refers to pain triggered or caused by primary damage and dysfunction of the nervous system.

As used herein, unless otherwise stated, "inflammatory pain" refers to pain caused by local acute inflammation or chronic inflammation that stimulates nerves.

As used herein, unless otherwise stated, "diabetic peripheral neuropathic pain" refers to pain caused by nerve injury complicated by diabetes, and nerve injury in diabetes is at least partially caused by decreased blood flow and high blood sugar.

As used herein, unless otherwise stated, "blood-brain barrier" or "BBB" can be interchangeably used herein to refer to a permeable barrier that exists in blood vessels passing through brain tissue, strictly limiting and closely regulating the exchange of substances between blood and brain tissue. The components of the blood-brain barrier include endothelial cells that form the innermost lining of all blood vessels, tight junctions between adjacent endothelial cells that serve as structural associations of the BBB, basement membranes of endothelial cells, and enlarged foot processes covering nearby astrocytes of almost all exposed outer surfaces of blood vessels.

As used herein, unless otherwise stated, "metabolic bone disease" refers to different types of bone diseases characterized by gradual and progressive losses of bone tissue. The metabolic bone disease described herein refers to a bone metabolic disease in which there is diffuse decrease in bone density and/or bone strength. This type of disease is characterized by histological appearance. Exemplary metabolic bone diseases include, but are not limited to, osteoporosis characterized by decreased mineral and bone matrix, and osteomalacia characterized by decreased mineral but intact bone matrix.

As used herein, unless otherwise stated, "osteopenia disease" or "osteopenia" can be used interchangeably herein, involving conditions with decreased calcification and/or bone density, and is a descriptive term used to represent all skeletal systems in which calcification and/or bone density decrease are/is observed. Osteopenia also refers to bone loss caused by insufficient synthesis of osteoid.

As used herein, unless otherwise stated, "osteoporosis" refers to a condition in which minerals and/or bone matrix are/is reduced and/or bone matrix is reduced.

As used herein, unless otherwise stated, "alkyl" is a monovalent, saturated hydrocarbon chain with a specified number of carbon atoms. For example, C₁₋₃ alkyl refers to alkyl with 1 to 3 carbon atoms. C₁₋₆ alkyl refers to alkyl with 1 to 6 carbon atoms. Alkyl can be linear or branched. In some embodiments, the branched alkyl may have one, two, or three branched chains. Exemplary alkyl includes, but is not limited to, methyl, methylethyl, ethyl, propyl (n-propyl and isopropyl), and butyl (n-butyl, isobutyl, and tert-butyl).

As used herein, unless otherwise stated, an "alkoxy" substituent is a group with the formula "R-O-", where R is the alkyl as defined above. For example, C₁₋₃ alkoxy refers to such alkoxy substituent containing 1 to 3 carbon. Exemplary alkoxy substituents include, but are not limited to, methoxy, ethoxy, n-propyloxy, n-butoxy, n-pentoxy, n-hexyloxy, isopropoxy, isobutoxy, sec-butoxy, tert-butoxy, isopentoxy, and neopentoxy.

As used herein, unless otherwise stated, "cycloalkyl" refers to a monovalent and saturated cyclic hydrocarbon group, including bridge rings and spiro rings, preferably having 3-7 cyclic carbon atoms, such as cyclopropyl, cyclobutyl, cyclopentyl, or [1.1.1]propellane, as well as those groups specifically exemplified in the following.

As used herein, unless otherwise stated, "aryl" refers to a hydrocarbon group containing one or more aromatic rings, such as phenyl or naphthyl.

As used herein, unless otherwise stated, "heteroaryl" refers to a stable single ring, double ring, or triple ring with up to 7 atoms in each ring, where at least one ring is aromatic and at least one ring contains 1 to 4 heteroatoms selected from O, N, and S. The heteroaryl within the scope of this definition includes but is not limited to acridinyl, carbazolyl, cinnolinyl, quinoxalinyl, quinazolinyl, pyrazolyl, indolyl, isoindolyl, 1H, 3H-1-oxoisoindolyl, benzotriazolyl, furanyl, thienyl, pyridinomorpholinyl, pyridinopyridyl, pyridinopyrrolidyl, benzothienyl, benzofuranyl, benzodioxane, benzodioxane, quinolinyl, isoquinolinyl, oxazolyl, isoxazolyl, benzoxazolyl, imidazolyl, pyrazinyl, pyridazinyl, pyridyl, pyrimidinyl, pyrrolyl, tetrahydroquinolinyl, thiazolyl, isothiazolyl, 1,2,3-triazolyl, 1,2,4-triazolyl, 1,2,4-oxadiazolyl, 1,2,4-thiadiazolyl, 1,3,5-triazinyl, 1,2,4-triazinyl, 1,2,4,5-tetrazinyl, tetrazolyl, xanthenyl, phenazinyl, phenothiazinyl, phenoxazinyl, azepinyl, oxepinyl, and thiepinyl. Special heteroaryl has 5- or 6-membered rings, such as furanyl, pyridyl, pyridazinyl, pyrimidinyl, pyrazinyl, thienyl, isoxazolyl, oxazolyl, diazolyl, imidazolyl, pyrryl, pyrazolyl, triazolyl, tetrazolyl, thiazolyl, isothiazolyl, thiadiazolyl, pyridinomorpholinyl, pyridinopiperidyl, and pyridinopyrrolidinyl.

As used herein, unless otherwise stated, "heterocyclic ring" or "heterocyclyl" refers to cyclic hydrocarbon in which 1 to 4 carbon atoms have been independently replaced by heteroatoms selected from N, N(R), S, S(O), S (O), and O. A heterocyclic ring can be saturated or unsaturated, but not aromatic. Heterocyclyl can also contain 1, 2, or 3 rings, including bridge ring and spiro ring structures. Instances of suitable heterocyclyl include, but are not limited to: azacyclobutane, oxocyclobutane, tetrahydrofuranyl, tetrahydrothienyl, pyrrolidinyl, 2-oxopyrrolidinyl, pyrrolinyl, pyranyl, dioxolanyl, piperidinyl, 2-oxopiperidinyl, pyrazolinyl, imidazolinyl, thiazolinyl, dithiolyl, oxothiolyl, dioxanyl, dioxenyl, dioxazolyl, oxathiozolyl, oxazolonyl, piperazinyl, morpholino, thiomorpholinyl, 3-oxomorpholinyl, dithialkyl, trithialkyl, and oxazinyl.

The term "bridge ring compound" refers to one or more atoms (i.e., C, O, N, or S) connecting two non-adjacent carbon atoms or nitrogen atoms. Preferred bridge rings include but are not limited to: one carbon atom, two carbon atoms, one nitrogen atom, two nitrogen atoms, and one carbon-nitrogen group. It is worth noting that a bridge always converts a single ring to a triple ring. In a bridge ring, substituents on the ring can also appear on the bridge.

The term "spiro ring compound" refers to a polycyclic compound in which two single rings share one carbon atom, and the shared carbon atom is called a spiro atom.

As used herein, unless otherwise stated, "halogen" refers to fluorine (F), chlorine (Cl), bromine (Br), or iodine (I). Halo refers to halogen groups: fluorine (-F), chlorine (-Cl), bromine (-Br), or iodine (-I).

As used herein, unless otherwise stated, "haloalkyl" refers to alkyl substituted by one or more halogen substituents, which may be the same or different. For example, C₁₋₃ haloalkyl refers to a haloalkyl substituent containing 1 to 3 carbon. Exemplary haloalkyl substituents include, but are not limited to, monofluoromethyl, difluoromethyl, trifluoromethyl, 1-chloro-2-fluoroethyl, trifluoropropyl, 3-fluoropropyl, and 2-fluoroethyl.

As used herein, unless otherwise stated, when two substituents on a ring combine with their interconnected atoms to form another ring, the ring may be spiro fused or unilateral fused. A spiro-fused ring system consists of two rings, which only have one common carbon atom. A unilateral-fused ring system consists of two rings, which share only two atoms and one bond.

As used herein, unless otherwise stated, "optionally substituted" indicates that a group or ring may be unsubstituted, or that the group or ring may be substituted by one or more substituents as defined herein.

As used herein, unless otherwise stated, "a 4-, 5-, or 6-membered saturated ring, optionally containing one heteroatom selected from N or O" refers to a 4-, 5-, or 6-membered saturated carbon ring and one carbon atom ring member being optionally substituted by one heteroatom selected from N or O, such as cyclobutyl, cyclopentanyl, cyclohexyl, azitidinyl, pyrrolidinyl, piperidinyl, oxocyclobutanyl, tetrahydrofuranyl, and tetrahydro-2H-pyranyl.

As used herein, unless otherwise stated, "treatment", "treat", or "treating" involving a disease refers to: (1) alleviating the disease or one or more biological manifestations of the disease, (2) interfering with (a) one or more points in the biological cascade that lead or cause the disease, or (b) one or more biological manifestations of the disease, (3) alleviating one or more symptoms or effects related to the disease, and/or (4) slowing down the progression of the disease or one or more biological manifestations of the disease, and/or (5) reducing the severity of the disease or the likelihood of biological manifestations of the disease.

As used herein, unless otherwise stated, "prevention" refers to the prophylactic administration of medications to reduce the likelihood or delay the occurrence of diseases or their biological manifestations.

As used herein, unless otherwise stated, a "subject" refers to a mammalian subject (such as dogs, cats, horses, cows, sheep, goats, and monkeys), and especially a human subject.

As used herein, unless otherwise stated, a "pharmaceutically-acceptable salt" refers to a salt that retains the required biological activity of a subject compound and exhibits the lowest undesired toxicological effect. These pharmaceutically-acceptable salts can be prepared in situ in the final separation and purification process of the compound, or by separately reacting purified compounds in the form of free acids or free bases with suitable bases or acids.

As used herein, unless otherwise stated, the term "therapeutically effective amount" refers to the amount that leads to treatment or prevention of diseases compared to corresponding subjects who did not receive the amount, but is sufficiently low within the range of reasonable medical judgment to avoid serious side effects (at a reasonable benefit/risk ratio). The therapeutically effective amount of a compound will change with the factors such as a specific compound selected (such as considering its efficacy, effect, and half-life), a selected route of administration; diseases to be treated; the severity of the diseases to be treated; the age, body size, weight, and physical condition of treated patients; the medical history of the treated patients; the duration of treatment; the nature of parallel treatment; and the required therapeutic effect, but can still be determined in a conventional manner by those skilled in the art.

### Synthesis of compounds

Those skilled in the art can understand that if a substituent described herein is incompatible with a synthesis method described herein, the substituent can be protected by a suitable protecting group that is stable under reaction conditions. The protecting group can be removed at appropriate points in the reaction sequence to obtain a required intermediate or target compound. Suitable protecting groups and methods for protecting and deprotecting different substituents by using such suitable protecting groups are well-known to those skilled in the art. Examples of this aspect can be found in I.Greene and P.Wuts, Protecting Groups in Chemical Synthesis (Third Edition), John Wiley&Sons, NY (1999). In some cases, substituents with reactivity under the reaction conditions used can be specifically selected. In these cases, the reaction conditions convert the selected substituent into another substituent that can be used as an intermediate compound or another substituent that can be used as a required substituent in the target compound.

### General scheme:

The general scheme provides a general synthesis route for compounds of formulas 1.9 and 2.8, where R₁, R₂, Rₓ, U, X, m, n and A are as defined in formula (I) 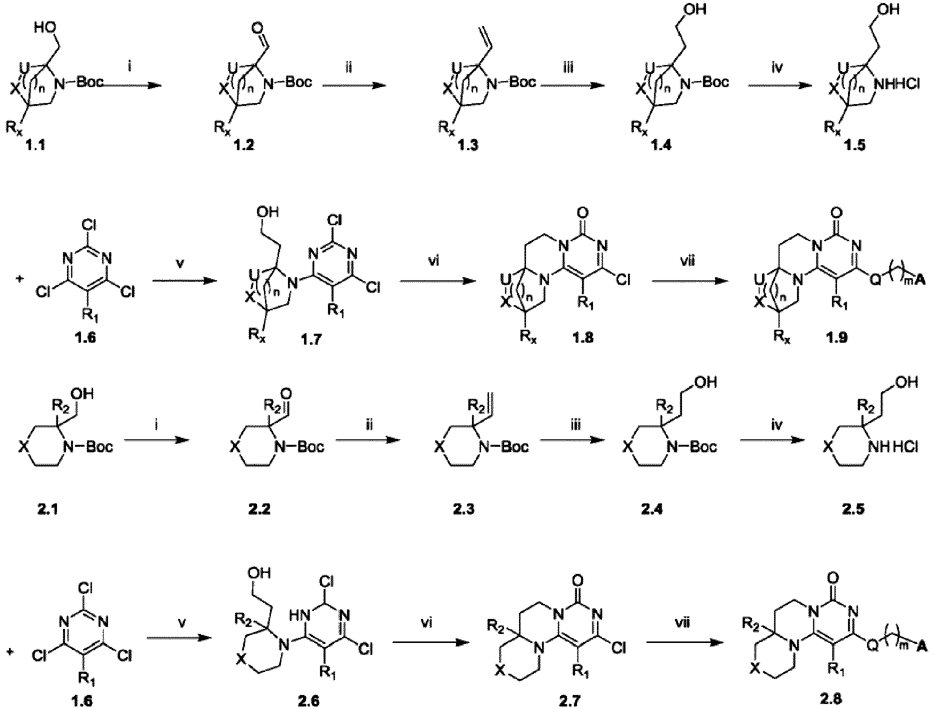 step (i), a compound 1.1 may react with a suitable reagent such as a Dess-Martin oxidant in a suitable solvent such as dichloromethane at a suitable temperature such as the room temperature to generate a compound 1.2. Step (ii), the compound 1.2 may have a Wittig reaction with a suitable reagent such as methyltriphenyl phosphonium bromide and NaH in a suitable solvent such as anhydrous tetrahydrofuran at a suitable temperature such as the room temperature to generate a compound 1.3. Step (iii), the compound 1.3 may react with a suitable reagent such as 9-borobicyclo[3.3.1]nonane in a suitable solvent such as anhydrous tetrahydrofuran at a suitable temperature such as the room temperature, and then react using a suitable reagent such as potassium hydroxide and hydrogen peroxide in a suitable solvent such as tetrahydrofuran and water at a suitable temperature such as 50°C to generate a compound 1.4. Step (iv), the compound 1.4 may remove a protecting group in a suitable reagent such as a hydrogen chloride/1,4-dioxane solution to generate a compound 1.5. Step (v), as an SN_{Ar} reaction, the compound 1.5 and a compound 1.6 may react using a suitable reagent such as triethylamine in a suitable solvent such as acetonitrile at a suitable temperature such as the room temperature to generate a compound 1.7. Step (vi), the compound 1.7 may convert hydroxyl into methanesulfonate or a chlorinated compound with a suitable reagent such as triethylamine and methylsulfonic chloride or dichlorosulfoxide at a suitable temperature such as 0°C or the room temperature, and then further conduct reflux reaction in a suitable base such as potassium carbonate and a suitable solvent such as acetonitrile without purification to obtain a compound 1.8 by cyclizing. Step (vii), the compound 1.8 reacts with corresponding alcohols (Q is -O-), mercaptans (Q is -S-) or amines (Q is -NR_{b}-), and H-Q-(CH₂)ₘ-A under the conditions of a suitable base such as NaH in a suitable solvent such as acetonitrile to obtain a final product 1.9. A compound 2.8 is prepared from alcohol 2.1. The changes in reaction condition and reactant are obvious to those skilled in the art.

### Use

The compounds of the present invention are Lp-PLA₂ inhibitors. Therefore, these compounds can be used for treatment, such as the treatment or prevention of diseases related to Lp-PLA₂ activity, including the use of therapeutically effective amounts of Lp-PLA₂ inhibitors to treat subjects in need of such treatment. Therefore, one aspect of the present invention relates to a method for treating or preventing diseases related to Lp-PLA₂ activity. Those skilled in the art can understand that specific diseases or their treatment may involve one or more potential mechanisms related to Lp-PLA₂ activity, including one or more of mechanisms described herein.

In some embodiments, the present invention provides a use of a compound of the present invention in the manufacture of medications for the treatment or prevention of any disease disclosed in the following disclosed patent applications: WO96/13484, WO96/19451, WO97/02242, WO97/12963, WO97/21675, WO97/21676, WO97/41098, WO97/41099, WO99/24420, WO00/10980, WO00/66566, WO00/66567, WO00/68208, WO01/60805, WO02/30904, WO02/30911, WO03/015786, WO03/016287, WO03/041712, WO03/042179, WO03/042206, WO03/042218, WO03/086400, WO03/87088, WO08/048867, US2008/0103156, US2008/0090851, US2008/0090852, WO08/048866, WO05/003118 (CA 2530816A1), WO06/063811, WO06/063813, WO2008/141176, WO2013013503A1, WO2013014185A1, WO2014114248A1, WO2014114694A1, WO2016011930A1, JP 200188847, US 2008/0279846 A1, US 2010/0239565 A1, and US 2008/0280829 A1.

In some embodiments, the present invention provides a use of the compound of the present invention in the manufacture of medications for treating eye diseases. The eye diseases applicable in the present invention may be related to the disruption of the blood retinal internal barrier (iBRB). Exemplary eye diseases involve diabetic eye diseases, including macular edema, diabetic retinopathy, posterior uveitis, retinal vein occlusion, etc. More eye diseases include, but are not limited to, central retinal vein occlusion, branch retinal vein occlusion, Iggar's syndrome (after cataract and surgery), retinitis pigmentosa, pars plana inflammation, shotgun like chorioretinopathy, outer retinal membrane, choroidal tumors, cystic macular edema, parafoveal telangiectasia, traction macular disease, vitreous macular traction syndrome, retinal detachment, retinitis optica, idiopathic macular edema, etc. More detailed information on the use of Lp-PLA₂ inhibitors for the treatment of eye diseases is provided in WO2012/080497, which is incorporated herein as a reference.

In addition, some embodiments of the present invention provide a use of the compound of the present invention in the manufacture of medications for treating or preventing diabetic macular edema in subjects. In some embodiments, the present invention provides a use of the compound of the present invention in the treatment of diabetic macular edema in subjects.

In certain embodiments, the present invention provides a use of the compound of the present invention in the manufacture of medications for treating or preventing subjects with macular edema or at risk of macular edema. In some embodiments, the present invention provides a use of the compound of the present invention in the manufacture of medications for treating subjects with macular edema or at risk of macular edema. In another embodiment, the macular edema is related to diabetic eye diseases, such as diabetic macular edema or diabetic retinopathy. In another implementation, the macular edema is related to posterior uveitis.

In certain embodiments, the present invention provides a use of the compound of the present invention in the manufacture of medications for treating or preventing glaucoma or macular degeneration. In some embodiments, the present invention provides a use of the compound of the present invention in the manufacture of medications for treating glaucoma or macular degeneration.

In one embodiment, the present invention provides a use of the compound of the present invention in the manufacture of medications for treating or preventing diseases related to the disruption of the blood retinal internal barrier in subjects in need of such treatment. In one embodiment, the present invention provides a use of the compound of the present invention in the manufacture of medications for treating diseases related to the disruption of the blood retinal internal barrier in subjects in need of such treatment.

In some embodiments, the present invention provides a use of the compound of the present invention in the manufacture of medications for treating or preventing any of the following diseases, which involve endothelial dysfunction, such as atherosclerosis (for example, peripheral vascular atherosclerosis and cerebral vascular atherosclerosis), diabetes, hypertension, angina, ischemia and reperfusion.

In some embodiments, the present invention provides a use of the compound of the present invention in the manufacture of medications for treating or preventing any of the following diseases, which involve lipid oxidation associated with enzyme activity, for example, other disorders other than diseases such as atherosclerosis and diabetes, such as rheumatoid arthritis, stroke, inflammatory diseases of the brain (such as Alzheimer's disease), various neuropsychiatric disorders (such as schizophrenia and autism), myocardial infarction, local ischemia, reperfusion injury, sepsis, and acute and chronic inflammation.

In some embodiments, the present invention provides a use of the compound of the present invention in the manufacture of medications for reducing the probability of central vascular events (such as heart attacks, myocardial infarction, or stroke) in patients with coronary heart disease.

In some embodiments, the present invention provides a use of the compound of the present invention in the manufacture of medications for treating or preventing the following diseases, which involve activated monocytes, macrophages, or lymphocytes, as all these cell types express Lp-PLA₂, including diseases involving activated macrophages (such as M1, dendritic, and/or other macrophages that generate oxidative stress). Exemplary diseases include but are not limited to bovine epilepsy, rheumatoid arthritis, wound healing, chronic obstructive pulmonary disease (COPD), cirrhosis, atopic dermatitis, emphysema, chronic pancreatitis, chronic gastritis, aortic aneurysm, atherosclerosis, multiple sclerosis, Alzheimer's disease and autoimmune diseases such as lupus.

In other embodiments, the present invention provides a use of the compound of the present invention in the manufacture of the following medications, which are used for the primary or secondary prevention of acute coronary events (e.g., caused by atherosclerosis); adjuvant treatment for preventing restenosis; or delay of the development of diabetic or hypertensive renal insufficiency. Prevention includes treating subjects at risk for such disorders.

In some embodiments, the present invention provides a method for treating or preventing neurological disorders related to abnormal blood brain barrier (BBB) functions, inflammation, and/or microglial activation in subjects in need of such treatment. In some embodiments, the present invention provides a method for treating or preventing neurological disorders related to abnormal blood brain barrier (BBB) functions, inflammation, and/or microglial activation in subjects in need of such treatment. The method includes administering a therapeutically effective amount of the compound of the present invention to a subject. In another embodiment, the abnormal BBB is a permeable BBB. In another embodiment, the disease is a neurodegenerative disease. This type of neurodegenerative disease is, but is not limited to, vascular dementia, Alzheimer's disease, Parkinson's disease, and Huntington's disease. In one embodiment, the present invention provides a method for treating or preventing diseases related to blood-brain barrier (BBB) leakage in subjects. In some embodiments, the present invention provides a method for treating diseases related to blood-brain barrier (BBB) leakage in subjects. Exemplary diseases include, but are not limited to, cerebral hemorrhage and cerebral amyloid angiopathy. In one embodiment, the neurodegenerative disease is Alzheimer's disease. In a specific embodiment, the neurodegenerative disease is vascular dementia. In one embodiment, the neurodegenerative disease is multiple sclerosis (MS).

In one embodiment, the compound of the present invention can be used to treat or prevent neurodegenerative diseases in subjects. The method includes administering the compound of the present invention (for example, in the form of a pharmaceutical composition containing the compound of the present invention) to a subject in need of such treatment. In one embodiment, the compound of the present invention can be used to treat neurodegenerative diseases in subjects. Exemplary neurodegenerative diseases include but are not limited to Alzheimer's disease, vascular dementia, Parkinson's disease, and Huntington's disease. In a specific embodiment, the neurodegenerative disease described in the present invention is related to an abnormal blood brain barrier. In one embodiment, the subjects who are administered medications that inhibit Lp-PLA₂ activity are humans.

In one embodiment, the present invention provides a method for treating or preventing subjects with vascular dementia or at the risk of vascular dementia. The method includes administering the compound of the present invention (such as a pharmaceutical composition containing a therapeutically effective amount of the compound of the present invention) to a subject. In one embodiment, the present invention provides a method for treating subjects with vascular dementia or at the risk of vascular dementia. In a specific embodiment, the vascular dementia is related to Alzheimer's disease.

In certain embodiments, the present invention relates to a method for treating or preventing metabolic bone diseases by administering a therapeutically effective amount of the compound of the present invention to subjects in need of such treatment. In some embodiments, the present invention relates to a method for treating metabolic bone diseases by administering a therapeutically effective amount of the compound of the present invention to subjects in need of such treatment. Exemplary metabolic bone diseases include diseases related to bone quality and bone density loss, including but not limited to osteoporosis and osteopenia. Exemplary osteoporosis and osteopenia include, but are not limited to, bone marrow abnormalities, dyslipidemia, Paget's disease, type II diabetes, metabolic syndrome, insulin resistance, hyperparathyroidism and related diseases. In another embodiment, the subject in need of such treatment is human.

It is believed that the method for preventing osteoporosis and/or osteopenia described herein may be influenced by inhibiting the expression of Lp-PLA₂ and/or inhibiting the protein activity of Lp-PLA₂. Therefore, some embodiments of the present invention provide a method for inhibiting Lp-PLA₂ by blocking enzyme activity. In another embodiment, a method for inhibiting Lp-PLA₂ by reducing and/or downregulating the expression of Lp-PLA₂ RNA is provided. In another embodiment, preventing and/or reducing bone loss and/or bone density loss lead(s) to the prevention or reduction of symptoms related to metabolic bone diseases such as osteoporosis and/or osteopenia.

In a specific embodiment, the method further includes administering other therapeutic agents for treating metabolic bone diseases to subjects in need of treatment. For example, when the metabolic bone disease is osteoporosis, other therapeutic agents can be used, such as bisphosphonates (such as alendronate, ibandronate, risephosphate, calcitonin and raloxifene), selective estrogen modulators (SERM), estrogen therapy, hormone replacement therapy (ET/HRT), and teriparatide.

In one embodiment, systemic inflammatory diseases such as juvenile rheumatoid arthritis, inflammatory bowel disease, Kawasaki disease, multiple sclerosis, sarcoidosis, polyarteritis, psoriatic arthritis, reactive arthritis, systemic lupus erythematosus, V-Kobayashi-Harada syndrome, Lyme disease, Behcet's disease, ankylosing spondylitis, chronic granulomatous disease, enthesitis may be the fundamental cause of posterior uveitis affecting the retina, and they can lead to macular edema. The present invention relates to a method for treating or preventing posterior uveitis or any of these systemic inflammatory diseases by administering a therapeutically effective amount of the compound of the present invention. In one embodiment, the present invention provides a method for treating posterior uveitis or any of these systemic inflammatory diseases by administering a therapeutically effective amount of the compound of the present invention.

The treatment and/or prevention of diseases related to Lp-PLA₂ activity can be achieved by using the compound of the present invention in single therapy or in dual or multiple combination therapies. For example, the compound of the present invention can be used in combination with anti-hyperlipidemia agents, anti-atherosclerosis agents, anti-diabetes agents, anti-angina agents, anti-inflammatory agents or anti-hypertension agents or agents for reducing lipoprotein (a) (Lp (a)) to treat or prevent the diseases described in the present invention. Examples of the aforementioned agents include but are not limited to cholesterol synthesis inhibitors, such as statins; antioxidants, such as propofol; insulin sensitizers; calcium channel blockers and anti-inflammatory drugs, such as non-steroidal anti-inflammatory drugs (NSAID). The agents for reducing Lp (a) include phosphoramidate described in WO 97/02037, WO 98/28310, WO 98/28311, and WO 98/28312. In one embodiment, the compound of the present invention can be used in combination with one or more statins. Statins are well-known cholesterol lowering agents, including atorvastatin, simvastatin, pravastatin, cilivastatin, fluvastatin, lovastatin, and rosuvastatin. In some embodiments, the compound of the present invention can be used together with anti-diabetes drugs or insulin sensitizers. In one embodiment, the compound of the present invention can be used together with PPAR γ activators such as GI262570 (GlaxoSmithKline) and glitazone compounds such as rosiglitazone, troglitazone, and pioglitazone. The agents can be administered in a therapeutically effective amount that is known in the art, or in an amount that is smaller or greater than the administration amount known to provide effective treatment in the art.

Combination treatment includes administering therapeutic agents in separate dosage forms or together in a single dosage form. Combination treatment can include simultaneous or separate administration of therapeutic agents, which can be essentially simultaneous or essentially separate administration. Typically, combination treatment includes administering each agent so that the therapeutically effective amount of each agent is present in the subject's body for at least a period of overlapping time.

### Use method

The therapeutically effective amount of the compound of the present invention will depend on many factors, for example, including the age and weight of an intended recipient, the precise condition and severity of the treatment required, the nature of a formulation, and the route of administration, and will ultimately depend on the judgment of a prescribing physician. However, the therapeutically effective amount of the compound of the present invention used for treating the diseases described in the text will typically range from 0.1 to 100 mg/kg recipient body weight/day, and more commonly from 1 to 10 mg/kg body weight/day. Therefore, for example, for adult mammals weighing 70 kg, the actual amount per day is usually 70 to 700 mg, and this amount can be given at a single dose per day or multiple sub-doses per day, such as two, three, four, five, or six doses per day. Alternatively, administration can be performed intermittently, such as once every other day, once a week, or once a month. It is expected that similar doses can be applied to treating other conditions mentioned above.

A pharmaceutical composition of the present invention may contain one or more compounds of the present invention. In some implementations, the pharmaceutical composition may contain more than one compound of the present invention. For example, in some implementations, the pharmaceutical composition may contain two or more compounds of the present invention. In addition, the pharmaceutical composition may further optionally contain one or more other pharmaceutical active compounds.

As used in the present invention, a "pharmaceutically-acceptable excipient" refers to pharmaceutically-acceptable raw materials, components, or carriers involved in imparting the morphology or consistency of the pharmaceutical composition. When mixed, each excipient can be compatible with other components of the pharmaceutical composition, thereby avoiding interactions that significantly reduce the efficacy of the compound of the present invention when administered to subjects and avoiding interactions that would result in pharmaceutically-unacceptable drug components.

The compound of the present invention and one or more pharmaceutically-acceptable excipients can be formulated into a dosage form suitable for administration to subjects through a required route of administration. For example, the dosage form includes those suitable for the following routes of administration: (1) oral administration (including oral or sublingual administration), such as tablets, capsules, caplets, pills, lozenges, powders, syrups, brews, suspensions, solutions, emulsions, sachets, and flat capsules; (2) parenteral administration (including subcutaneous, intramuscular, intravenous, or intradermal administration), such as sterile solutions, suspensions, and powders for reconstruction; (3) transdermal administration, such as transdermal patches; (4) rectal administration, such as suppositories; (5) nasal inhalation, such as dry powders, aerosols, suspensions, and solutions; and (6) local administration (including oral, sublingual or transdermal administration), such as creams, ointments, lotions, solutions, pastes, sprays, foams and gels. This type of composition can be prepared by any known method in the pharmaceutical field, such as by combining the compounds of the above formulas with a carrier or excipient.

Pharmaceutical compositions suitable for oral administration can exist as discrete units, such as capsules or tablets; powders or particles; solutions or suspensions in the form of an aqueous or non-aqueous liquid; edible foams or whips; or oil-in-water liquid emulsions or water-in-oil liquid emulsions.

Suitable pharmaceutically-acceptable excipients can vary depending on the specific dosage form selected. In addition, appropriate pharmaceutically-acceptable excipients can be selected based on their specific functions in the composition. For example, some pharmaceutically-acceptable excipients can be selected because they have the ability to promote the production of uniform dosage forms. Some pharmaceutically-acceptable excipients can be selected because they have the ability to promote the production of stable dosage forms. Some pharmaceutically-acceptable excipients can be selected because they promote the ability of one or more compounds of the present invention to be delivered or transported from one organ or part of the body to another organ or part of the body when administered to subjects. Some pharmaceutically-acceptable excipients can be selected because their ability to increase the patient's compliance.

Suitable pharmaceutically-acceptable excipients include the following types of excipients: diluents, fillers, adhesives, disintegrants, lubricants, flow aids, granulators, coating agents, wetting agents, solvents, cosolvents, suspensions, emulsifiers, sweeteners, seasonings, masking agents, colorants, anti-caking agents, humectants, chelating agents, plasticizers, viscosifiers, antioxidants, preservatives, stabilizers, surfactants and buffers. Those skilled in the art should understand that some pharmaceutically-acceptable excipients can provide more than one function, depending on the amount of excipients present in a formulation and what other components exist in the formulation that can provide other functions.

Skilled personnel possess knowledge and skills in the art, enabling them to select appropriate amounts of pharmaceutically-acceptable excipients for use in the present invention. In addition, skilled personnel have access to many resources that describe the pharmaceutically-acceptable excipients and can be used to select appropriate pharmaceutically-acceptable excipients. Examples include Remington's Pharmaceutical Sciences (Mack Publishing), The Hand book of Pharmaceutical Additives (Gower Publishing Limited), and The Handbook of Pharmaceutical Excipients (American Pharmacological Association and Drug Press).

The pharmaceutical composition of the present invention is prepared using techniques and methods known to those skilled in the art. Some commonly used methods in the art are described in Remington's Pharmaceutical Sciences (Mack Press).

In one aspect, the present invention relates to a solid oral dosage form containing a therapeutically effective amount of the compound of the present invention and a diluent or filler, such as tablets or capsules. Suitable diluents and fillers include lactose, saccharose, glucose, mannitol, sorbitol, starch (such as corn starch, potato starch, and pre-gelatinized starch), cellulose and its derivatives (such as microcrystalline cellulose), calcium sulfate, and calcium hydrogen phosphate. Oral solid dosage forms may further include adhesives. Suitable adhesives include starch (such as corn starch, potato starch, and pre-gelatinized starch), gelatin, arabic gum, sodium alginate, alginate, xanthan, guar gum, polyvidone, and cellulose and its derivatives (such as microcrystalline cellulose). Oral solid dosage forms may further include disintegrants. Suitable disintegrants include cross-linked polyvidone, starch sodium hydroxyacetate, croscarmelose, alginate, and carboxymethyl cellulose sodium. Oral solid dosage forms may further contain lubricants. Suitable lubricants include stearic acid, magnesium stearate, calcium stearate, and talc.

In specific implementations, the present invention relates to a pharmaceutical composition containing one or more compounds of the above formulas described herein or pharmaceutically-acceptable salts thereof, ranging from 0.01 mg to 1000 mg, and one or more pharmaceutically-acceptable excipients ranging from 0.01 g to 5 g.

### Preparation in Examples

### Intermediate 1

### Tert-butyl (1S,4R)-4-(hydroxymethyl)-2-oxa-5-azabicyclo[2.2.1]heptane-5-carboxylate

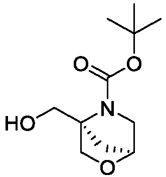

Methyl (1S,4S)-5-tert-butoxycarbonyl-2-oxa-5-azabicyclo[2.2.1]heptane-4-carboxylate (0.88 g, 3.4 mmol, Chemistry Letters, 2017, 566-568) was added to anhydrous tetrahydrofuran (30 mL), and cooled to 0°C, lithium borohydride (222 mg, 10.2 mmol) was slowly added, and stirring was conducted overnight at the room temperature. A reaction solution was cooled to 0°C, and sodium sulfate decahydrate was added for quenching. A reaction mixture was diluted with dichloromethane, anhydrous sodium sulfate was added for drying, filtering was conducted, a filter cake was washed with dichloromethane/methanol=20/1, and a filtrate was concentrated to obtain a crude title compound (1.5 g). LC-MS: m/z [M+H-tBu]⁺=174.

### Tert-butyl (1S,4S)-4-formyl-2-oxa-5-azabicyclo[2.2.1]heptane-5-carboxylate

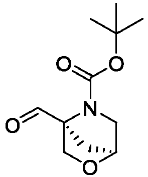

At the room temperature, tert-butyl (1S,4R)-4-(hydroxymethyl)-2-oxa-5-azabicyclo[2.2.1]heptane-5-carboxylate (5 g, 21.81 mmol) and a Dess-Martin reagent (10.18 g, 23.99 mmol) were added to dichloromethane (50 mL), and a reaction solution was stirred at the room temperature for 2 hours. The reaction solution was quenched with a saturated sodium thiosulfate aqueous solution, extracted with dichloromethane, concentrated in the organic phase, and purified by column chromatography (petroleum ether/ethyl acetate=5/1) to obtain the title compound (6.3 g, 127%). LC-MS: m/z [M+H-tBu]⁺=172.

### Tert-butyl (1S,4R)-4-vinyl-2-oxa-5-azabicyclo[2.2.1]heptane-5-carboxylate

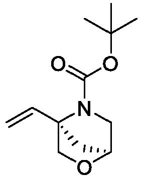

At the room temperature, tert-butyl (1S,4S)-4-formyl-2-oxa-5-azabicyclo[2.2.1]heptane-5-carboxylate (3 g, 13.20 mmol) and methyltriphenyl bromide (5.66 g, 15.84 mmol) were added to tetrahydrofuran (20 mL), and sodium hydride (60%, 1.58 g, 39.60 mmol) was slowly added with stirring. A reaction solution was stirred at the room temperature for 3 hours. The reaction solution was quenched with a saturated ammonium chloride solution, extracted with ethyl acetate, concentrated in the organic phase, and purified by column chromatography (petroleum ether/ethyl acetate=10/1) to obtain the title compound (2 g, 67%). LC-MS: m/z [M+H-tBu]⁺=170.

### Tert-butyl (1S,4S)-4-(2-hydroxyethyl)-2-oxa-5-azabicyclo[2.2.1]heptane-5-carboxylate

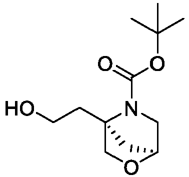

At the room temperature, tert-butyl (1S,4R)-4-vinyl-2-oxa-5-azabicyclo[2.2.1]heptane-5-carboxylate (1.8 g, 7.99 mmol) was added to a tetrahydrofuran solution of 9-borobicyclo[3.3.1]nonane (0.5 M, 64 mL, 32 mmol), and a reaction solution was stirred overnight at the room temperature. Water (8 mL), a sodium hydroxide aqueous solution (3 M, 40 mL), and hydrogen peroxide (37%, 40 mL) were added to the reaction solution in sequence, and the reaction solution was stirred at 50°C for 2 hours. After being cooled to the room temperature, the reaction solution was quenched with a saturated sodium thiosulfate aqueous solution, extracted with ethyl acetate, concentrated in the organic phase, and purified by column chromatography (dichloromethane/methanol=8/1) to obtain the title compound (1.8 g, 93%). LC-MS: m/z [M+H-tBu]⁺=188.

### 2-((1S,4S)-2-oxa-5-azabicyclo[2.2.1]hept-4-yl)ethanol hydrochloride

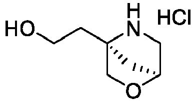

At the room temperature, tert-butyl (1S,4S)-4-(2-hydroxyethyl)-2-oxa-5-azabicyclo[2.2.1]heptane-5-carboxylate (800 mg, 3.30 mmol) was added to a hydrogen chloride/1,4-dioxane solution (4 M, 2 mL) and dichloromethane (8 mL), and a reaction solution was stirred at the room temperature for 2 hours. The reaction solution was concentrated, and residues were directly for the next step. LC-MS: m/z [M+H]⁺=144.

### 2-((1S,4S)-5-(2,6-dichloropyrimidin-4-yl)-2-oxa-5-azabicyclo[2.2.1]hept-4-yl)ethan-1-ol

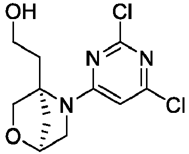

At the room temperature, 2-((1S,4S)-2-oxa-5-azabicyclo[2.2.1]hept-4-yl)ethanol hydrochloride (470 mg, 3.30 mmol), 2,4,6-trichloropyrimidine (0.91 g, 4.96 mmol), and sodium carbonate solid (1.05 g, 9.93 mmol) were added to acetonitrile (20 mL), and a reaction solution was stirred overnight at the room temperature. The reaction solution was filtered, a filtrate was concentrated, and purification was conducted through column chromatography (petroleum ether/ethyl acetate=1/1) to obtain the title compound (470 mg, two-step yield 49%). LC-MS: m/z [M+H]⁺=290.

### (7aS,10S)-2-chloro-6,7,10,11-tetrahydro-4H,8H-7a,10-methylaminopyrimidine[6',1':2,3 ]pyrimidine[6,1-c] [1,4] oxazin-4-one

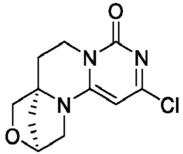

At the room temperature, 2-((1S,4S)-5-(2,6-dichloropyrimidin-4-yl)-2-oxa-5-azabicyclo[2.2.1]hept-4-yl)ethan-1-ol (235 mg, 0.81 mmol) was added to dichloromethane (8 mL), sulfoxide chloride (2 mL) was added with stirring, and stirring was conducted at the room temperature for 1 hour. A reaction solution was concentrated to dryness, sodium carbonate (0.086 g, 0.81 mmol) and acetonitrile (10 mL) were added to residues in sequence, and reflux stirring was conducted overnight. A reaction solution was filtered, and a filtrate was concentrated to obtain the title compound (150 mg, 73%). LC-MS: m/z [M+H]⁺=254.

### Intermediate 2

### Tert-butyl (1R,4S)-4-(hydroxymethyl)-2-oxa-5-azabicyclo[2.2.1]heptane-5-carboxylate

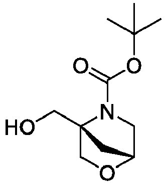

Methyl (1R,4R)-5-tert-butoxycarbonyl-2-oxa-5-azabicyclo[2.2.1]heptane-4-carboxylate (430 mg, 1.67 mmol, Chemistry Letters, 2017, 566-568) was added to anhydrous tetrahydrofuran (10 mL), and cooled to 0°C, lithium borohydride (430 mg, 1.67 mmol) was slowly added, and stirring was conducted overnight at the room temperature. A reaction solution was cooled to 0°C, and water was added for quenching. A reaction mixture was diluted with ethyl acetate, anhydrous sodium sulfate was added for drying, filtering was conducted, a filter cake was washed with ethanol, and a filtrate was concentrated. Dichloromethane was added to dissolve residues, filtering was conducted, and a filtrate was concentrated to obtain a crude title compound (380 mg, 99%). LC-MS: m/z [M+H-tBu]⁺=174.

### Tert-butyl (1R,4R)-4-formyl-2-oxa-5-azabicyclo[2.2.1]heptane-5-carboxylate

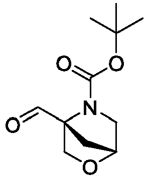

At the room temperature, tert-butyl (1R,4S)-4-(hydroxymethyl)-2-oxa-5-azabicyclo[2.2.1]heptane-5-carboxylate (9 g, 39.26 mmol) and a Dess-Martin reagent (19.98 g, 47.11 mmol) were added to dichloromethane (90 mL), and a reaction solution was stirred at the room temperature for 2 hours. The reaction solution was quenched with a saturated sodium thiosulfate aqueous solution, extracted with dichloromethane, concentrated in the organic phase, and purified by column chromatography (petroleum ether/ethyl acetate=5/1) to obtain the title compound (8.1 g, 98%). LC-MS: m/z [M+H-Boc]⁺=128.

### Tert-butyl (1R,4S)-4-vinyl-2-oxa-5-azabicyclo[2.2.1]heptane-5-carboxylate

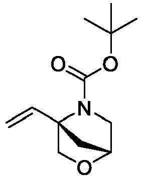

At the room temperature, tert-butyl (1R,4R)-4-formyl-2-oxa-5-azabicyclo[2.2.1]heptane-5-carboxylate (8.7 g, 38.28 mmol) and methyltriphenyl bromide (16.41 g, 45.94 mmol) were added to tetrahydrofuran (90 mL), and sodium hydride (60%, 4.59 g, 114.75 mmol) was slowly added with stirring. A reaction solution was stirred at the room temperature for 3 hours. The reaction solution was quenched with a saturated ammonium chloride solution, extracted with ethyl acetate, concentrated in the organic phase, and purified by column chromatography (petroleum ether/ethyl acetate=10/1) to obtain the title compound (6.8 g, 79%). LC-MS: m/z [M+H-Boc]⁺=126.

### Tert-butyl (1R,4R)-4-(2-hydroxyethyl)-2-oxa-5-azabicyclo[2.2.1]heptane-5-carboxylate

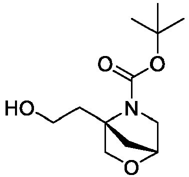

At the room temperature, tert-butyl (1R,4S)-4-vinyl-2-oxa-5-azabicyclo[2.2.1]heptane-5-carboxylate (6.8 g, 30.18 mmol) was added to a tetrahydrofuran solution of 9-borobicyclo[3.3.1]nonane (0.5 M, 181 mL, 90.5 mmol), and a reaction solution was stirred overnight at the room temperature. Water (18 mL), a sodium hydroxide aqueous solution (3 M, 90 mL), and hydrogen peroxide (37%, 90 mL) were added to the reaction solution in sequence, and the reaction solution was stirred at 50°C for 2 hours. After being cooled to the room temperature, the reaction solution was quenched with a saturated sodium thiosulfate aqueous solution, extracted with ethyl acetate, concentrated in the organic phase, and purified by column chromatography (dichloromethane/methanol=8/1) to obtain the title compound (5.5 g, 75%). LC-MS: m/z [M+H-Boc]⁺=144.

### 2-((1R,4R)-2-oxa-5-azabicyclo[2.2.1]hept-4-yl)ethanol hydrochloride

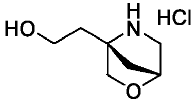

At the room temperature, tert-butyl (1R,4R)-4-(2-hydroxyethyl)-2-oxa-5-azabicyclo[2.2.1]heptane-5-carboxylate (5.5 g, 22.61 mmol) was added to a hydrogen chloride/1,4-dioxane solution (4 M, 17 mL) and dichloromethane (55 mL), and a reaction solution was stirred at the room temperature for 2 hours. The reaction solution was concentrated, and residues were directly for the next step. LC-MS: m/z [M+H]⁺=144.

### 2-((1R,4R)-5-(6-chloro-2-methoxypyrimidin-4-yl)-2-oxa-5-azabicyclo[2.2.1]hept-4-yl)et han-1-ol

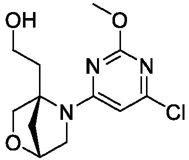

At the room temperature, 2-((1R,4R)-2-oxa-5-azabicyclo[2.2.1]hept-4-yl)ethanol hydrochloride (3.8 g, 26.54 mmol), 4,6-dichloro-2-methoxypyrimidine (7.13 g, 39.81 mmol), and sodium carbonate solid (8.44 g, 79.62 mmol) were added to acetonitrile (38 mL), and a reaction solution was stirred under reflux overnight. The reaction solution was filtered, reclaimed water was poured in, ethyl acetate was used for extraction, concentration was conducted in the organic phase, and purification was conducted through column chromatography (petroleum ether/ethyl acetate=1/1) to obtain the title compound (470 mg, two-step yield 49%). LC-MS: m/z [M+H]⁺=286.

### (7aR,10R)-2-chloro-6,7,10,11-tetrahydro-4H,8H-7a,10-methylaminopyrimidine[6',1':2, 3]pyrimidine[6,1-c] [1,4] oxazin-4-one

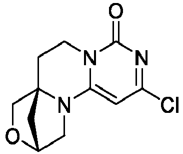

At the room temperature, 2-((1R,4R)-5-(2,6-dichloropyrimidin-4-yl)-2-oxa-5-azabicyclo[2.2.1]hept-4-yl)ethan-1-ol (4.33 g, 15.15 mmol) was added to dichloromethane (50 mL), sulfoxide chloride (3.3 mL) was added with stirring, and stirring was conducted at the room temperature for 1 hour. A reaction solution was concentrated to dryness, residues were dissolved in water (30 mL), a sodium hydroxide aqueous solution (5%, 25 mL) was dropwise added with stirring, stirring was conducted at the room temperature for 30 minutes, extraction was conducted with dichloromethane, concentration was conducted in the organic phase, ethyl acetate (10 mL) was added to residues, and stirring was conducted at the room temperature for 30 minutes. Filtering was conducted, and a filter cake was dried to obtain the title compound (1.43 g, 37%). LC-MS: m/z [M+H]⁺=254.

### Intermediate 3

### Tert-butyl 1-formyl-2-azabicyclo[2.1.1]hexane-2-carboxylate

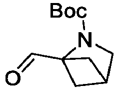

At the room temperature, tert-butyl 1-(hydroxymethyl)-2-azabicyclo[2.1.1]hexane-2-carboxylate (18.50 g, 86.74 mmol) and a Dess-Martin reagent (44.15 g, 104.09 mmol) were added to dichloromethane (200 mL), and a reaction solution was stirred at the room temperature for 2 hours. The reaction solution was quenched with a saturated sodium thiosulfate aqueous solution, extracted with dichloromethane, concentrated in the organic phase, and purified by column chromatography (petroleum ether/ethyl acetate=5/1) to obtain the title compound (15.2 g, 98%). LC-MS: m/z [M+H-Boc]⁺=112.

### Tert-butyl 1-vinyl-2-azabicyclo[2.1.1]hexane-2-carboxylate

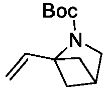

At the room temperature, tert-butyl 1-formyl-2-azabicyclo[2.1.1]hexane-2-carboxylate (8 g, 37.87 mmol) and methyltriphenyl bromide (16.23 g, 45.44 mmol) were added to tetrahydrofuran (80 mL), and sodium hydride (60%, 6.06 g, 151.48 mmol) was slowly added with stirring. A reaction solution was stirred at the room temperature for 3 hours. The reaction solution was quenched with a saturated ammonium chloride solution, extracted with ethyl acetate, concentrated in the organic phase, and purified by column chromatography (petroleum ether/ethyl acetate=10/1) to obtain the title compound (6.3 g, 79%). LC-MS: m/z [M+H-Boc]⁺=110.

### Tert-butyl 1-(2-hydroxyethyl)-2-azabicyclo[2.1.1]hexane-2-carboxylate

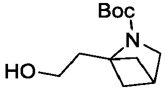

At the room temperature, tert-butyl 1-vinyl-2-azabicyclo[2.1.1]hexane-2-carboxylate (6.3 g, 30.10 mmol) was added to a tetrahydrofuran solution of 9-borobicyclo[3.3.1]nonane (0.5 M, 90.3 mL, 180.6 mmol), and a reaction solution was stirred overnight at the room temperature. Water (18 mL), a sodium hydroxide aqueous solution (3 M, 90 mL), and hydrogen peroxide (37%, 90 mL) were added to the reaction solution in sequence, and the reaction solution was stirred at 50°C for 2 hours. After being cooled to the room temperature, the reaction solution was quenched with a saturated sodium thiosulfate aqueous solution, extracted with ethyl acetate, concentrated in the organic phase, and purified by column chromatography (dichloromethane/methanol=8/1) to obtain the title compound (5.8 g, 85%). LC-MS: m/z [M+H-Boc]⁺=128.

### 2-(2-azabicyclo[2.1.1]hex-1-yl)ethan-1-ol hydrochloride

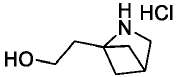

At the room temperature, tert-butyl 1-(2-hydroxyethyl)-2-azabicyclo[2.1.1]hexane-2-carboxylate (5.8 g, 25.52 mmol) was added to a hydrogen chloride/1,4-dioxane solution (4 M, 19 mL) and dichloromethane (60 mL), and a reaction solution was stirred at the room temperature for 2 hours. The reaction solution was concentrated, and residues were directly used for the next step. LC-MS: m/z [M+H]⁺=128.

### 2-(2-(6-chloro-2-methoxypyrimidin-4-yl)-2-azabicyclo[2.1.1]hex-1-yl)ethan-1-ol

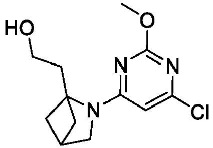

At the room temperature, 2-(2-azabicyclo[2.1.1]hex-1-yl)ethan-1-ol hydrochloride (3.7 g, 29.09 mmol), 4,6-dichloro-2-methoxypyrimidine (7.81 g, 43.63 mmol), and sodium carbonate solid (9.25 g, 87.27 mmol) were added to acetonitrile (40 mL), and a reaction solution was stirred under reflux overnight. The reaction solution was filtered, reclaimed water was poured in, extraction was conducted with ethyl acetate, concentration was conducted in the organic phase, and purification was conducted through column chromatography (petroleum ether/ethyl acetate=1/1) to obtain the title compound (4.5 g, two-step yield 57%). LC-MS: m/z [M+H]⁺=270.

### 2-chloro-6,7,9,10-tetrahydro-4H,8H-7a,9-methylaminopyrimidinyl[1,6-a]pyrrolo[t,2-c] pyrimidin-4-one

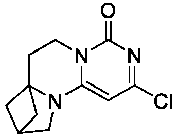

At the room temperature, 2-(2-(6-chloro-2-methoxypyrimidin-4-yl)-2-azabicyclo[2.1.1]hex-1-yl)ethan-1-ol (4.5 g, 16.68 mmol) was added to dichloromethane (50 mL), sulfoxide chloride (4 mL) was added with stirring, and stirring was conducted at the room temperature for 1 hour. A reaction solution was concentrated to dryness, water (30 mL) was added to residues, a saturated sodium bicarbonate aqueous solution was dropwise added with stirring to adjust pH=8-9, stirring was conducted at the room temperature for 30 minutes, extraction was conducted with dichloromethane, concentration was conducted in the organic phase, ethyl acetate (10 mL) was added to residues, stirring was conducted at the room temperature for 30 minutes, filtering was conducted, and a filter cake was dried to obtain the title compound (1.56 g, 40%). LC-MS: m/z [M+H]⁺=238.

### Intermediate 4

### Tert-butyl 2-formylpyrrolidine-1-carboxylate

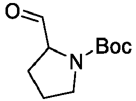

At the room temperature, tert-butyl 2-(hydroxymethyl)pyrrolidine-1-carboxylate (10 g, 49.69 mmol) and a Dess-Martin reagent (25.29 g, 59.63 mmol) were added to dichloromethane (100 mL), and a reaction solution was stirred at the room temperature for 2 hours. The reaction solution was quenched with a saturated sodium thiosulfate aqueous solution, extracted with dichloromethane, concentrated in the organic phase, and purified by column chromatography (petroleum ether/ethyl acetate=5/1) to obtain the title compound (13.8 g, 139%). LC-MS: m/z [M+H-tBu]⁺=144.

### Tert-butyl 2-vinylpyrrolidine-1-carboxylate

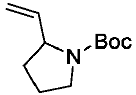

At the room temperature, tert-butyl 2-formylpyrrolidine-1-carboxylate (13.8 g, 69.26 mmol) and methyltriphenyl bromide (29.69 g, 83.11 mmol) were added to tetrahydrofuran (100 mL), and sodium hydride (60%, 8.31 g, 207.78 mmol) was slowly added with stirring. A reaction solution was stirred at the room temperature for 3 hours. The reaction solution was quenched with a saturated ammonium chloride solution, extracted with ethyl acetate, concentrated in the organic phase, and purified by column chromatography (petroleum ether/ethyl acetate=10/1) to obtain the title compound (7 g, 51%). LC-MS: m/z [M+H-tBu]⁺=142.

### Tert-butyl 2-(2-hydroxyethyl)pyrrolidine-1-carboxylate

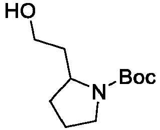

At the room temperature, tert-butyl 2-vinylpyrrolidine-1-carboxylate (7 g, 35.48 mmol) was added to a tetrahydrofuran solution of 9-borobicyclo[3.3.1]nonane (0.5 M, 210 mL, 106.44 mmol), and a reaction solution was stirred overnight at the room temperature. Water (21 mL), a sodium hydroxide aqueous solution (3 M, 105 mL), and hydrogen peroxide (37%, 105 mL) were added to the reaction solution in sequence, and the reaction solution was stirred at 50°C for 2 hours. After being cooled to the room temperature, the reaction solution was quenched with a saturated sodium thiosulfate aqueous solution, extracted with ethyl acetate, concentrated in the organic phase, and purified by column chromatography (dichloromethane/methanol=8/1) to obtain the title compound (6.4 g, 83%). LC-MS: m/z [M+H-Boc]⁺=116.

### 2-(pyrrolidin-2-yl)ethan-1-ol hydrochloride

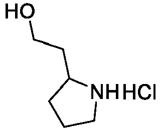

At the room temperature, tert-butyl 2-(2-hydroxyethyl)pyrrolidine-1-carboxylate (8 g, 13.93 mmol) was added to a hydrogen chloride/1,4-dioxane solution (4 M, 20 mL) and dichloromethane (20 mL), and a reaction solution was stirred at the room temperature for 2 hours. The reaction solution was concentrated, and residues were directly used for the next step. LC-MS: m/z [M+H]⁺=116.

### 2-(1-(6-chloro-2-methoxypyrimidin-4-yl)pyrrolidin-2-yl)ethan-1-ol

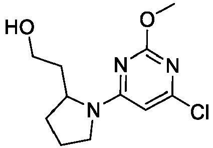

At the room temperature, 2-(pyrrolidin-2-yl)ethan-1-ol hydrochloride (1.6 g, 13.89 mmol), 4,6-dichloro-2-methoxypyrimidine (3.73 g, 20.84 mmol), and sodium carbonate solid (4.42 g, 41.67 mmol) were added to acetonitrile (40 mL), and a reaction solution was stirred under reflux overnight. The reaction solution was filtered, reclaimed water was poured in, extraction was conducted with ethyl acetate, concentration was conducted in the organic phase, and purification was conducted through column chromatography (petroleum ether/ethyl acetate=1/1) to obtain the title compound (2.9 g, two-step yield 81%). LC-MS: m/z [M+H]⁺=258.

### 2-chloro-6,7,7a,8,9,10-hexahydro-4H-pyrimidinyl[1,6-a] pyrrolo [1,2-c]pyrimidin-4-one

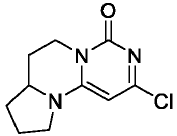

At the room temperature, 2-(1-(6-chloro-2-methoxypyrimidin-4-yl)pyrrolidin-2-yl)ethan-1-ol (2.8 g, 10.91 mmol) was added to dichloromethane (30 mL), sulfoxide chloride (8 mL) was added with stirring, and stirring was conducted at the room temperature for 1 hour. A reaction solution was concentrated to dryness, water (30 mL) was added to residues, pH was adjusted to alkaline with a sodium hydroxide aqueous solution (5%, 50 mL) with stirring, and stirring was conducted at the room temperature for 30 minutes. The reaction solution was extracted with dichloromethane and concentrated in the organic phase, ethyl acetate (10 mL) was added to residues, and stirring was conducted at the room temperature for 0.5 hours. Filtering was conducted, and a filter cake was concentrated under reduced pressure to obtain the title compound (1.2 g, 54%). LC-MS: m/z [M+H]⁺=225.

### Intermediate 5

### Methyl benzyl proline ester

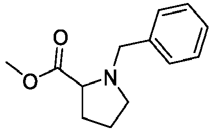

1-Boc-2-pyrrolidone methyl formate (25 g, 109.04 mmol) was added to a hydrogen chloride ethyl acetate solution (4 M, 100 mL) and stirred at the room temperature for 4 hours. A reaction solution was concentrated, residues, benzyl bromide (22.38 g, 130.85 mmol), and anhydrous potassium carbonate (30.1 g, 218.08 mmol) were added to acetonitrile (100 mL), and the temperature was raised for reflux stirring overnight. The reaction solution was poured into water, extracted with ethyl acetate, concentrated in the organic phase, and purified by column chromatography (petroleum ether/ethyl acetate=200/1-80/1) to obtain the title compound (18 g, 75%). LC-MS: m/z [M+H]⁺=220.

### Methyl 1-benzyl-2-methylpyrrolidine-2-carboxylate

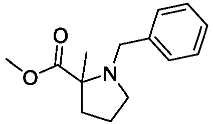

Methyl benzyl proline ester (8 g, 36.48 mmol) was added to tetrahydrofuran (100 mL) and cooled to -20°C under argon protection. Iodomethane (15.5 g, 109.44 mmol) was added to a reaction solution and cooled to -50°C, a tetrahydrofuran solution of diisopropylamino lithium (2 M, 63.84 mL, 127.68 mmol) was dropwise added by controlling the temperature at -50°C to -40°C, and after the dropwise addition, stirring was conducted for 3 hours by controlling the temperature at -50°C to -40°C. Methanol (50 mL) was added to the reaction solution for quenching, and then the reaction solution was poured into water, extracted with ethyl acetate, concentrated in the organic phase, and purified by column chromatography (petroleum ether/ethyl acetate=200/1-100/1) to obtain the title compound (5 g, 59%). LC-MS: m/z [M+H]⁺=234.

### (1-benzyl-2-methylpyrrolidin-2-yl)methanol

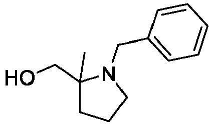

At the room temperature, lithium aluminum hydride (1.63 g, 42.90 mmol) was added to anhydrous tetrahydrofuran (25 mL) and cooled to 0°C under argon protection. An anhydrous tetrahydrofuran (25 mL) solution of methyl 1-benzyl-2-methylpyrrolidine-2-carboxylate (5 g, 21.45 mmol) was dropwise added. Stirring was conducted overnight at the room temperature. Sodium sulfate decahydrate (20 g) was added to a reaction solution and stirred at the room temperature for 30 minutes. The reaction solution was filtered, and a filtrate was concentrated to obtain a crude title compound (3.8 g, 86%). LC-MS: m/z [M+H]⁺=206.

### (2-methylpyrrolidin-2-yl)methanol

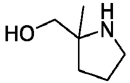

At the room temperature, (1-benzyl-2-methylpyrrolidin-2-yl)methanol (4.10 g, 19.97 mmol) and Pd/C (10%, 410 mg) were added to methanol (20 mL) for hydrogenation reaction at 50°C overnight. A reaction solution was filtered, and concentration was conducted in the organic phase to obtain a crude title compound, which is directly used for the next step of reaction. LC-MS: m/z [M+H]⁺=116.

### Tert-butyl 2-(hydroxymethyl)-2-methylpyrrolidine-1-carboxylate

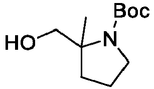

At the room temperature, (2-methylpyrrolidin-2-yl)methanol (2.3 g, 19.97 mmol) was added to dichloromethane (25 mL), sodium carbonate solid (4.23 g, 39.94 mmol) and di-tert-butyl dicarbonate (6.54 g, 29.95 mmol) were added in sequence with stirring, and stirring was conducted at the room temperature for 1.5 hours. A reaction solution was poured into water, extracted with ethyl acetate, and concentrated in the organic phase to obtain a crude title compound (7.2 g, 167%). LC-MS: m/z [M+H-tBu]⁺=160.

### Tert-butyl 2-formyl-2-methylpyrrolidine-1-carboxylate

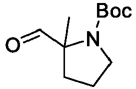

At the room temperature, tert-butyl 2-(hydroxymethyl)-2-methylpyrrolidine-1-carboxylate (7.1 g, 32.98 mmol) was added to dichloromethane (100 mL), a Dess-Martine oxidant (16.79 g, 39.58 mmol) was added with stirring, and stirring was conducted at the room temperature under argon protection for 1 hour. A reaction solution was quenched with a saturated sodium thiosulfate aqueous solution, extracted with ethyl acetate, concentrated in the organic phase, and purified by column chromatography (petroleum ether/ethyl acetate=20/1) to obtain the title compound (5.22 g, 74%). LC-MS: m/z [M+H-tBu]⁺=158.

### Tert-butyl 2-methyl-2-vinylpyrrolidine-1-carboxylate

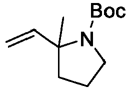

At the room temperature, tert-butyl 2-formyl-2-methylpyrrolidine-1-carboxylate (5.22 g, 24.48 mmol) was added to anhydrous tetrahydrofuran (50 mL), methyltriphenyl phosphonium bromide (10.49 g, 29.38 mmol) was added with stirring, the temperature was reduced to 0°C under argon protection, sodium hydride (60%, 2.94 g, 73.44 mmol) was added in batches, and after feeding, stirring was conducted at the room temperature for 3 hours. A reaction solution was quenched with a saturated ammonium chloride aqueous solution, extracted with ethyl acetate, concentrated in the organic phase, and purified by column chromatography (petroleum ether/ethyl acetate=20/1) to obtain the title compound (2.6 g, 50%). LC-MS: m/z [M+H-tBu]⁺=156.

### Tert-butyl 2-(2-hydroxyethyl)-2-methylpyrrolidine-1-carboxylate

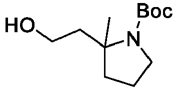

At the room temperature, tert-butyl 2-methyl-2-vinylpyrrolidine-1-carboxylate (2.6 g, 12.30 mmol) was added to a tetrahydrofuran solution of 9-borobicyclo[3.3.1]nonane (0.5 M, 98 mL, 49.00 mmol), and stirred overnight at the room temperature. Water (4 mL), a sodium hydroxide aqueous solution (3 M, 20 mL), and hydrogen peroxide (37%, 20 mL) were added to a reaction solution in sequence, and the reaction solution was stirred at 50°C for 2 hours. After being cooled to the room temperature, the reaction solution was quenched with a saturated sodium thiosulfate aqueous solution, extracted with ethyl acetate, concentrated in the organic phase, and purified by column chromatography (petroleum ether/ethyl acetate=2/1) to obtain the title compound (2.4 g, 85%). LC-MS: m/z [M+H-tBu]⁺=174.

### 2-(2-methylpyrrolidin-2-yl)ethane-1-ol hydrochloride

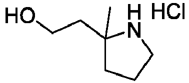

At the room temperature, tert-butyl 2-(2-hydroxyethyl)-2-methylpyrrolidine-1-carboxylate (2.3 g, 10.03 mmol) was added to a 1,4-dioxane solution of hydrogen chloride (4 M, 20 mL) and stirred at the room temperature for 1 hour. A reaction solution was concentrated to obtain a crude title compound (1.5 g, 116%). LC-MS: m/z [M+H]⁺=130.

### 2-(1-(6-chloro-2-methoxypyrimidin-4-yl)-2-methylpyrrolidin-2-yl)ethan-1-ol

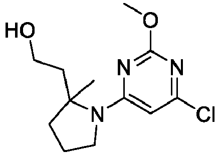

At the room temperature, 4,6-dichloro-2-methoxypyrimidine (2.13 g, 11.92 mmol) was added to acetonitrile (50 mL), 2-(2-methylpyrrolidin-2-yl)ethane-1-ol hydrochloride (1.4 g, 10.84 mmol) and sodium carbonate solid (3.45 g, 32.52 mmol) were added in sequence with stirring, and a reaction solution was stirred overnight at the room temperature. The reaction solution was poured into water, extracted with ethyl acetate, concentrated in the organic phase, and purified by column chromatography (dichloromethane/methanol=50/1) to obtain the title compound (2.52 g, 86%). LC-MS: m/z [M+H]⁺=272.

### 2-chloro-7a-methyl-6,7,7a,8,9,10-hexahydro-4H-pyrimidinyl[1,6-a]pyrrolo[1,2-c]pyrimi din-4-one

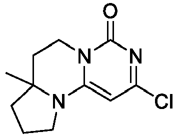

At the room temperature, 2-(1-(6-chloro-2-methoxypyrimidin-4-yl)-2-methylpyrrolidin-2-yl)ethan-1-ol (2.42 g, 8.91 mmol) and triethylamine (2.70 g, 26.73 mmol) were added to dichloromethane (20 mL), the temperature was reduced to 0°C, and methylsulfonic anhydride (2.33 g, 13.37 mmol) was added and stirred at the room temperature for 1 hour. A reaction solution was concentrated, potassium carbonate solid (3.56 g, 25.74 mmol) and acetonitrile (50 mL) were added to residues in sequence, and the reaction solution was stirred overnight at 85°C. The reaction solution was poured into water, extracted with dichloromethane, concentrated in the organic phase, and purified by column chromatography (dichloromethane/methanol=50/1) to obtain the title compound (705 mg, 34%). LC-MS: m/z [M+H]⁺=240.

### Intermediate 6

### Methyl 1-benzyl-2-ethylpyrrolidine-2-carboxylate

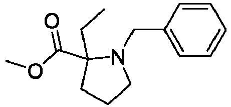

Methyl benzyl proline ester (8 g, 36.48 mmol) was added to tetrahydrofuran (100 mL) and cooled to -20°C under argon protection. Iodine ethane (17.1 g, 109.45 mmol) was added to a reaction solution and cooled to -50°C, a tetrahydrofuran solution of diisopropylamino lithium (2 M, 63.84 mL, 127.68 mmol) was dropwise added by controlling the temperature at -50°C to -40°C, and after the dropwise addition, stirring was conducted for 3 hours by controlling the temperature at -50°C to -40°C. After adding methanol to the reaction solution for quenching, the reaction solution was poured into water, extracted with ethyl acetate, concentrated in the organic phase, and purified by column chromatography (petroleum ether/ethyl acetate=200/1-100/1) to obtain the title compound (4.9 g, 54%). LC-MS: m/z [M+H]⁺=248.

### (1-benzyl-2-ethylpyrrolidin-2-yl)methanol

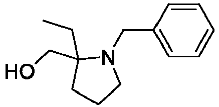

Lithium aluminum hydride (1.51 g, 39.66 mmol) was added to anhydrous tetrahydrofuran (25 mL) and cooled to 0°C under argon protection. An anhydrous tetrahydrofuran (25 mL) solution of methyl 1-benzyl-2-ethylpyrrolidine-2-carboxylate (4.9 g, 19.83 mmol) was dropwise added. Stirring was conducted overnight at the room temperature. Sodium sulfate decahydrate (5 g) was added to a reaction solution and stirred at the room temperature for 30 minutes. The reaction solution was filtered, and a filtrate was concentrated to obtain a crude title compound (3.5 g, 81%). LC-MS: m/z [M+H]⁺=220.

### (2-ethylpyrrolidin-2-yl)methanol

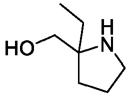

At the room temperature, (1-benzyl-2-ethylpyrrolidin-2-yl)methanol (8.3 g, 37.84 mmol) and Pd/C (10%, 830 mg) were added to methanol (45 mL) in sequence for hydrogenation reaction at 50°C overnight. A reaction solution was filtered, and a filtrate was concentrated to obtain a crude title compound, which is directly used for the next step of reaction. LC-MS: m/z [M+H]⁺=130.

### Tert-butyl 2-(hydroxymethyl)-2-ethylpyrrolidine-1-carboxylate

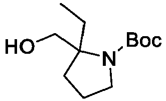

At the room temperature, (2-ethylpyrrolidin-2-yl)methanol (4.89, 37.85 mol) was added to dichloromethane (40 mL), and sodium carbonate solid (8.02 g, 75.7 mmol) and di-tert-butyl dicarbonate (13.39 g, 56.78 mmol) were added in sequence with stirring and stirred at the room temperature for 1.5 hours. A reaction solution was poured into water, extracted with ethyl acetate, and concentrated in the organic phase to obtain a crude title compound (14.1 g, 162%). LC-MS: m/z [M+H-tBu]⁺=174.

### Tert-butyl 2-formyl-2-ethylpyrolidine-1-carboxylate

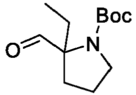

At the room temperature, tert-butyl 2-(hydroxymethyl)-2-ethylpyrrolidine-1-carboxylate (14 g, 61.05 mmol) was added to dichloromethane (150 mL), a Dess-Martin oxidant (31.07 g, 73.26 mmol) was added with stirring, and stirring was conducted at the room temperature for 1 hour under argon protection. A reaction solution was quenched with a saturated sodium thiosulfate aqueous solution, extracted with ethyl acetate, concentrated in the organic phase, and purified by column chromatography (petroleum ether/ethyl acetate=20/1) to obtain the title compound (8.1 g, 58%). LC-MS: m/z [M+H-tBu]⁺=172.

### Tert-butyl 2-ethyl-2-vinylpyrrolidine-1-carboxylate

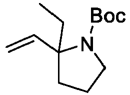

At the room temperature, tert-butyl 2-formyl-2-ethylpyrrolidine-1-carboxylate (8.1 g, 35.64 mmol) was added to anhydrous tetrahydrofuran (50 mL), methyltriphenyl phosphonium bromide (15.28 g, 42.77 mmol) was added with stirring, the temperature was reduced to 0°C under argon protection, sodium hydride (60%, 4.28 g, 106.92 mmol) was added in batches, and after feeding, stirring was conducted at the room temperature for 3 hours. A reaction solution was quenched with a saturated ammonium chloride aqueous solution, extracted with ethyl acetate, concentrated in the organic phase, and purified by column chromatography (petroleum ether/ethyl acetate=20/1) to obtain the title compound (4.3 g, 54%). LC-MS: m/z [M+H-tBu]⁺=170.

### Tert-butyl 2-(2-hydroxyethyl)-2-ethylpyrrolidine-1-carboxylate

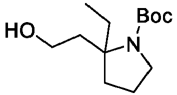

At the room temperature, tert-butyl 2-ethyl-2-vinylpyrrolidine-1-carboxylate (4.3 g, 19.08 mmol) was added to a tetrahydrofuran solution of 9-borobicyclo[3.3.1]nonane (0.5 M, 40 mL, 20.00 mmol), and stirred overnight at the room temperature. Water (4 mL), a sodium hydroxide aqueous solution (3 M, 20 mL), and hydrogen peroxide (37%, 20 mL) were added to a reaction solution in sequence, and the reaction solution was stirred at 50°C for 2 hours. After being cooled to the room temperature, the reaction solution was quenched with a saturated sodium thiosulfate aqueous solution, extracted with ethyl acetate, concentrated in the organic phase, and purified by column chromatography (petroleum ether/ethyl acetate=2/1) to obtain the title compound (2.91 g, 63%). LC-MS: m/z [M+H-tBu]⁺=188.

### 2-(2-ethylpyrrolidin-2-yl)ethane-1-ol hydrochloride

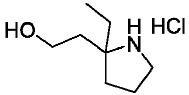

At the room temperature, tert-butyl 2-(2-hydroxyethyl)-2-ethylpyrrolidine-1-carboxylate (2.81 g, 11.55 mmol) was added to a 1,4-dioxane solution (20 mL) of hydrogen chloride and stirred for 1 hour at the room temperature. A reaction solution was concentrated to obtain a crude title compound (2.08 g, 100%). LC-MS: m/z [M+H]⁺=144.

### 2-(1-(6-chloro-2-methoxypyrimidin-4-yl)-2-ethylpyrrolidin-2-yl)ethan-1-ol

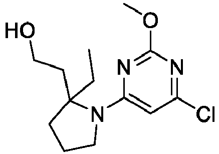

At the room temperature, 4,6-dichloro-2-methoxypyrimidine (2.08 g, 11.55 mmol) was added to acetonitrile (50 mL), 2-(2-ethylpyrrolidin-2-yl)ethane-1-ol hydrochloride (2.08 g, 11.55 mmol) and sodium carbonate solid (3.66 g, 34.56 mmol) were added in sequence with stirring, and a reaction solution was stirred overnight at the room temperature. The reaction solution was poured into water, extracted with ethyl acetate, concentrated in the organic phase, and purified by column chromatography (dichloromethane/methanol=50/1) to obtain the title compound (0.90 g, 27%). LC-MS: m/z [M+H]⁺=272.

### 2-chloro-7a-ethyl-6,7,7a,8,9,10-hexahydro-4H-pyrimidinyl[1,6-a]pyrrolo[1,2-c]pyrimidi n-4-one

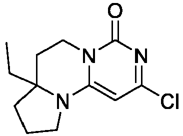

At the room temperature, 2-(1-(6-chloro-2-methoxypyrimidin-4-yl)-2-ethylpyrrolidin-2-yl)ethan-1-ol (0.90 g, 3.15 mmol) and triethylamine (0.95 g, 9.45 mmol) were added to dichloromethane (20 mL) and cooled to 0°C, and methylsulfonic anhydride (0.82 g, 4.73 mmol) was added and stirred at the room temperature for 1 hour. A reaction solution was concentrated, potassium carbonate solid (1.31 g, 9.48 mmol) and acetonitrile (50 mL) were added to residues in sequence, and the reaction solution was stirred overnight at 85°C. The reaction solution was poured into water, extracted with dichloromethane, concentrated in the organic phase, and purified by column chromatography (dichloromethane/methanol=50/1) to obtain the title compound (705 mg, 34%). LC-MS: m/z [M+H]⁺=254.

### Intermediate 7

### (S)-2-(1-(2,6-dichloropyrimidin-4-yl)piperidin-2-yl)ethan-1-ol

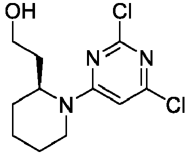

At the room temperature, 2,4,6-trichloropyrimidine (300.81 mg, 1.64 mmol) was added to acetonitrile (10 mL), and (S)-2-(piperidin-2-yl)ethan-1-ol (211.89 mg, 1.64 mmol) and sodium carbonate solid (521.47 mg, 4.92 mol) were added in sequence with stirring and stirred at the room temperature for 2 hours. A reaction solution was poured into water, extracted with ethyl acetate, concentrated in the organic phase, and purified by thin layer chromatography (petroleum ether/ethyl acetate=2/1) to obtain the title compound (310 mg, 68%). LC-MS: m/z [M+H]⁺=276.

### (S)-2-chloro-7,7a,8,9,10,11-hexahydro-4H,6H-pyridyl[1,2-c]pyrimidinyl[1,6-a]pyrimidi n-4-one

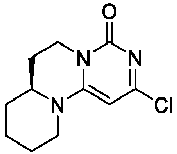

At the room temperature, (S)-2-(1-(2,6-dichloropyrimidin-4-yl)piperidin-2-yl)ethan-1-ol (198.84 mg, 0.72 mmol) was added to dichloromethane (4 mL), and was cooled to 0°C through reaction, and triethylamine (36.43 mg, 0.36 mmol) and methylsulfonic anhydride (188.14 mg, 1.08 mmol) were sequentially added with stirring, and stirred overnight at the room temperature. A reaction solution was concentrated, acetonitrile (5 mL) and potassium carbonate solid (302.68 mg, 2.19 mmol) were sequentially added to residues, and the reaction solution was stirred under reflux overnight. The reaction solution was poured into water, extracted with dichloromethane, and concentrated in the organic phase to obtain a crude title compound, which is directly used for the next step of reaction. LC-MS: m/z [M+H]⁺=240.

Referring to the table below, in addition to replacing corresponding raw materials with raw materials described in the column "raw material", the following intermediate was prepared with reference to the preparation method of the intermediate 7.

| **No. of interm ediate** | **Name of intermediate** | **Structural formula** | **LC-MS (m/z [M+H]⁺)** | **Raw material** |
|---|---|---|---|---|
| **8** | 2-chloro-7,7a ,8,9,10,11-he xahydro-4H,6 H-pyridyl[1,2 -c]pyrimidiny l[1,6-a]pyrim idine-4-one | 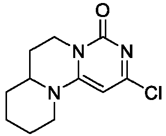 | 240 | 2,4,6-trichloropy rimidine, 2-(piperidin-2-yl )ethan-1-ol |

### Intermediate 9

### Methyl N-Boc-2-methylpiperidine-2-formate

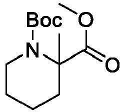

Methyl N-BOC-piperidine-2-formate (10.0 g, 41.15 mmol) and iodomethane (16.2 g, 123.4 mmol) were added to tetrahydrofuran (60 mL) and cooled to 0°C, and a tetrahydrofuran solution of diisopropylamino lithium (2 M, 61.7 mL, 123.4 mmol) was dropwise added. After stirring for 3 hours, a reaction solution was poured into a saturated ammonium chloride solution, extracted with ethyl acetate, concentrated in the organic phase, and purified by column chromatography (petroleum ether/ethyl acetate=20/1) to obtain the title compound (10.0 g, 95%). LC-MS: m/z [M+H-Boc]⁺=158.

### Methyl 2-methylpiperidine-2-formate hydrochloride

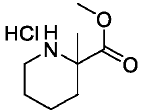

At the room temperature, methyl N-Boc-2-methylpiperidine-2-formate (2 g, 7.77 mmol) was added to hydrogen chloride/1,4-dioxane (4 M, 10 mL) and stirred at the room temperature for 0.5 hours. A reaction solution was concentrated to obtain a crude title compound, which is directly used for the next step of reaction. LC-MS: m/z [M+H]⁺=158.

### (2-methylpiperidin-2-yl)methanol

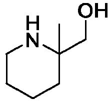

At the room temperature, methyl 2-methylpiperidine-2-formate hydrochloride (1.5 g, 7.76 mmol) was added to anhydrous tetrahydrofuran (16 mL), and lithium aluminum tetrahydride (0.59 g, 15.52 mmol) was added with stirring, and stirred at the room temperature for 0.5 hours. Sodium sulfate decahydrate was added to a reaction solution and stirred at the room temperature for 0.5 hours. Filtering was conducted, and a filtrate was concentrated to obtain a crude title compound, which is directly used for the next step of reaction. LC-MS: m/z [M+H]⁺=130.

### Tert-butyl 2-(hydroxymethyl)-2-methylpiperidine-1-formate

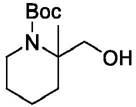

(2-methylpiperidin-2-yl)methanol (1.00 g, 7.74 mmol) was added to dichloromethane (12 mL), and di-tert-butyl dicarbonate (2.03 g, 9.29 mmol) and sodium carbonate solid (2.46 g, 23.22 mmol) were sequentially added with stirring, and stirred overnight at the room temperature. A reaction solution was filtered, a filtrate was concentrated, and purification was conducted through column chromatography (petroleum ether/ethyl acetate=50/1-10/1) to obtain the title compound (0.6 g, three-step yield: 34%). LC-MS: m/z [M+H-Boc]⁺=130.

### Tert-butyl 2-formyl-2-methylpiperidine-1-carboxylate

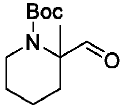

At the room temperature, tert-butyl 2-(hydroxymethyl)-2-methylpiperidine-1-carboxylate (2.20 g, 9.59 mmol) was added to dichloromethane (24 mL), and a Dess-Martin oxidant (4.88 g, 11.51 mmol) was added with stirring, and stirred at the room temperature for 3 hours. A reaction solution was quenched with a sodium thiosulfate aqueous solution, extracted with dichloromethane, concentrated in the organic phase, and purified by column chromatography (petroleum ether/ethyl acetate=50/1-20/1) to obtain the title compound (1.1 g, 50%). LC-MS: m/z [M+H-Boc]⁺=128.

### Tert-butyl 2-methyl-2-vinylpiperidine-1-carboxylate

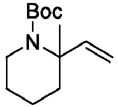

At the room temperature, tert-butyl 2-formyl-2-methylpiperidine-1-carboxylate (1.10 g, 4.84 mmol) was added to anhydrous tetrahydrofuran (16 mL), and methyltriphenyl phosphonium bromide (2.07 g, 5.81 mmol) and sodium hydride (60%, 0.58 g, 14.52 mmol) were sequentially added with stirring, and stirred at the room temperature for 6 hours. A reaction solution was quenched with a saturated ammonium chloride aqueous solution, extracted with ethyl acetate, concentrated in the organic phase, and purified by column chromatography (petroleum ether/ethyl acetate=100/1-20/1) to obtain the title compound (760 mg, 70%). LC-MS: m/z [M+H-Boc]⁺=126.

### Tert-butyl 2-(2-hydroxyethyl)-2-methylpiperidine-1-formate

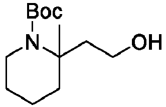

At the room temperature, tert-butyl 2-methyl-2-vinylpiperidine-1-carboxylate (760 mg, 3.37 mmol) was added to a tetrahydrofuran solution of 9-borobicyclo[3.3.1]nonane (0.5 M, 27 mL, 13.5 mmol), and a reaction solution was stirred overnight at the room temperature. Water (3 mL), a sodium hydroxide aqueous solution (3 M, 16 mL), and hydrogen peroxide (37%, 8 mL) were sequentially added to the reaction solution, and the reaction solution was stirred at 50°C for 2 hours. After being cooled to the room temperature, the reaction solution was quenched with a saturated sodium thiosulfate aqueous solution, extracted with ethyl acetate, concentrated in the organic phase, and purified by column chromatography (dichloromethane/methanol=20/1) to obtain the title compound (560 mg, 69%). LC-MS: m/z [M+H-Boc]⁺=144.

### 2-(2-methylpiperidin-2-yl)ethan-1-ol hydrochloride

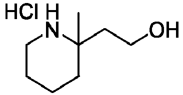

At the room temperature, tert-butyl 2-(2-hydroxyethyl)-2-methylpiperidine-1-formate (560 mg, 2.30 mmol) was added to a hydrogen chloride/1,4-dioxane solution (4 M, 4 mL), and a reaction solution was stirred at the room temperature for 0.5 hours. The reaction solution was concentrated, and residues were directly used for the next step. LC-MS: m/z [M+H]⁺=144.

### 2-(1-(6-chloro-2-methoxypyrimidin-4-yl)-2-methylpiperidin-2-yl)ethan-1-ol

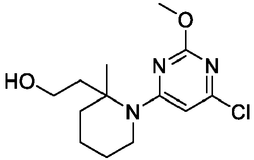

At the room temperature, 2-(2-methylpiperidin-2-yl)ethan-1-ol hydrochloride (410 mg, 2.30 mmol), 4,6-dichloro-2-methoxypyrimidine (0.61 g, 3.42 mmol), and sodium carbonate solid (0.97 g, 9.12 mmol) were added to acetonitrile (24 mL), and a reaction solution was stirred under reflux for 72 hours. The reaction solution was filtered, a filtrate was concentrated, and purification was conducted through column chromatography (dichloromethane/methanol=20/1) to obtain the title compound (200 mg, two-step yield: 30%). LC-MS: m/z [M+H]⁺=286.

### 2-chloro-7a-methyl-7,7a,8,9,10,11-hexahydro-4H,6H-pyridyl[1,2-c]pyrimidinyl[1,6-a]p yrimidin-4-one

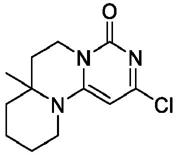

At the room temperature, 2-(1-(6-chloro-2-methoxypyrimidin-4-yl)-2-methylpiperidin-2-yl)ethan-1-ol (200 mg, 0.70 mmol) was added to dichloromethane (6 mL), and sulfoxide chloride (0.25 mL) was added with stirring, and stirred at the room temperature for 0.5 hours. A reaction solution was concentrated to dryness, and water (6 mL) and potassium carbonate (0.39 g, 2.84 mmol) were sequentially added to residues, and stirred at the room temperature for 0.5 hours. The reaction solution was poured into water, extracted with dichloromethane, concentrated in the organic phase, and purified by preparation of thin layer chromatography (dichloromethane/methanol=20/1) to obtain the title compound (70 mg, 39%). LC-MS: m/z [M+H]⁺=254.

### Intermediate 10

### Tert-butyl 3-formylmorpholine-4-carboxylate

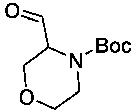

At the room temperature, tert-butyl 3-(hydroxymethyl)morpholine-4-carboxylate (10 g, 46.03 mmol) and a Dess-Martin reagent (23.43 g, 55.24 mmol) were added to dichloromethane (100 mL), and a reaction solution was stirred at the room temperature for 2 hours. The reaction solution was quenched with a saturated sodium thiosulfate aqueous solution, extracted with dichloromethane, concentrated in the organic phase, and purified by column chromatography (petroleum ether/ethyl acetate=5/1) to obtain the title compound (7.2 g, 73%). LC-MS: m/z [M+H-Boc]⁺=116.

### Tert-butyl 3-vinylmorpholine-4-carboxylate

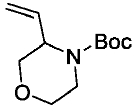

At the room temperature, tert-butyl 3-formylmorpholine-4-carboxylate (7.2 g, 33.45 mmol) and methyltriphenyl bromide (14.34 g, 40.14 mmol) was added to tetrahydrofuran (80 mL), and sodium hydride (60%, 5.35 g, 133.8 mmol) was slowly added with stirring. A reaction solution was stirred at the room temperature for 3 hours. The reaction solution was quenched with a saturated ammonium chloride solution, extracted with ethyl acetate, concentrated in the organic phase, and purified by column chromatography (petroleum ether/ethyl acetate=10/1) to obtain the title compound (5.1 g, 71%). LC-MS: m/z [M+H-Boc]⁺=114.

### Tert-butyl 3-(2-hydroxyethyl)morpholine-4-carboxylate

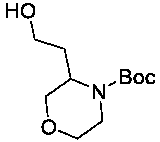

At the room temperature, tert-butyl 3-vinylmorpholine-4-carboxylate (5.1 g, 23.91 mmol) was added to a tetrahydrofuran solution of 9-borobicyclo[3.3.1]nonane (0.5 M, 143 mL, 71.7 mmol), and a reaction solution was stirred overnight at the room temperature. Water (14 mL), a sodium hydroxide aqueous solution (3 M, 36 mL), and hydrogen peroxide (37%, 36 mL) were sequentially added to the reaction solution, and the reaction was stirred at 50°C for 2 hours. After being cooled to the room temperature, the reaction solution was quenched with a saturated sodium thiosulfate aqueous solution, extracted with ethyl acetate, concentrated in the organic phase, and purified by column chromatography (dichloromethane/methanol=80/1) to obtain the title compound (4.9 g, 89%). LC-MS: m/z [M+H-Boc]⁺=132.

### 2-(morpholin-3-yl)ethan-1-ol hydrochloride

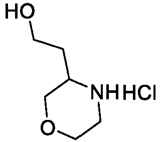

At the room temperature, tert-butyl 3-(2-hydroxyethyl)morpholine-4-carboxylate (3 g, 12.97 mmol) was added to a hydrogen chloride/1,4-dioxane solution (4 M, 9.73 mL) and dichloromethane (30 mL), and a reaction solution was stirred at the room temperature for 2 hours. The reaction solution was concentrated, and residues were directly used for the next step. LC-MS: m/z [M+H]⁺=132.

### 2-(4-(2,6-dichloropyrimidin-4-yl)morpholin-3-yl)ethan-1-ol

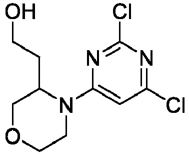

At the room temperature, 2-(morpholin-3-yl)ethan-1-ol hydrochloride (1.86 mg, 14.18 mmol), 4,6-dichloro-2-methoxypyrimidine (3.81 g, 21.27 mmol), and sodium carbonate solid (4.51 g, 42.54 mmol) were added to acetonitrile (20 mL), and a reaction solution was stirred under reflux overnight. The reaction solution was filtered, reclaimed water was poured in, extraction was conducted with ethyl acetate, concentration was conducted in the organic phase, and purification was conducted through column chromatography (petroleum ether/ethyl acetate=1/1) to obtain the title compound (1.93 g, 50%). LC-MS: m/z [M+H]⁺=274.

### 2-chloro-6,7,7a,8,10,11-hexahydro-4H-pyrimidinyl[6',1':2,3]pyrimidinyl[6,1-c][1,4]oxaz in-4-one

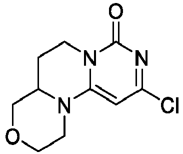

At the room temperature, 2-(4-(2,6-dichloropyrimidin-4-yl)morpholin-3-yl)ethan-1-ol (1.8 g, 6.58 mmol) was added to dichloromethane (20 mL), and sulfoxide chloride (1.43 mL) was added with stirring, and stirred at the room temperature for 1 hour. A reaction solution was concentrated to dryness, and potassium sodium carbonate (2.81 g, 20.34 mmol) and acetonitrile (20 mL) were sequentially added to residues, and stirred under reflux overnight. The reaction solution was filtered, a filtrate was concentrated, beating was conducted with ethyl acetate, and a filter cake was dried to obtain the title compound (0.91 g, 56%). LC-MS: m/z [M+H]⁺=242.

### Intermediate 11

### 2-((1S,4S)-5-(2,6-dichloro-5-methylpyrimidin-4-yl)-2-oxa-5-azabicyclo[2.2.1]heptane-4-yl)ethan-1-ol

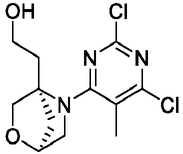

At the room temperature, 2,4,6-trichloro-5-methylpyrimidine (1.21 g, 6.15 mmol), 2-((1S,4S)-2-oxa-5-azabicyclo[2.2.1]heptane-4-yl)ethan-1-ol hydrochloride (0.88 g, 6.15 mmol), and sodium carbonate (1.96 g, 18.45 mmol) were sequentially added to acetonitrile (20 mL), and stirred overnight at the room temperature. Filtering was conducted, a filtrate was concentrated, and purification was conducted through column chromatography to obtain the title compound (470 mg, 25%). LC-MS: m/z [M+H]⁺=304.

### (7aS,10S)-2-chloro-1-methyl-6,7,10,11-tetrahydro-4H,8H-7a,10-methylaminopyrimidin e[6',1':2,3]pyrimidine[6,1-c][1,4]oxazin-4-one

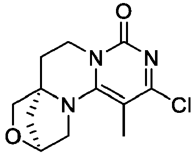

At the room temperature, 2-((1S,4R)-5-(2,6-dichloro-5-methylpyrimidin-4-yl)-2-oxa-5-azabicyclo[2.2.1]heptyl-4-yl)et hanol (140 mg, 0.46 mmol), methylsulfonic anhydride (130 mg, 0.74 mmol), and triethylamine (93 mg, 0.92 mmol) were sequentially added to dichloromethane (15 mL) and stirred at the room temperature for 1 hour. A reaction solution was concentrated, and potassium carbonate (140 mg, 1.35 mmol) and acetonitrile (10 mL) were sequentially added to residues, and stirred at 80°C overnight. The reaction solution was concentrated, and thin layer chromatography was prepared for isolation (dichloromethane/methanol=20/1) to obtain the title compound (50 mg, 41%). LC-MS: m/z [M+H]⁺=268.

### Intermediate 12

### 2-((1S,4R)-5-(2,5,6-trichloropyrimidin-4-yl)-2-oxa-5-azabicyclo[2.2.1]heptyl-4-yl)ethan ol

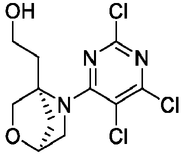

At the room temperature, tetrachloropyrimidine (300 mg, 1.38 mmol), 2-((1S,4S)-2-oxa-5-azabicyclo[2.2.1]heptane-4-yl)ethan-1-ol hydrochloride (600 mg, 3.34 mmol), and sodium carbonate (1.06 g, 10.02 mmol) were added to acetonitrile (20 mL), and stirred overnight at the room temperature. A reaction solution was filtered, a filtrate was concentrated, and isolation was conducted through column chromatography (petroleum ether/ethyl acetate=1/1) to obtain the title compound (200 mg, 18%). LC-MS: m/z [M+H]⁺=324.

### (7aS,10S)-1,2-dichloro-6,7,10,11-tetrahydro-4H,8H-7a,10-methylaminopyrimidine[6',1' :2,3]pyrimidine[6,1-c][1,4]oxazin-4-one

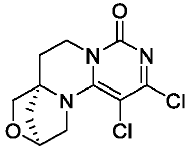

At the room temperature, 2-((1S,4S)-5-(2,5,6-trichloropyrimidin-4-yl)-2-oxa-5-azabicyclo[2.2.1]heptane-4-yl)ethan-1-ol (200 mg, 0.62 mmol) was added to dichlorosulfoxide (1 mL) and dichloromethane (4 mL), and stirred at the room temperature for 1 hour. A reaction solution was concentrated, and potassium carbonate (200 mg, 1.86 mmol) and acetonitrile (10 mL) were sequentially added to residues, and stirred at 80°C overnight. The reaction solution was concentrated, and thin layer chromatography was prepared for isolation (dichloromethane/methanol=20/1) to obtain the title compound (40 mg, 22%). LC-MS: m/z [M+H]⁺=288.

Referring to the table below, in addition to replacing corresponding raw materials with raw materials described in the column "raw material", the following intermediates were prepared with reference to the preparation method of the intermediate 12.

| **No. of interm ediate** | **Name of intermediate** | **Structural formula** | **LC-MS (m/z [M+H]⁺)** | **Raw material** |
|---|---|---|---|---|
| **13** | (7aS,10S)-2-c hloro-1-fluor o-6,7,10,11-t etrahydro-4H ,8H-7a,10-me thylaminopyr imidine[6',1': 2,3]pyrimidin e[6,1-c][1,4]o xazin-4-one | 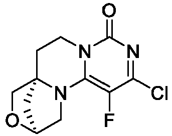 | 272 | 2,4,6-trichloro-5-fluoropyrimidine , 2-((1S,4S)-2-oxa -5-azabicyclo[2. 2.1]hept-4-yl)eth anol hydrochloride |
| **14** | (7aS,10S)-2-c hloro-1-ethyl -6,7,10,11-tet rahydro-4H,8 H-7a,10-meth ylaminopyri midine[6',1':2 ,3]pyrimidine [6,1-c][1,4]o xazin-4-one | 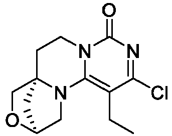 | 282 | 2,4,6-trichloro-5-ethylpyrimidine, 2-((1S,4S)-2-oxa -5-azabicyclo[2. 2.1]hept-4-yl)eth anol hydrochloride |
| **15** | (7aS,10S)-2-c hloro-1-meth oxy-6,7,10,11 -tetrahydro-4 H,8H-7a,10-methylamino pyrimidine[6' ,1':2,3]pyrimi dine[6,1-c][1, 4]oxazin-4-o ne | 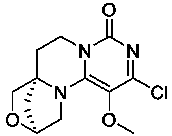 | 284 | 2,4,6-trichloro-5-methoxypyrimidi ne, 2-((1S,4S)-2-oxa -5-azabicyclo[2. 2.1]hept-4-yl)eth anol hydrochloride |
| **16** | 2-chloro-4-ox o-6,7,7a,8,9,1 0-hexahydro-4H-pyrimidin yl[1,6-a]pyrr olo[1,2-c]pyri midine-1-nitr ile | 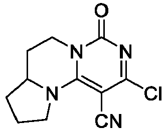 | 251 | 2,4,6-trichloro-5-cyanopyrimidine 2-(pyrrolidin-2-y l)ethan-1-ol hydrochloride |

### Intermediate 17

### (S)-2-(1-(2,6-dichloro-5-methoxypyrimidin-4-yl)piperidin-2-yl)ethan-1-ol

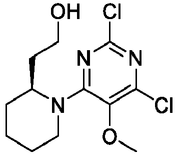

At the room temperature, 2,4,6-trichloro-5-methoxypyrimidine (300 mg, 1.41 mmol), (S)-2-(piperidin-2-yl)ethan-1-ol (180 mg, 1.41 mmol), and sodium carbonate (450 mg, 4.23 mmol) were added to acetonitrile (15 ml) and stirred at the room temperature for 2 hours. A reaction solution was filtered, a filtrate was concentrated, and thin layer chromatography was prepared for isolation (petroleum ether/ethyl acetate=2/1) to obtain the title compound (365 mg, 84%). LC-MS: m/z [M+H]⁺=306.

### (S)-2-chloro-1-methoxy-7,7a,8,9,10,11-hexahydro-4H,6H-pyridyl[1,2c]pyrimidinyl[1,6-a]pyrimidin-4-one

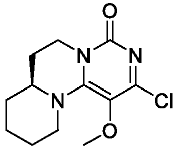

At the room temperature, (S)-2-(1-(2,6-dichloro-5-methoxypyrimidin-4-yl)piperidin-2-yl)ethan-1-ol (170 mg, 0.56 mmol) was added to dichlorosulfoxide (4 mL) and dichloromethane (8 ml), and stirred at the room temperature for 1 hour. A reaction solution was concentrated, and potassium carbonate (230 mg, 1.65 mmol) and acetonitrile (15 mL) were sequentially added to residues, and stirred at 80°C overnight. The reaction solution was concentrated, and thin layer chromatography was prepared for isolation (petroleum ether/ethyl acetate=2/1) to obtain the title compound (35 mg, 23%). LC-MS: m/z [M+H]⁺=270.

### Intermediate 18

### 5-formyl-2-(3-(trifluoromethyl)phenoxy)benzonitrile

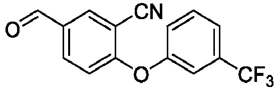

At the room temperature, 2-fluoro-5-formylbenzonitrile (15.0 g, 0.1 mol), 3-(trifluoromethyl)phenol (16.0 g, 0.1 mol), and potassium carbonate (13.8 g, 0.1 mol) were added to DMF (100 mL), and stirred at 105°C for 8 hours, a reaction solution was poured into water, extracted with dichloromethane and concentrated in the organic phase, ethanol (50 mL) was added to residues and stirred at the room temperature for 30 minutes, filtering was conducted, and a filter cake was concentrated to dryness to obtain the title compound (22.1 g, 76%). ¹H NMR (400MHz, DMSO-d₆) δ 9.96 (s, 1 H), 8.49 (s, 1 H), 8.14 (d, 1 H), 7.88 (d, 2 H), 7.47 (d, 2 H), 7.19 (d, 1 H).

### 5-(hydroxymethyl)-2-(3-(trifluoromethyl)phenoxy)benzonitrile

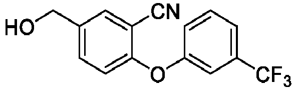

At the room temperature, 5-formyl-2-(3-(trifluoromethyl)phenoxy)benzonitrile (24.3 g, 83.44 mmol) was added to methanol (250 mL), and sodium borohydride (4.86 g, 127.9 mmol) was added in batches to react at the room temperature for 0.5 hours. A reaction solution was poured into water, extracted with dichloromethane, and concentrated in the organic phase to obtain the title compound (24.2 g, 99%). ¹H NMR (400 MHz, DMSO-d₆) δ 7.80 (s, 1 H), 7.73 - 7.53 (m, 3 H), 7.52 - 7.27 (m, 2 H), 7.09 (d, 1 H), 5.41 (s, 1 H), 4.51 (s, 2 H).

Referring to the table below, in addition to replacing corresponding raw materials with raw materials described in the column "raw material", the following intermediates were prepared with reference to the preparation method of the intermediate 19.

| **No. of interm ediate** | **Name of intermediate** | **Structural formula** | **¹H NMR (400 MHz)** | **Raw material** |
|---|---|---|---|---|
| **19** | (3,5-difluoro-4-((2-(trifluor omethyl)pyri d-4-yl)oxy)p henyl)methan ol | 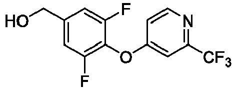 | DMSO-d₆: δ 8.68 (d, *J* = 5.9 Hz, 1 H), 7.60 (d, *J* = 2.4 Hz, 1 H), 7.32 (s, 1 H), 7.29 (s, 1 H), 7.27 (dd, *J* = 2.4, 5.9 Hz, 1 H), 5.57 (t, *J* = 5.6 Hz, 1 H), 4.57 (d, *J* = 5.4 Hz, 2 H) | 3,4,5-trifluoro benzaldehyde, 4-hydroxyl-2-(trifluorometh yl)pyridine |
| **20** | (3-fluoro-4-(( 2-(trifluorom ethyl)pyrid-4 -yl)oxy)phen yl)methanol | 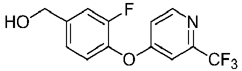 | DMSO-d₆: δ 8.64 (d, J = 5.4 Hz, 1 H), 7.41 (dd, J = 8.1, 14.4 Hz, 3 H), 7.28 (d, J = 8.3 Hz, 1 H), 7.16 (d, J = 3.4 Hz, 1 H), 5.44 (br. s., 1 H), 4.57 (br. s., 2 H). | 3,4-difluorobe nzaldehyde, 4-hydroxyl-2-(trifluorometh yl)pyridine |
| **21** | (4-(4-chloro-3-(trifluorom ethyl)phenox y)-3,5-difluor ophenyl)meth anol | 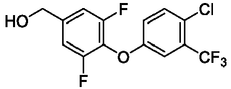 | DMSO-d₆: δ 7.70 (d, J = 8.8 Hz, 1 H), 7.47 (d, J = 2.0 Hz, 1 H), 7.27 (d, J = 9.3 Hz, 3 H), 5.53 (br. s., 1 H), 4.55 (d, J = 2.9 Hz, 2 H) | 3,4,5-trifluoro benzaldehyde, 4-chloro-3-(tri fluoromethyl) phenol |
| **22** | (3,5-difluoro-4-((2-methyl pyrid-4-yl)ox y)phenyl)met hanol | 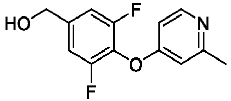 | CDCl₃: δ 8.40 - 8.26 (m, 1 H), 7.09 (d, J = 8.3 Hz, 2 H), 6.67 (s, 1 H), 6.70 (s, 1 H), 4.74 (br. s., 2 H), 2.52 (br. s., 3 H), 2.39 (br. s., 1 H) | 3,4,5-trifluoro benzaldehyde, 4-hydroxyl-2-methylpyridin e |
| **23** | (3,5-difluoro-4-((2-(trifluor omethyl)pyri midin-5-yl)o xy)phenyl)m ethanol | 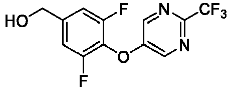 | CDCl₃: δ 8.61 (br. s., 1 H), 8.55 (br. s., 1 H), 7.42 (br. s., 1 H), 7.10 (d, *J* = 8.3 Hz, 2 H), 4.75 (br. s., 2 H), 2.47 (br. s., 1 H) | 3,4,5-trifluoro benzaldehyde, 2-(trifluorome thyl)pyrimidi ne-5-ol |
| **24** | (4-((2-chloro pyridyl-4-yl) oxy)-3,5-difl uorophenyl) methanol | 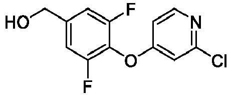 | CDCl₃: δ 8.28 (br. s., 1 H), 7.11 (d, J = 8.3 Hz, 2 H), 6.85 (br. s., 2 H), 4.85 - 4.67 (m, 2 H), 2.09 (br. s., 1 H) | 3,4,5-trifluoro benzaldehyde, 2-chloro-4-hy droxylpyridin e |

### Intermediate 25

### 4-(benzyloxy)-2-(trifluoromethoxy)pyridine

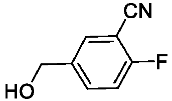

At the room temperature, 2-fluoro-5-formylbenzonitrile (3.00 g, 20.12 mmol) was added to tetrahydrofuran (15 mL) and methanol (15 mL), and sodium borohydride (0.76 g, 20.12 mmol) was added with stirring, and stirred at the room temperature for 0.5 hours. A reaction solution was quenched with a saturated ammonium chloride aqueous solution (10 mL), extracted with ethyl acetate, and concentrated in the organic phase to obtain the title compound (2.4 g, 79%). ¹H NMR (400 MHz, CDCl₃) δ 7.70 - 7.55 (m, 2 H), 7.25 - 7.15 (m, 1 H), 4.72 (s, 2 H).

### Intermediate 26

### 4-(benzyloxy)-2-(trifluoromethoxy)pyridine

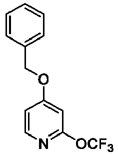

4-(benzyloxy)pyridine-2-ol (1.91 g, 9.48 mmol) and 1-trifluoromethyl-1,2-phenyliodyl-3(H)-one (1 g, 3.16 mmol) were added to nitromethane (25 mL) and stirred at 100°C overnight. A reaction solution was concentrated, and residues were purified by column chromatography (petroleum ether/ethyl acetate=10/1) to obtain the title compound (530 mg, 47%). LC-MS: m/z [M+H]⁺=270.

### 2-(trifluoromethoxy)pyridine-4-ol

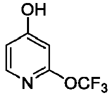

4-(benzyloxy)-2-(trifluoromethoxy)pyridine (530 mg, 1.97 mmol) and Pd/C (10%, 125 mg) were added to methanol (20 mL), and stirred overnight under hydrogen gas at 60°C and 3 bar pressure. A reaction solution was filtered, and a filtrate was concentrated to obtain a crude title compound (330 mg, 94%). LC-MS: m/z [M+H]⁺=180.

### (3,5-difluoro-4-((2-(trifluoromethoxy)pyrid-4-yl)oxy)phenyl)methanol

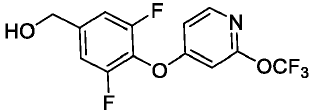

3,4,5-trifluorobenzaldehyde (294.4 mg, 1.84 mol), 2-(trifluoromethoxy)pyridine-4-ol (330 mg, 1.84 mol), and potassium carbonate (330.6 mg, 2.39 mol) were added to DMF (10 mL), and stirred at 120°C for 2 hours, a reaction solution was poured into water, extracted with ethyl acetate, and concentrated in the organic phase to obtain crude 3,5-difluoro-4-((2-(trifluoromethoxy)pyrid-4-yl)oxy)benzaldehyde, the crude was added to ethanol (250.0 mL), and sodium borohydride (69.61 mg, 1.84 mmol) was added and stirred at the room temperature for 1 hour. The reaction was quenched with water, extracted with ethyl acetate, and dried and concentrated in the organic phase to obtain the title compound (400 mg, 68%). ¹H NMR (400MHz, DMSO-*d*₆) δ 8.30 (d, J = 5.9 Hz, 1 H), 7.29 (d, J = 9.3 Hz, 2 H), 7.07 (d, J = 3.9 Hz, 1 H), 6.95 (s, 1 H), 5.56 (br. s., 1 H), 4.56 (d, J = 4.9 Hz, 2 H);LC-MS: m/z [M+H]⁺ =322.

### Intermediate 27

### 4-(4-chloro-3-(trifluoromethyl)phenoxy)benzaldehyde

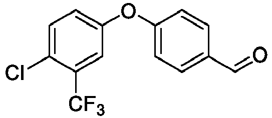

2-chloro-5-hydroxyltrifluoromethylbenzene (15 g, 76.53 mmol), 4-fluorobenzaldehyde (9.50 g, 76.53 mmol), and potassium carbonate (21.12 g, 153.06 mmol) were added to DMF (150 mL), and stirred at 120°C overnight, and a reaction solution was poured into water, extracted with ethyl acetate, concentrated in the organic phase, and purified by column chromatography (petroleum ether/ethyl acetate=50/1-5/1) to obtain the title compound (14.6 g, 64%). LC-MS: m/z [M+H]⁺=301.

### 1-chloro-2-(trifluoromethyl)-4-(4-vinylphenoxy)benzene

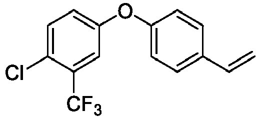

4-(4-chloro-3-(trifluoromethyl)phenoxy)benzaldehyde (14.6 g, 48.67 mmol) and methyltriphenyl phosphonium bromide (19.12 g, 53.53 mmol) were added to THF (150 mL), and cooled to 0°C under argon protection, and sodium hydride (60%, 9.73 g, 243.35 mmol) was slowly added with stirring, and stirred overnight at the room temperature. After being quenched with water, the reaction solution was poured into ice water, extracted with dichloromethane, concentrated in the organic phase, and purified by column chromatography (petroleum ether/ethyl acetate=50/1-5/1) to obtain the title compound (13.6 g, 94%). LC-MS: m/z [M+H]⁺=299.

### 2-(4-(4-chloro-3-(trifluoromethyl)phenoxy)phenyl)ethan-1-ol

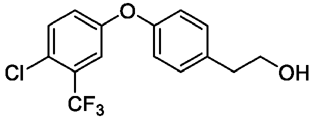

At the room temperature, 1-chloro-2-(trifluoromethyl)-4-(4-vinylphenoxy)benzene (13.6 g, 45.48 mmol) was dissolved in anhydrous tetrahydrofuran (150 mL), and cooled to 0°C, and a tetrahydrofuran solution of 9-borobicyclo[3.3.1]nonane (0.5 M, 136 mL, 68.00 mmol) and stirred overnight at the room temperature. Water (14 mL), a sodium hydroxide aqueous solution (3 M, 70 mL), and hydrogen peroxide (30%, 70 mL) were sequentially added to a reaction solution, and stirred at 50°C for 2 hours. The reaction solution was quenched with a saturated sodium thiosulfate aqueous solution and then poured into water, extracted with dichloromethane, concentrated in the organic phase, and purified by column chromatography (dichloromethane/methanol=30/1) to obtain the title compound (10.6 g, 74%). LC-MS: m/z [M+H]⁺=317.

### Intermediate 28

### 4-bromo-2-(difluoromethyl)pyridine

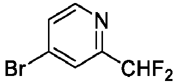

4-bromopyridine formaldehyde (10 g, 53.76 mmol) was added to dichloromethane (100 mL) and cooled to 0°C. Diethylaminosulfur trifluoride (17.3 g, 107.52 mmol) was dropped into a reaction solution with stirring, and stirred overnight at the room temperature. The reaction solution was slowly dropped into water, extracted with dichloromethane, and concentrated in the organic phase to obtain a crude title compound, which is directly used for the next step of reaction. LC-MS: m/z [M+H]⁺=208.

### 2-(difluoromethyl)-4-methoxypyridine

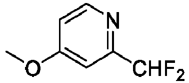

4-bromo-2-(difluoromethyl)pyridine (11.18 g, 53.76 mmol) and sodium methoxide (5.81 g, 107.52 mmol) were added to methanol (100 mL), and stirred at 90°C overnight. A reaction solution was cooled to the room temperature and poured into water (300 mL), and extracted with ethyl acetate. Concentration was conducted in the organic phase to obtain a crude title compound, which was directly used for the next step of reaction. LC-MS: m/z [M+H]⁺=160.

### 2-(difluoromethyl)pyridine-4-ol

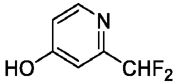

2-(difluoromethyl)-4-methoxypyridine (9.54 g, 59.95 mmol) was added to hydrobromic acid (40% aqueous solution, 58 mL), and stirred at 90°C for 2 days. A reaction solution was concentrated, residues were diluted with water, sodium bicarbonate solid was added with stirring until there were no bubbles produced in the system, extraction was conducted with ethyl acetate, concentration was conducted in the organic phase, and purification was conducted through column chromatography (petroleum ether/ethyl acetate=10/1-1/1) to obtain the title compound (2.5 g, three-step total yield 26%). LC-MS: m/z [M+H]⁺=146.

### 4-((2-(difluoromethyl)pyrid-4-yl)oxy)-3-fluorobenzaldehyde

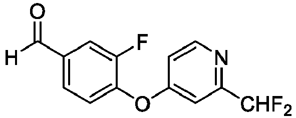

3,4-difluorobenzaldehyde (350 mg, 2.5 mmol), 2-(difluoromethyl)carbonate-4-ol (357 mg, 2.5 mmol), and potassium carbonate solid (1020 mg, 7.4 mmol) were added to N,N-dimethylformamide (5 mL), and stirred at 100°C overnight. Purification was conducted through column chromatography (petroleum ether/ethyl acetate=15/1) to obtain the title compound (510 mg, 77%). LC-MS: m/z [M+H]⁺=268.

### (4-((2-(difluoromethyl)pyrid-4-yl)oxy)-3-fluorophenyl)methanol

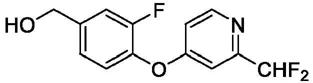

4-((2-(difluoromethyl)pyrid-4-yl)oxy)-3-fluorobenzaldehyde (510 mg, 1.9 mmol) was added to anhydrous ethanol (5 mL), and NaBH₄ (140 mg, 3.8 mmol) was added with stirring, and stirred at the room temperature for 1 hour. A reaction solution was poured into water, extracted with dichloromethane, and concentrated in the organic phase to obtain the title compound (381 mg, 75%). LC-MS: m/z [M+H]⁺=270.

### Example

### Example 1

### Method A

### (7aS,10S)-2-((3-fluoro-4-((2-(trifluoromethyl)pyrid-4-yl)oxy)benzyl)oxy)-6,7,10,11-tetra hydro-4H,8H-7a,10-methanopyrimido[6',1':2,3]pyrimido[6,1-c][1,4]oxazin-4-one

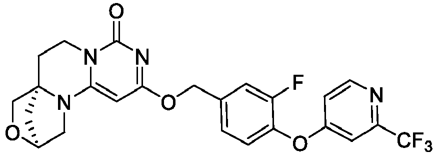

At the room temperature, (7aS,10S)-2-chloro-6,7,10,11-tetrahydro-4H,8H-7a,10-methanopyrimido[6',1':2,3]pyrimidin e[6,1-c][1,4]oxazin-4-one (149.67 mg, 0.59 mmol), (3-fluoro-4-((2-(trifluoromethyl)pyrid-4-yl)oxy)phenyl)methanol (170 mg, 0.59 mmol), and cesium carbonate (580 mg, 1.77 mmol) were added to toluene (10 mL), and stirred under reflux under argon protection overnight. After being cooled to the room temperature, a reaction solution was filtered, a filtrate was concentrated, and thin layer chromatography was prepared for isolation (dichloromethane/methanol=20/1) to obtain the title compound (85 mg, 28%). ¹H NMR (400 MHz, CDCl₃) δ 8.57 (d, J = 5.9 Hz, 1 H), 7.33 (d, J = 10.8 Hz, 1 H), 7.27 - 7.22 (m, 2 H), 7.21 - 7.15 (m, 1 H), 6.96 (d, J = 3.9 Hz, 1 H), 5.43 (s, 2 H), 4.95 (s, 1 H), 4.89 - 4.81 (m, 1 H), 4.79 (s, 1 H), 3.97 (d, J = 7.3 Hz, 1 H), 3.74 (d, J = 6.8 Hz, 1 H), 3.52 - 3.35 (m, 2 H), 3.24 (d, J = 3.9 Hz, 1 H), 2.48 - 2.38 (m, 1 H), 2.36 - 2.22 (m, 2 H), 1.74 (d, J = 10.3 Hz, 1 H);LC-MS: m/z [M+H]⁺ =505.

### Example 2

### Method B

### (7aS,10S)-2-(((4-(4-chloro-3-(trifluoromethyl)phenoxy)-3-fluorobenzyl)oxy)-6,7,10,11-te trahydro-4H,8H-7a,10-methanopyrimido[6',1':2,3]pyrimido[6,1-c][1,4]oxazin-4-one

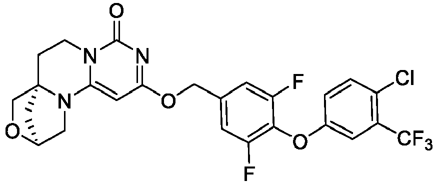

At the room temperature, (7aS,10S)-2-chloro-6,7,10,11-tetrahydro-4H,8H-7a,10-methylaminopyrimidine[6',1':2,3]pyri midine[6,1-c][1,4]oxazin-4-one (100 mg, 0.39 mmol) and (4-(4-chloro-3-(trifluoromethyl)phenoxy)-3,5-difluorophenyl)methanol (160 mg, 0.47 mmol) were added to acetonitrile (7 mL), and sodium hydride (11 mg, 0.47 mmol) was added with stirring, and stirred at the room temperature for 1 hour. A reaction solution was quenched with a saturated ammonium chloride aqueous solution and extracted with ethyl acetate. Concentration was conducted in the organic phase, and thin layer chromatography was prepared for isolation (dichloromethane/methanol=20/1) to obtain the title compound (82 mg, 37%). ¹H NMR (400 MHz, CDCl₃) δ 7.40 (d, J = 8.8 Hz, 1 H), 7.34 - 7.23 (m, 1 H), 7.08 (d, J = 8.3 Hz, 2 H), 7.00 (d, J = 8.3 Hz, 1 H), 5.38 (br. s., 2 H), 4.94 (s, 1 H), 4.87 - 4.70 (m, 2 H), 3.96 (d, J = 6.8 Hz, 1 H), 3.73 (d, J = 6.8 Hz, 1 H), 3.51 - 3.32 (m, 2 H), 3.28 - 3.14 (m, 1 H), 2.47 - 2.37 (m, 1 H), 2.33 - 2.19 (m, 2 H), 1.73 (d, J = 9.8 Hz, 1 H);LC-MS: m/z [M+H]⁺ =556.

Examples 3-64 listed in the table below were prepared through steps similar to those of Examples 1-2, and started with corresponding intermediates:

| **Example #** | **Name** | **Structure** | **Use method** | **m/z** | **¹H NMR (400 MHz)** |
|---|---|---|---|---|---|
| 3 | (7aS,10S)-2-(((3,5 -difluoro-4-((2-(tri fluoromethyl)pyri d-4-yl)oxy)benzyl )oxy)-6,7,10,11-te trahydro-4H,8H-7 a,10-methanopyri mido[6',1':2,3]pyr imido[6,1-c][1,4]o xazin-4-one | 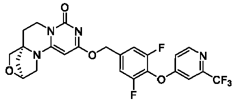 | B | 523 | CDCl₃: δ 8.61 (d, J = 5.4 Hz, 1 H), 7.14 (d, J = 8.3 Hz, 2 H), 6.99 (d, J = 3.9 Hz, 1 H), 5.43 (d, J = 2.9 Hz, 2 H), 4.96 (s, 1 H), 4.88 - 4.79 (m, 2 H), 3.98 (d, J = 6.8 Hz, 1 H), 3.75 (d, J = 7.3 Hz, 1 H), 3.49 - 3.44 (m, 1 H), 3.44 - 3.39 (m, 1 H), 3.48 - 3.39 (m, 1 H), 3.25 (d, J = 3.9 Hz, 1 H), 2.42 (br. s., 1 H), 2.36 - 2.24 (m, 2 H), 1.75 (d, J = 9.8 Hz, 1 H) |
| 4 | (7aS,10S)-2-(((3,5 -difluoro-4-((2-me thylpyridyl-4-yl)o xy)benzyl)oxy)-6, 7,10,11-tetrahydro -4H,8H-7a,10-met hanopyrimido[6',1 ':2,3]pyrimido[6,1 -c][1,4]oxazin-4-o ne | 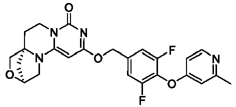 | B | 469 | CDCl₃: δ 8.36 (d, J = 5.4 Hz, 1 H), 7.09 (d, J = 7.8 Hz, 2 H), 6.75 - 6.63 (m, 2 H), 5.48 - 5.35 (m, 2 H), 4.95 (s, 1 H), 4.89 - 4.71 (m, 2 H), 3.98 (d, J = 7.3 Hz, 1 H), 3.74 (d, J = 6.8 Hz, 1 H), 3.53 - 3.37 (m, 2 H), 3.33 - 3.18 (m, 1 H), 2.52 (s, 3 H), 2.43 (d, J = 13.7 Hz, 1 H), 2.36 - 2.23 (m, 2 H), 1.76 (br. s., 1 H) |
| 5 | 5-((((((7aS,10S)-4 -one-6,7,10,11-tet rahydro-4H,8H-7a ,10-methanopyrim ido[6',1':2,3]pyri mido[6,1-c][1,4]o xazin-2-yl)oxy)m ethyl)-2-(3-(triflu oromethyl)phenox y)benzonitrile | 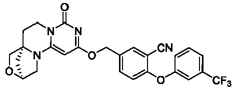 | B | 511 | CDCl₃: δ 7.73 (s, 1 H), 7.51 (dt, J = 8.1, 19.4 Hz, 3 H), 7.32 (br. s., 1 H), 7.24 (d, J = 7.3 Hz, 1 H), 6.89 (d, J = 8.3 Hz, 1 H), 5.39 (d, J = 7.3 Hz, 2 H), 4.92 (s, 1 H), 4.87 - 4.76 (m, 2 H), 3.96 (d, J = 7.3 Hz, 1 H), 3.73 (d, J = 7.3 Hz, 1 H), 3.51 - 3.35 (m, 2 H), 3.23 (d, J = 2.9 Hz, 1 H), 2.46 - 2.37 (m, 1 H), 2.32 - 2.21 (m, 2 H), 1.74 (d, J = 10.3 Hz, 1 H) |
| 6 | (7aS,10S)-2-(((4-( (2-chloropyrid-4-yl)oxy)-3,5-difluo robenzyl)oxy)-6,7 ,10,11-tetrahydro-4H,8H-7a,10-met hanopyrimido[6',1 ':2,3]pyrimido[6,1 -c][1,4]oxazin-4-o ne | 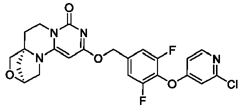 | B | 489 | CDCl₃: δ 8.27 (d, J = 5.9 Hz, 1 H), 7.21 - 7.07 (m, 2 H), 6.91 - 6.70 (m, 2 H), 5.48 - 5.34 (m, 2 H), 4.96 (s, 1 H), 4.87 - 4.68 (m, 2 H), 3.98 (d, J = 7.3 Hz, 1 H), 3.75 (d, J = 7.3 Hz, 1 H), 3.50 - 3.36 (m, 2 H), 3.32 - 3.16 (m, 1 H), 2.50 - 2.39 (m, 1 H), 2.35 - 2.22 (m, 2 H), 1.75 (d, J = 9.8 Hz, 1 H) |
| 7 | (7aS,10S)-2-(((3,5 -difluoro-4-((2-(tri fluoromethoxy)py rid-4-yl)oxy)benz yl)oxy)-6,7,10,11-tetrahydro-4H,8H -7a,10-methanopy rimido[6',1':2,3]p yrimidine[6,1-c][1 ,4]oxazin-4-one | 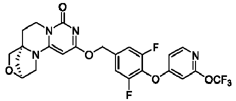 | B | 539 | CDCl₃: δ 8.21 (d, J = 5.9 Hz, 1 H), 7.12 (d, J = 8.3 Hz, 2 H), 6.82 (d, J = 5.4 Hz, 1 H), 6.50 (s, 1 H), 5.56 - 5.33 (m, 2 H), 4.96 (s, 1 H), 4.89 - 4.66 (m, 2 H), 3.98 (d, J = 7.3 Hz, 1 H), 3.75 (d, J = 6.8 Hz, 1 H), 3.56 - 3.35 (m, 2 H), 3.25 (dt, J = 3.4, 13.7 Hz, 1 H), 2.55 - 2.38 (m, 1 H), 2.37 - 2.23 (m, 2 H), 1.75 (d, J = 9.8 Hz, 1 H) |
| 8 | (7aS,10S)-2-(((4-( (2-(difluoromethy l)pyrid-4-yl)oxy)-3-fluorobenzyl)ox y)-6,7,10,11-tetra hydro-4H,8H-7a,1 0-methanopyrimid o[6',1':2,3]pyrimi dinyl[6,1-c][1,4]o xazin-4-one | 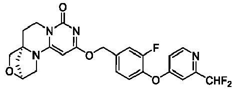 | A | 487 | CDCl₃: δ 8.50 (d, J = 5.8 Hz, 1 H), 7.32 (dd, J = 1.9, 10.7 Hz, 1 H), 7.27 - 7.22 (m, 1 H), 7.20 - 7.13 (m, 2 H), 6.91 (dd, J = 2.2, 5.5 Hz, 1 H), 6.74 - 6.44 (m, 1 H), 5.42 (d, J = 1.6 Hz, 2 H), 4.94 (s, 1 H), 4.87 - 4.76 (m, 2 H), 3.97 (d, J = 6.9 Hz, 1 H), 3.73 (d, J = 7.1 Hz, 1 H), 3.47 - 3.36 (m, 2 H), 3.24 (dt, J = 3.6, 13.7 Hz, 1 H), 2.46 - 2.38 (m, 1 H), 2.35 - 2.23 (m, 2 H), 1.74 (d, J = 10.2 Hz, 1 H) |
| 9 | (7aS,10S)-2-(((3,5 -difluoro-4-((2-(tri fluoromethyl)pyri midin- 5 -yl)oxy)be nzyl)oxy)-6,7,10,1 1-tetrahydro-4H,8 H-7a,10-methano pyrimido[6',1':2,3 ]pyrimido[6,1-c] [ 1,4]oxazin-4-one | 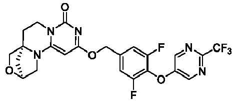 | B | 524 | CDCl₃: δ 8.54 (s, 2 H), 7.14 (d, J = 8.3 Hz, 2 H), 5.41 (br. s., 2 H), 4.96 (s, 1 H), 4.86 - 4.75 (m, 2 H), 3.97 (d, J = 6.8 Hz, 1 H), 3.74 (br. s., 1 H), 3.51 - 3.36 (m, 2 H), 3.23 (br. s., 1 H), 2.47 - 2.38 (m, 1 H), 2.35 - 2.22 (m, 2 H), 1.74 (d, J = 10.3 Hz, 1 H) |
| 10 | 2-fluoro-5-(((((((7 aS,10S)-4-one-6,7 ,10,11-hexahydro-4H,8H-7a,10-met hanopyrimido[6',1 ':2,3]pyrimido[6,1 -c][1,4]oxazin-2-y l)oxy)methyl)benz onitrile | 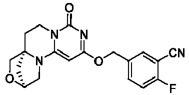 | A | 369 | CDCl₃: δ 7.75 - 7.60 (m, 2 H), 7.20 (t, J = 8.6 Hz, 1 H), 5.45 - 5.33 (m, 2 H), 4.92 (s, 1 H), 4.87 - 4.77 (m, 2 H), 3.97 (d, J = 7.3 Hz, 1 H), 3.74 (d, J = 7.3 Hz, 1 H), 3.48 - 3.36 (m, 2 H), 3.24 (dt, J = 3.9, 13.7 Hz, 1 H), 2.47 - 2.39 (m, 1 H), 2.35 - 2.24 (m, 2 H), 1.75 (d, J = 10.3 Hz, 1 H) |
| 11 | (7aS,10S)-2-(4-(4 -chloro-3-(trifluor omethyl)phenoxy) phenylethoxy) -6,7 ,10,11-tetrahydro-4H,8H-7a,10-met hanopyrimido[6',1 ':2,3]pyrimido[6,1 -c][1,4]oxazin-4-o ne | 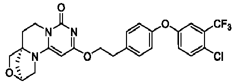 | B | 534 | CDCl₃: δ 7.40 (d, J = 8.8 Hz, 1 H), 7.32 - 7.22 (m, 3 H), 7.03 (dd, J = 2.4, 8.8 Hz, 1 H), 6.93 (d, J = 8.3 Hz, 2 H), 4.85 - 4.73 (m, 3 H), 4.54 (t, J = 6.8 Hz, 2 H), 3.93 (d, J = 7.3 Hz, 1 H), 3.71 (d, J = 6.8 Hz, 1 H), 3.44 - 3.30 (m, 2 H), 3.20 (dt, J = 3.7, 13.8 Hz, 1 H), 3.00 (t, J = 6.8 Hz, 2 H), 2.44 - 2.35 (m, 1 H), 2.32 - 2.19 (m, 2 H), 1.71 (d, J = 9.8 Hz, 1 H) |
| 12 | 2-((3,5-difluoro-4 -((2-(trifluoromet hyl)pyrid-4-yl)ox y)benzyl)oxy)-6,7 ,9,10-tetrahydro-4 H,8H-7a,9-metha nopyrimido[1,6-a] pyrrolo[1,2-c]pyri midine-4-one | 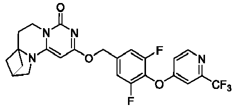 | B | 507 | CDCl₃: δ 8.58 (br. s., 1 H), 7.25 (br. s., 1 H), 7.12 (d, J = 8.8 Hz, 2 H), 6.97 (br. s., 1 H), 5.40 (br. s., 2 H), 5.04 (br. s., 1 H), 3.98 (br. s., 2 H), 3.38 (br. s., 2 H), 3.01 (br. s., 1 H), 2.26 (br. s., 2 H), 1.98 (br. s., 2 H), 1.60 (br. s., 2 H) |
| 13 | 2-((3-fluoro-4-((2-(trifluoromethyl)p yrid-4-yl)oxy)ben zyl)oxy)-6,7,9,10-tetrahydro-4H,8H -7a,9-methanopyri mido[1,6-a]pyrrol o[1,2-c]pyrimidin e-4-one | 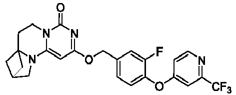 | B | 489 | CDCl₃: δ 8.55 (d, J = 5.4 Hz, 1 H), 7.32 (d, J = 10.8 Hz, 1 H), 7.25 (d, J = 9.3 Hz, 1 H), 7.21 (br. s., 1H), 7.19 - 7.12 (m, 1 H), 6.98 - 6.89 (m, 1 H), 5.41 (br. s., 2 H), 5.03 (s, 1 H), 3.98 (t, J = 5.1 Hz, 2 H), 3.36 (br. s., 2 H), 3.00 (br. s., 1 H), 2.25 (t, J = 5.1 Hz, 2 H), 1.97 (br. s., 2 H), 1.59 (br. s., 2 H) |
| 14 | 2-((4-(4-chloro-3-(trifluoromethyl)p henoxy)-3,5-diflu orobenzyl)oxy)-6, 7,9,10-tetrahydro-4H,8H-7a,9-meth anopyrimido[1,6-a]pyrrolo[1,2-c]py rimidine-4-one | 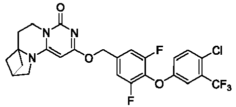 | B | 540 | CDCl₃: δ 7.45 - 7.34 (m, 1 H), 7.28 (br. s., 1 H), 7.12 - 7.03 (m, 2 H), 6.99 (br. s., 1 H), 5.37 (br. s., 2 H), 5.02 (br. s., 1 H), 3.97 (br. s., 2 H), 3.36 (br. s., 2 H), 3.00 (br. s., 1 H), 2.25 (br. s., 2 H), 1.97 (br. s., 2 H), 1.59 (br. s., 2 H) |
| 15 | 2-((3,5-difluoro-4 -((2-methylpyrid-4-yl)oxy)benzyl)o xy)-6,7,9,10-tetra hydro-4H,8H-7a,9 -methanopyrimido [1,6-a]pyrrolo[1,2 -c]pyrimidine-4-o ne | 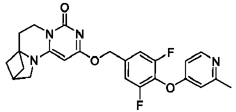 | B | 453 | CDCl₃: δ 8.32 (d, J = 5.5 Hz, 1 H), 7.06 (d, J = 8.2 Hz, 2 H), 6.69 - 6.58 (m, 2 H), 5.36 (s, 2 H), 5.00 (s, 1 H), 4.01 - 3.89 (m, 2 H), 3.35 (s, 2 H), 2.98 (br. s., 1 H), 2.47 (s, 3 H), 2.26 - 2.20 (m, 2 H), 1.95 (br. s., 2 H), 1.59 - 1.54 (m, 2 H) |
| 16 | 5-((((4-one-6,7,9, 10-hexahydro-4H, 8H-7a,9-methano pyrimido[1,6-a]py rrolo[1,2-c]pyrimi din-2-yl)oxy)meth yl)-2-(3-(trifluoro methyl)phenoxy)b enzonitrile | 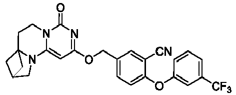 | B | 495 | CDCl₃: δ 7.75 (s, 1 H), 7.57 (d, J = 8.8 Hz, 1 H), 7.52 (d, J = 7.8 Hz, 1 H), 7.49 - 7.45 (m, 1 H), 7.33 (br. s., 1 H), 7.25 (d, J = 8.3 Hz, 1 H), 6.89 (d, J = 8.8 Hz, 1 H), 5.39 (s, 2 H), 5.02 (s, 1 H), 3.99 (t, J = 5.6 Hz, 2 H), 3.38 (s, 2 H), 3.02 (br. s., 1 H), 2.27 (t, J = 5.6 Hz, 2 H), 1.99 (br. s., 2 H), 1.73 (br. s., 2 H) |
| 17 | 2-(((4-((2-chlorop yrid-4-yl)oxy)-3,5 -difluorophenyl)o xy)-6,7,9,10-tetra hydro-4H,8H-7a,9 -methanopyrimido [1,6-a]pyrrolo[1,2 -c]pyrimidine-4-o ne | 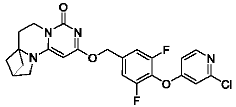 | B | 473 | CDCl₃: δ 8.24 (d, J = 5.9 Hz, 1 H), 7.09 (d, J = 8.8 Hz, 2 H), 6.86 - 6.74 (m, 2 H), 5.38 (s, 2 H), 5.03 (s, 1 H), 3.97 (t, J = 5.9 Hz, 2 H), 3.36 (s, 2 H), 3.00 (br. s., 1 H), 2.25 (t, J = 5.6 Hz, 2 H), 1.97 (br. s., 2 H), 1.63 - 1.57 (m, 2 H) |
| 18 | 2-((3-fluoro-4-((2-(trifluoromethoxy )pyrid-4-yl)oxy)b enzyl)oxy)-6,7,9,1 0-tetrahydro-4H,8 H-7a,9-methanop yrimido[1,6-a]pyr rolo[1,2-c]pyrimi dine-4-one | 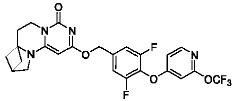 | B | 523 | CDCl₃: δ 8.17 (d, J = 5.9 Hz, 1 H), 7.09 (d, J = 8.3 Hz, 2 H), 6.79 (d, J = 4.9 Hz, 1 H), 6.47 (s, 1 H), 5.38 (s, 2 H), 5.02 (s, 1 H), 3.96 (t, J = 5.6 Hz, 2 H), 3.36 (s, 2 H), 2.99 (br. s., 1 H), 2.24 (t, J = 5.6 Hz, 2 H), 1.96 (br. s., 2 H), 1.58 (d, J = 3.4 Hz, 2 H) |
| 19 | 2-((4-((2-(difluoro methyl)pyrid-4-yl )oxy)-3-fluoroben zyloxy)-6,7,9,10-t etrahydro-4H,8H-7a,9-methanopyrimido[1,6-a]pyrrol o[1,2-c]pyrimidin e-4-one | 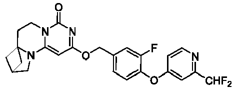 | A | 471 | CDCl₃: δ 8.65 - 8.43 (m, 1 H), 7.33 - 7.24 (m, 2 H), 7.20 - 7.08 (m, 2 H), 6.90 (br. s., 1 H), 5.40 (br. s., 2 H), 5.02 (br. s., 1 H), 4.11 - 3.92 (m, 2 H), 3.36 (br. s., 2 H), 3.00 (br. s., 1 H), 2.41 - 2.21 (m, 2 H), 1.97 (br. s., 2 H), 1.59 (br. s., 2 H) |
| 20 | 2-((3,5-difluoro-4 -((2-(trifluoromet hyl)pyrimidin-5-y l)oxy)benzyl)oxy) -6,7,9,10-tetrahyd ro-4H,8H-7a,9-me thanopyrimido[1, 6-a]pyrrolo[1,2-c] pyrimidine-4-one | 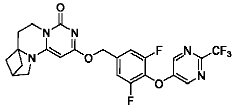 | B | 508 | CDCl₃: δ 8.53 (s, 2 H), 7.13 (d, J = 8.3 Hz, 2 H), 5.39 (s, 2 H), 5.02 (s, 1 H), 3.97 (t, J = 5.9 Hz, 2 H), 3.37 (s, 2 H), 3.00 (br. s., 1 H), 2.24 (d, J = 5.9 Hz, 2 H), 1.97 (br. s., 2 H), 1.59 (d, J = 3.9 Hz, 2 H) |
| 21 | 2-fluoro-5-(((((4-o ne-6,7,9,10-tetrah ydro-4H,8H-7a,9-methanopyrimido[ 1,6-a]pyrrolo[1,2-c]pyrimido-2-yl)o xy)methyl)benzon itrile | 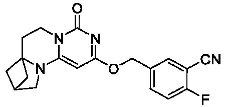 | A | 353 | CDCl₃: δ 7.71 - 7.56 (m, 2 H), 7.17 (t, J = 8.7 Hz, 1 H), 5.36 (s, 2 H), 4.98 (s, 1 H), 4.02 - 3.91 (m, 2 H), 3.36 (s, 2 H), 3.00 (t, J = 2.9 Hz, 1 H), 2.28 - 2.19 (m, 2 H), 1.97 (br. s., 2 H), 1.59 (dd, J = 1.9, 4.7 Hz, 2 H) |
| 22 | 2-(4-(4-chloro-3-( trifluoromethyl)ph enoxy)phenyletho xy)-6,7,9,10-tetra hydro-4H,8H-7a,9 -methanopyrimido [1,6-a]pyrrolo[1,2 -c]pyrimidine-4-o ne | 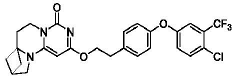 | B | 518 | CDCl₃: δ 7.40 (d, J = 8.8 Hz, 1 H), 7.29 (br. s., 1 H), 7.26 - 7.21 (m, 2 H), 7.03 (d, J = 7.3 Hz, 1 H), 6.93 (d, J = 7.8 Hz, 2 H), 4.94 (s, 1 H), 4.59 - 4.50 (m, 2 H), 3.96 (br. s., 2 H), 3.36 (br. s., 2 H), 2.99 (d, J = 6.8 Hz, 3 H), 2.23 (br. s., 2 H), 1.95 (br. s., 2 H), 1.58 (br. s., 2 H) |
| 23 | (7aR,10R)-2-(((3, 5-difluoro-4-((2-(t rifluoromethyl)py rid-4-yl)oxy)benz yl)oxy)-6,7,10,11-tetrahydro-4H,8H -7a,10-methanopy rimido[6',1':2,3]p yrimidine[6,1-c][1 ,4]oxazin-4-one | 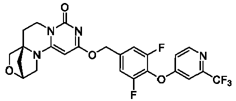 | B | 523 | CDCl₃: δ 8.57 (d, J = 5.4 Hz, 1 H), 7.23 (br. s., 1 H), 7.10 (d, J = 8.3 Hz, 2 H), 6.99 - 6.92 (m, 1 H), 5.39 (br. s., 2 H), 4.93 (s, 1 H), 4.84 - 4.73 (m, 2 H), 3.94 (d, J = 7.3 Hz, 1 H), 3.71 (d, J = 6.8 Hz, 1 H), 3.48 - 3.34 (m, 2 H), 3.28 - 3.15 (m, 1 H), 2.46 - 2.36 (m, 1 H), 2.32 - 2.20 (m, 2 H), 1.72 (d, J = 10.3 Hz, 1 H) |
| 24 | (7aR,10R)-2-(((4-(4-chloro-3-(triflu oromethyl)phenox y)-3,5-difluoroben zyl)oxy)-6,7,10,11 -tetrahydro-4H,8 H-7a,10-methano pyrimido[6',1':2,3 ]pyrimido[6,1-c] [ 1,4]oxazin-4-one | 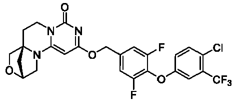 | B | 556 | CDCl₃: δ 7.38 (d, J = 8.8 Hz, 1 H), 7.27 (br. s., 1 H), 7.06 (d, J = 8.3 Hz, 2 H), 6.98 (d, J = 7.8 Hz, 1 H), 5.42 - 5.32 (m, 2 H), 4.93 (s, 1 H), 4.82 (br. s., 1 H), 4.77 (br. s., 1 H), 3.95 (d, J = 6.8 Hz, 1 H), 3.72 (d, J = 6.8 Hz, 1 H), 3.47 - 3.34 (m, 2 H), 3.21 (t, J = 12.5 Hz, 1 H), 2.45 - 2.35 (m, 1 H), 2.31 - 2.21 (m, 2 H), 1.72 (d, J = 9.8 Hz, 1 H) |
| 25 | 5-((((((7aR,10R)-4-one-6,7,10,11-te trahydro-4H,8H-7 a,10-methanopyri mido[6',1':2,3]pyr imido[6,1-c][1,4]o xazin-2-yl)oxy)methyl)-2-(3-(triflu oromethyl)phenox y)benzonitrile | 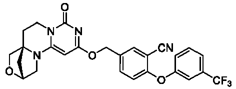 | B | 511 | CDCl₃: δ 7.69 (s, 1 H), 7.55 - 7.46 (m, 2 H), 7.45 - 7.39 (m, 1 H), 7.28 (br. s., 1 H), 7.21 (d, J = 7.8 Hz, 1 H), 6.85 (d, J = 8.8 Hz, 1 H), 5.40 - 5.28 (m, 2 H), 4.88 (s, 1 H), 4.81 -4.71 (m, 2 H), 3.92 (d, J = 7.3 Hz, 1 H), 3.69 (d, J = 7.3 Hz, 1 H), 3.44 - 3.31 (m, 2 H), 3.22 - 3.13 (m, 1 H), 2.42 - 2.34 (m, 1 H), 2.30 - 2.19 (m, 2 H), 1.70 (d, J = 9.8 Hz, 1 H) |
| 26 | (7aR,10R)-2-(((3, 5-difluoro-4-((2-(t rifluoromethoxy)p yrid-4-yl)oxy)ben zyl)oxy)-6,7,10,11 -tetrahydro-4H,8 H-7a,10-methano pyrimido[6',1':2,3 ]pyrimido[6,1-c][ 1,4]oxazin-4-one | 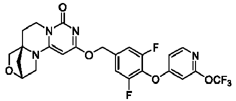 | B | 539 | CDCl₃: δ 8.15 (d, J = 5.9 Hz, 1 H), 7.07 (d, J = 8.3 Hz, 2 H), 6.77 (d, J = 4.9 Hz, 1 H), 6.45 (s, 1 H), 5.42 - 5.30 (m, 2 H), 4.91 (s, 1 H), 4.83 - 4.70 (m, 2 H), 3.93 (d, J = 6.8 Hz, 1 H), 3.70 (d, J = 6.8 Hz, 1 H), 3.46 - 3.32 (m, 2 H), 3.20 (dt, J = 3.4, 13.7 Hz, 1 H), 2.44 - 2.35 (m, 1 H), 2.30 - 2.19 (m, 2 H), 1.70 (d, J = 9.8 Hz, 1 H) |
| 27 | (7aR,10R)-2-(((3, 5-difluoro-4-((2-(t rifluoromethyl)py rimidin-5-yl)oxy) benzyl)oxy)-6,7,1 0,11-tetrahydro-4 H,8H-7a,10-meth anopyrimido[6',1': 2,3]pyrimido[6,1-c][1,4]oxazin-4-o ne | 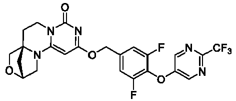 | B | 524 | CDCl₃: δ 8.58 (s, 2 H), 7.18 - 7.16 (m, 1 H), 7.15 - 7.13 (m, 1 H), 5.46 - 5.42 (m, 2 H), 5.00 - 4.96 (m, 1 H), 4.91 - 4.84 (m, 1 H), 4.84 - 4.80 (m, 1 H), 4.10 - 3.94 (m, 1 H), 3.77 (d, J = 7.1 Hz, 1 H), 3.55 - 3.38 (m, 2 H), 3.31 - 3.20 (m, 1 H), 2.54 - 2.42 (m, 1 H), 2.38 - 2.27 (m, 2 H), 1.82 - 1.73 (m, 1 H) |
| 28 | (7aR,10R)-2-(4-(4 -chloro-3-(trifluor omethyl)phenoxy) phenylethoxy) -6,7 ,10,11-tetrahydro-4H,8H-7a,10-met hanopyrimido[6',1 ':2,3]pyrimido[6,1 -c][1,4]oxazin-4-o ne | 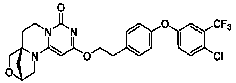 | B | 534 | CDCl₃: δ 7.40 (d, J = 8.8 Hz, 1 H), 7.28 (d, J = 2.5 Hz, 1 H), 7.26 (s, 1 H), 7.23 (s, 1 H), 7.03 (dd, J = 2.6, 8.7 Hz, 1 H), 6.93 (d, J = 8.5 Hz, 2 H), 4.85 - 4.72 (m, 3 H), 4.54 (t, J = 6.7 Hz, 2 H), 3.93 (d, J = 7.1 Hz, 1 H), 3.71 (d, J = 7.1 Hz, 1 H), 3.45 - 3.31 (m, 2 H), 3.20 (dt, J = 3.2, 13.7 Hz, 1 H), 3.00 (t, J = 6.6 Hz, 2 H), 2.44 - 2.34 (m, 1 H), 2.30 - 2.19 (m, 2 H), 1.69 (br. s., 1 H) |
| 29 | 2-((3,5-difluoro-4 -((2-(trifluoromet hyl)pyrid-4-yl)ox y)benzyl)oxy)-6,7 ,7a,8,9,10-hexahy dro-4H-pyrimidin yl[1,6-a]pyrrolo[1 ,2-c]pyrimidine-4-one | 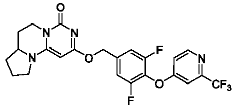 | B | 495 | CDCl₃: δ 8.77 - 8.49 (m, 1 H), 7.29 (br. s., 1 H), 7.22 - 7.09 (m, 2 H), 7.06 - 6.95 (m, 1 H), 5.52 - 5.37 (m, 2 H), 5.05 - 4.96 (m, 1 H), 4.73 - 4.61 (m, 1 H), 3.66 - 3.50 (m, 1 H), 3.49 - 3.26 (m, 3 H), 2.50 - 2.36 (m, 1 H), 2.36 - 2.14 (m, 2 H), 2.12 - 1.95 (m, 1 H), 1.60 - 1.47 (m, 2 H) |
| 30 | 2-((4-((2-chloropy ridyl-4-yl)oxy)-3, 5-difluorobenzyl) oxy)-6,7,7a,8,9,10 -hexahydro-4H-py rimidinyl[1,6-a]py rrolo[1,2-c]pyrimi dine-4-one | 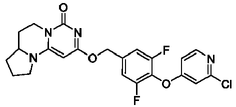 | B | 461 | CDCl₃: δ 8.26 (d, J = 5.8 Hz, 1 H), 7.11 (d, J = 8.0 Hz, 2 H), 6.90 - 6.76 (m, 2 H), 5.40 (d, J = 4.1 Hz, 2 H), 4.99 (s, 1 H), 4.62 (br. s., 1 H), 3.61 - 3.48 (m, 1 H), 3.46 - 3.24 (m, 3 H), 2.38 (br. s., 1 H), 2.31 - 2.12 (m, 2 H), 2.10 - 1.96 (m, 1 H), 1.62 - 1.45 (m, 2 H) |
| 31 | 2-((3,5-difluoro-4 -((2-(trifluoromet hoxy)pyrimidin-5-yl)oxy)benzyl)oxy )-6,7,7a,8,9,10-he xahydro-4H-pyri midinyl[1,6-a]pyr rolo[1,2-c]pyrimi dine-4-one | 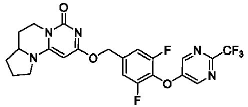 | B | 496 | CDCl₃: δ 8.55 (s, 2 H), 7.15 (d, J = 8.5 Hz, 2 H), 5.41 (d, J = 4.9 Hz, 2 H), 4.99 (s, 1 H), 4.65 (dd, J = 2.3, 14.2 Hz, 1 H), 3.55 (dd, J = 3.7, 4.3 Hz, 1 H), 3.45 - 3.26 (m, 3 H), 2.40 (dd, J = 1.1, 13.5 Hz, 1 H), 2.33 - 2.14 (m, 2 H), 2.10 - 1.96 (m, 1 H), 1.62 - 1.48 (m, 2 H) |
| 32 | 5-((((4-one-6,7,7a ,8,9,10-hexahydro -4H-pyrimido[1,6 -a]pyrrolo[1,2-c]p yrimidin-2-yl)oxy )methyl)-2-(3-(trif luoromethyl)phen oxy)benzonitrile | 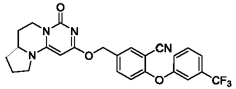 | B | 483 | CDCl₃: δ 7.72 (d, J = 1.9 Hz, 1 H), 7.60 - 7.41 (m, 3 H), 7.31 (s, 1 H), 7.29 - 7.20 (m, 1 H), 6.92 - 6.83 (m, 1 H), 5.44 - 5.31 (m, 2 H), 4.95 (s, 1 H), 4.67 - 4.57 (m, 1 H), 3.52 (tt, J = 4.3, 10.8 Hz, 1 H), 3.43 - 3.22 (m, 3 H), 2.43 - 2.34 (m, 1 H), 2.30 - 2.12 (m, 2 H), 2.09 - 1.92 (m, 1 H), 1.59 - 1.44 (m, 2 H) |
| 33 | 2-((4-(4-chloro-3-(trifluoromethyl)p henoxy)-3,5-diflu orobenzyl)oxy)-6, 7,7a,8,9,10-hexah ydro-4H-pyrimidi nyl[1,6-a]pyrrolo[ 1,2-c]pyrimidine-4-one | 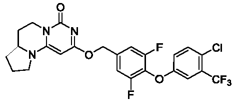 | B | 528 | CDCl₃: δ 7.40 (d, J = 8.8 Hz, 1 H), 7.30 (d, J = 2.7 Hz, 1 H), 7.12 - 7.05 (m, 2 H), 7.00 (dd, J = 3.0, 8.8 Hz, 1 H), 5.44 - 5.34 (m, 2 H), 4.98 (s, 1 H), 4.69 - 4.60 (m, 1 H), 3.59 - 3.48 (m, 1 H), 3.45 - 3.25 (m, 3 H), 2.45 - 2.35 (m, 1 H), 2.32 - 2.15 (m, 2 H), 2.09 - 1.94 (m, 1 H), 1.60 - 1.47 (m, 2 H) |
| 34 | 2-((3-fluoro-4-((2-(trifluoromethyl)p yrid-4-yl)oxy)ben zyl)oxy)-7a-meth yl-6,7,7a,8,9,10-h exahydro-4H-pyri midinyl[1,6-a]pyr rolo[1,2-c]pyrimi dine-4-one | 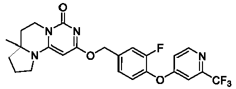 | B | 491 | CDCl₃: δ 8.64 - 8.50 (m, 1 H), 7.33 (d, J = 10.7 Hz, 1 H), 7.27 - 7.12 (m, 3 H), 7.01 - 6.91 (m, 1 H), 5.48 - 5.37 (m, 2 H), 5.02 - 4.92 (m, 1 H), 4.56 - 4.42 (m, 1 H), 3.63 - 3.52 (m, 1 H), 3.50 - 3.41 (m, 1 H), 3.37 - 3.28 (m, 1 H), 2.26 - 2.04 (m, 4 H), 1.72 - 1.61 (m, 2 H), 1.22 (s, 3 H) |
| 35 | 2-((3,5-fluoro-4-(( 2-methylpyrid-4-y l)oxy)benzyl)oxy) -7a-methyl-6,7,7a, 8,9,10-hexahydro-4H-pyrimidinyl[1, 6-a]pyrrolo[1,2-c] pyrimidine-4-one | 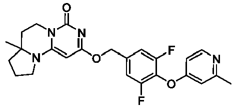 | B | 455 | CDCl₃: δ 8.36 (d, J = 5.5 Hz, 1 H), 7.12 - 7.07 (m, 2 H), 6.71 - 6.66 (m, 2 H), 5.40 (s, 2 H), 4.97 (s, 1 H), 4.52 - 4.44 (m, 1 H), 3.63 - 3.52 (m, 1 H), 3.50 - 3.43 (m, 1 H), 3.34 (dt, J = 8.4, 10.1 Hz, 1 H), 2.52 (s, 3 H), 2.28 - 2.19 (m, 2 H), 2.17 - 2.03 (m, 2 H), 1.73 - 1.61 (m, 2 H), 1.23 (s, 3 H) |
| 36 | 2-(((4-((2-chlorop yridyl-4-yl)oxy)-3 ,5-difluorobenzen e)oxy)-7a-methyl-6,7,7a,8,9,10-hexa hydro-4H-pyrimid inyl[1,6-a]pyrrolo [1,2-c]pyrimidine-4-one | 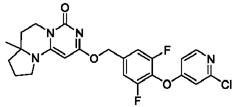 | B | 475 | CDCl₃: δ 8.27 (d, J = 5.8 Hz, 1 H), 7.14 - 7.09 (m, 2 H), 6.86 - 6.81 (m, 2 H), 5.40 (s, 2 H), 4.97 (s, 1 H), 4.52 - 4.44 (m, 1 H), 3.57 (dt, J = 4.4, 14.2 Hz, 1 H), 3.49 - 3.43 (m, 1 H), 3.38 - 3.29 (m, 1 H), 2.28 - 2.19 (m, 2 H), 2.16 - 2.04 (m, 2 H), 1.72 - 1.62 (m, 2 H), 1.22 (s, 3 H) |
| 37 | 2-fluoro-5-(((((7a-methyl-4-one-6,7, 7a,8,9,10-hexahyd ro-4H-pyrimido[1 ,6-a]pyrrolo[1,2-c ]pyrimidin-2-yl)o xy)methyl)benzon itrile | 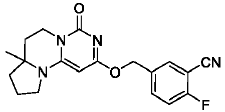 | A | 355 | CDCl₃: δ 7.71 - 7.61 (m, 2 H), 7.19 (t, J = 8.7 Hz, 1 H), 5.40 (d, J = 0.8 Hz, 2 H), 4.94 (s, 1 H), 4.53 - 4.44 (m, 1 H), 3.62 - 3.52 (m, 1 H), 3.46 (d, J = 1.9 Hz, 1 H), 3.38 - 3.29 (m, 1 H), 2.28 - 2.20 (m, 2 H), 2.18 - 2.04 (m, 2 H), 1.72 - 1.64 (m, 2 H), 1.23 (s, 3 H) |
| 38 | 2-((3,5-difluoro-4 -((2-(trifluoromet hyl)pyrid-4-yl)ox y)benzyl)oxy)-7a-ethyl-6,7,7a,8,9,1 0-hexahydro-4H-p yrimido[1,6-a]pyr rolo[1,2-c]pyrimi dine-4-one | 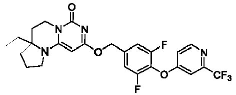 | B | 523 | CDCl₃: δ 8.60 (d, J = 5.8 Hz, 1 H), 7.27 (s, 1 H), 7.17 - 7.11 (m, 2 H), 6.99 (dd, J = 2.5, 5.8 Hz, 1 H), 5.42 (s, 1 H), 4.99 (s, 1 H), 4.42 - 4.35 (m, 1 H), 3.55 - 3.43 (m, 2 H), 3.40 - 3.31 (m, 1 H), 2.47 - 2.39 (m, 1 H), 2.28 - 2.09 (m, 3 H), 1.59 - 1.44 (m, 4 H), 0.92 - 0.87 (m, 3 H) |
| 39 | 2-((4-(4-chloro-3-(trifluoromethyl)p henoxy)-3,5-diflu orobenzyl)oxy)-7a -ethyl-6,7,7a,8,9,1 0-hexahydro-4H-p yrimidinyl[1,6-a]p yrrolo[1,2-c]pyri midine-4-one | 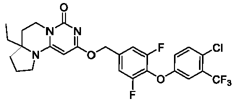 | B | 556 | CDCl₃: δ 7.41 (d, J = 8.8 Hz, 1 H), 7.31 (d, J = 3.0 Hz, 1 H), 7.13 - 7.07 (m, 2 H), 7.01 (dd, J = 3.0, 8.8 Hz, 1 H), 5.40 (s, 2 H), 4.98 (s, 1 H), 4.43 - 4.34 (m, 1 H), 3.54 - 3.42 (m, 2 H), 3.40 - 3.31 (m, 1 H), 2.47 - 2.39 (m, 1 H), 2.28 - 2.09 (m, 3 H), 1.58 - 1.45 (m, 4 H), 0.90 (t, J = 7.6 Hz, 3 H) |
| 40 | 5-(((((7a-ethyl-4-o ne-6,7,7a,8,9,10-h exahydro-4H-pyri midinyl[1,6-a]pyr rolo[1,2-c]pyrimi din-2-yl)oxy)meth yl)-2-(3-(trifluoro methyl)phenoxy)b enzonitrile | 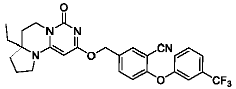 | B | 511 | CDCl₃: δ 7.75 (d, J = 1.9 Hz, 1 H), 7.57 (dd, J = 2.2, 8.8 Hz, 1 H), 7.54 - 7.46 (m, 2 H), 7.33 (s, 1 H), 7.26 (d, J = 0.5 Hz, 1 H), 6.89 (d, J = 8.5 Hz, 1 H), 5.40 (d, J = 2.7 Hz, 2 H), 4.96 (s, 1 H), 4.42 - 4.34 (m, 1 H), 3.53-3.43 (m, 2 H), 3.38-3.31 (m, 1 H), 2.47 - 2.39 (m, 1 H), 2.28 - 2.20 (m, 1 H), 2.18 - 2.09 (m, 2 H), 1.56 - 1.47 (m, 4 H), 0.92 - 0.88 (m, 3 H) |
| 41 | 2-(4-(4-chloro-3-( trifluoromethyl)ph enoxy)phenylethoxy)-7a-ethyl-6,7,7 a,8,9,10-hexahydr o-4H-pyrimidine[ 1,6-a]pyrrole[1,2-c]pyrimidine-4-on e | 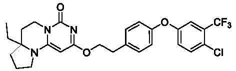 | B | 534 | CDCl₃: δ 7.57 - 7.51 (m, 1 H), 7.46 - 7.39 (m, 1 H), 7.38 - 7.34 (m, 1 H), 7.33- 7.29 (m, 2 H), 7.16 - 7.11 (m, 1 H), 6.96 - 6.93 (m, 1 H), 5.41 - 5.32 (m, 2 H), 4.88 - 4.86 (m, 1 H), 4.57 - 4.54 (m, 1 H), 3.51 - 3.39 (m, 1 H), 3.38 - 3.26 (m, 1 H), 3.05 - 2.99 (m, 1 H), 2.48 - 2.33 (m, 1 H), 2.28 - 2.13 (m, 3 H), 1.52 - 1.49 (m, 4 H), 0.90 (br. s., 3 H) |
| 42 | 2-((3-fluoro-4-((2-(trifluoromethyl)p yrid-4-yl)oxy)ben zyl)oxy)-7,7a,8,9, 10,11-hexahydro-4H,6H-pyridine[1, 2-c]pyrimidine[1, 6-a]pyrimidine-4-one | 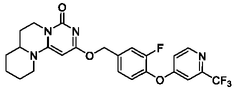 | B | 491 | CDCl₃: δ 8.57 (d, J = 5.5 Hz, 1 H), 7.34 (dd, J = 1.8, 10.9 Hz, 1 H), 7.27 - 7.25 (m, 1 H), 7.23 (d, J = 2.2 Hz, 1 H), 7.21 - 7.16 (m, 1 H), 6.96 (dd, J = 2.3, 5.6 Hz, 1 H), 5.42 (s, 2 H), 5.23 (s, 1 H), 4.10 (d, J = 3.3 Hz, 1 H), 3.95 - 3.86 (m, 1 H), 3.85 - 3.78 (m, 1 H), 3.26 (dd, J = 2.6, 6.5 Hz, 1 H), 2.99 (d, J = 0.5 Hz, 1 H), 2.22 - 2.18 (m, 1 H), 1.95 - 1.91 (m, 1 H), 1.81 - 1.76 (m, 3 H), 1.62 - 1.54 (m, 3 H) |
| 43 | 2-((4-(4-chloro-3-(trifluoromethyl)p henoxy)-3,5-diflu orobenzyl)oxy)-7, 7a,8,9,10,11-hexa hydro-4H,6H-pyri dyl[1,2-c]pyrimid o[1,6-a]pyrimidin e-4-one | 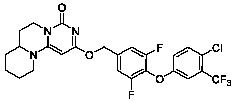 | B | 542 | CDCl₃: δ 7.42 (d, J = 9.1 Hz, 1 H), 7.31 (d, J = 3.0 Hz, 1 H), 7.12 - 7.07 (m, 2 H), 7.01 (dd, J = 3.0, 9.1 Hz, 1 H), 5.41 - 5.37 (m, 2 H), 5.25 - 5.22 (m, 1 H), 4.16 - 4.06 (m, 1 H), 3.95 - 3.85 (m, 1 H), 3.83 - 3.75 (m, 1 H), 3.34 - 3.24 (m, 1 H), 3.05 - 2.94 (m, 1 H), 2.27 - 2.15 (m, 1 H), 2.00 - 1.90 (m, 1 H), 1.85 - 1.75 (m, 3 H), 1.62 - 1.54 (m, 3 H) |
| 44 | 5-((((4-one-7,7a,8 ,9,10,11-hexahydr o-4H,6H-pyridine [1,2-c]pyrimido[1, 6-a]pyrimidin-2-y l)oxy)methyl)-2-( 3-(trifluoromethyl )phenoxy)benzoni trile | 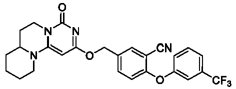 | B | 497 | CDCl₃: δ 7.77 - 7.73 (m, 1 H), 7.59 - 7.46 (m, 3 H), 7.33 (s, 1 H), 7.27 - 7.23 (m, 1 H), 6.89 (d, J = 8.5 Hz, 1 H), 5.43 - 5.34 (m, 2 H), 5.21 (s, 1 H), 4.15 - 4.06 (m, 1 H), 3.94 - 3.84 (m, 1 H), 3.82 - 3.74 (m, 1 H), 3.34 - 3.23 (m, 1 H), 3.04 - 2.93 (m, 1 H), 2.26 - 2.14 (m, 1 H), 1.97 - 1.89 (m, 1 H), 1.83 - 1.76 (m, 2 H), 1.73 - 1.62 (m, 2 H), 1.60 - 1.50 (m, 2 H) |
| 45 | 2-((3,5-difluoro-4 -((2-(trifluoromet hyl)pyrimidin-5-y l)oxy)benzyl)oxy) -7,7a,8,9,10,11-he xahydro-4H,6H-p yridino[1,2-c]pyri mido[1,6-a]pyrimidine-4-one | 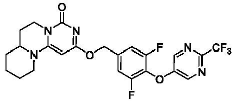 | B | 510 | CDCl₃: δ 8.59 - 8.51 (s, 2 H), 7.20 - 7.10 (m, 2 H), 5.44 - 5.37 (m, 2 H), 5.25 - 5.21 (m, 1 H), 4.17 - 4.04 (m, 1 H), 3.95 - 3.85 (m, 1 H), 3.84 - 3.73 (m, 1 H), 3.35 - 3.23 (m, 1 H), 3.05 - 2.93 (m, 1 H), 2.27- 2.13 (m, 1 H), 1.99 - 1.88 (m, 1 H), 1.84 - 1.76 (m, 3 H), 1.69 - 1.58 (m, 3 H) |
| 46 | 2-(4-(4-chloro-3-( trifluoromethyl)ph enoxy)phenyletho xy)-7,7a,8,9,10,11 -hexahydro-4H,6 H-pyridyl[1,2-c]p yrimidinyl[1,6 -a]p yrimidine-4-one | 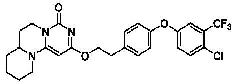 | B | 520 | CDCl₃: δ 7.51 - 7.38 (m, 2 H), 7.36 - 7.29 (m, 2 H), 7.12 - 7.04 (m, 1 H), 7.03 - 6.84 (m, 2 H), 5.28 - 5.01 (m, 1 H), 4.72 - 4.43 (m, 2 H), 4.17 - 4.02 (m, 1 H), 3.94 - 3.69 (m, 2 H), 3.33 - 3.21 (m, 1 H), 3.13 - 2.89 (m, 3 H), 2.37 - 1.84 (m, 8 H) |
| 47 | (S)-2-((3-fluoro-4 -((2-(trifluoromet hyl)pyrid-4-yl)ox y)benzyl)oxy) -7,7 a,8,9,10,11-hexah ydro-4H,6H-pyrid ino[1,2-c]pyrimidi ne[1,6-a]pyrimidi ne-4-one | 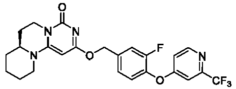 | A | 491 | CDCl₃: δ 8.58 (d, J = 5.9 Hz, 1 H), 7.34 (d, J = 10.8 Hz, 1 H), 7.30 - 7.27 (m, 1 H), 7.24 (s, 1 H), 7.22 - 7.15 (m, 1 H), 6.96 (d, J = 4.9 Hz, 1 H), 5.43 (s, 2 H), 5.23 (s, 1 H), 4.16 - 4.07 (m, 1 H), 3.95 - 3.86 (m, 1 H), 3.79 (d, J = 12.7 Hz, 1 H), 3.28 (d, J = 4.4 Hz, 1 H), 2.99 (t, J = 11.2 Hz, 1 H), 2.21 (d, J = 3.9 Hz, 1 H), 1.93 (br. s., 1 H), 1.84 - 1.78 (m, 3 H), 1.59 (d, J = 10.3 Hz, 3H) |
| 48 | (S)-2-(((3-fluoro-4-((2-(trifluorome thyl)pyrid-4-yl)ox y)benzyl)oxy)-1-methoxy-7,7a,8,9, 10,11-hexahydro-4H,6H-pyridyl[1, 2-c]pyrimidinyl[1, 6-a]pyrimidine-4-one | 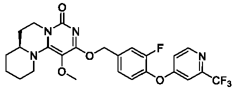 | A | 521 | CDCl₃: δ 8.58 (d, J = 5.9 Hz, 1 H), 7.37 (d, J = 10.8 Hz, 1 H), 7.30 (d, J = 8.3 Hz, 1 H), 7.25 - 7.16 (m, 2 H), 6.96 (d, J = 3.4 Hz, 1 H), 5.48 (s, 2 H), 4.60 (d, J = 12.7 Hz, 1 H), 4.44 - 4.30 (m, 1 H), 3.77 - 3.54 (m, 4 H), 3.26 (d, J = 5.9 Hz, 1 H), 3.09 - 2.93 (m, 1 H), 2.23 - 2.12 (m, 1 H), 1.95 (d, J = 11.7 Hz, 1 H), 1.83 - 1.61 (m, 5 H), 1.39 (d, J = 12.7 Hz, 1 H) |
| 49 | 2-((3-fluoro-4-((2-(trifluoromethyl)p yrid-4-yl)oxy)ben zyl)oxy)-7a-meth yl-7,7a,8,9,10,11-hexahydro-4H,6H -pyridino[1,2-c]py rimidine[1,6-a]pyr imidine-4-one | 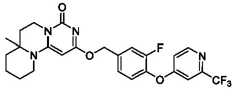 | A | 505 | CDCl₃: δ 8.56 (d, J = 5.9 Hz, 1 H), 7.32 (d, J = 11.2 Hz, 1 H), 7.28 - 7.21 (m, 2 H), 7.20 - 7.13 (m, 1 H), 6.95 (d, J = 3.4 Hz, 1 H), 5.41 (s, 2 H), 5.21 (s, 1 H), 4.24 (td, J = 4.6, 13.8 Hz, 1 H), 3.88 - 3.69 (m, 1 H), 3.54 (d, J = 11.2 Hz, 1 H), 3.19 - 2.95 (m, 1 H), 2.06 - 1.95 (m, 1 H), 1.94 - 1.78 (m, 3 H), 1.73 (d, J = 2.9 Hz, 2 H), 1.62 - 1.46 (m, 2 H), 1.31 (s, 3 H) |
| 50 | 2-((3,5-difluoro-4 -((2-methylpyrid-4-yl)oxy)benzyl)o xy)-7a-methyl-7,7 a,8,9,10,11-hexah ydro-4H,6H-pyridino[1,2-c]pyrimidi ne[1,6-a]pyrimidi ne-4-one | 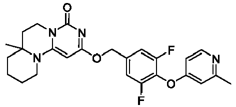 | A | 469 | CDCl₃: δ 8.35 (d, J = 5.4 Hz, 1 H), 7.08 (d, J = 8.3 Hz, 2 H), 6.74 - 6.58 (m, 2 H), 5.46 - 5.28 (m, 2 H), 5.21 (s, 1 H), 4.23 (td, J = 4.5, 14.1 Hz, 1 H), 3.84 -3.66 (m, 1 H), 3.54 (d, J = 11.7 Hz, 1 H), 3.27 - 2.98 (m, 1 H), 2.50 (s, 3 H), 2.06 - 1.95 (m, 1 H), 1.93 - 1.78 (m, 2 H), 1.77 - 1.63 (m, 3 H), 1.60 - 1.45 (m, 2 H), 1.30 (s, 3 H) |
| 51 | 2-((3,5-difluoro-4 -((2-(trifluoromet hyl)pyrid-4-yl)ox y)benzyl)oxy)-6,7 ,7a,8,10,11-hexah ydro-4H-pyrimidi nyl[6',1':2,3]pyri midine[6,1-c][1,4] oxazin-4-one | 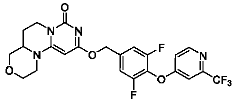 | B | 511 | CDCl₃: δ 8.56 (d, J = 5.5 Hz, 1 H), 7.22 (d, J = 2.2 Hz, 1 H), 7.10 (s, 1 H), 7.08 (s, 1 H), 6.95 (dd, J = 2.2, 5.5 Hz, 1 H), 5.42 - 5.33 (m, 2 H), 5.19 (s, 1 H), 4.28 (td, J = 4.8, 13.7 Hz, 1 H), 3.99 (dd, J = 3.2, 11.7 Hz, 1 H), 3.89 (dd, J = 3.0, 11.3 Hz, 1 H), 3.69 (ddd, J = 4.1, 10.2, 13.9 Hz, 1 H), 3.59 (dt, J = 2.6, 11.9 Hz, 1 H), 3.53 - 3.40 (m, 2 H), 3.28 - 3.18 (m, 2 H), 2.11 (qd, J = 4.8, 14.1 Hz, 1 H), 1.74 - 1.61 (m, 1 H) |
| 52 | 2-((4-(4-chloro-3-(trifluoromethyl)p henoxy)-3,5-diflu orobenzyl)oxy)-6, 7,7a,8,10,11-hexa hydro-4H-pyrimid inyl[6',1':2,3]pyri mido[6,1-c][1,4]o xazin-4-one | 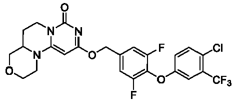 | B | 544 | CDCl₃: δ 7.39 (d, J = 8.8 Hz, 1 H), 7.27 (d, J = 2.7 Hz, 1 H), 7.06 (d, J = 8.2 Hz, 2 H), 6.98 (dd, J = 2.5, 8.8 Hz, 1 H), 5.48 - 5.34 (m, 2 H), 5.19 (s, 1 H), 4.29 (td, J = 4.8, 13.7 Hz, 1 H), 3.99 (dd, J = 3.0, 11.5 Hz, 1 H), 3.90 (dd, J = 2.9, 11.4 Hz, 1 H), 3.70 (ddd, J = 3.8, 10.3, 13.9 Hz, 1 H), 3.60 (dt, J = 2.5, 11.8 Hz, 1 H), 3.53 - 3.42 (m, 2 H), 3.31 - 3.20 (m, 2 H), 2.12 (dt, J = 4.8, 9.3 Hz, 1 H), 1.74 - 1.60 (m, 1 H) |
| 53 | 5-((((4-one-6,7,7a ,8,10,11-hexahydr o-4H-pyrimido[6', 1':2,3]pyrimido[6, 1-c][1,4]oxazin-2-yl)oxy)methyl)-2-(3-(trifluoromethy l)phenoxy)benzon itrile | 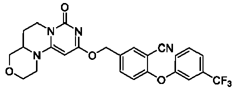 | B | 499 | CDCl₃: δ 7.71 (s, 1 H), 7.57 - 7.47 (m, 2 H), 7.47 - 7.42 (m, 1 H), 7.30 (s, 1 H), 7.23 (d, J = 8.0 Hz, 1 H), 6.86 (d, J = 8.8 Hz, 1 H), 5.41 - 5.31 (m, 2 H), 5.17 (s, 1 H), 4.28 (td, J = 4.7, 13.6 Hz, 1 H), 4.02 - 3.95 (m, 1 H), 3.89 (dd, J = 2.6, 11.4 Hz, 1 H), 3.74 - 3.64 (m, 1 H), 3.63 - 3.55 (m, 1 H), 3.53 - 3.40 (m, 2 H), 3.29 - 3.17 (m, 2 H), 2.11 (qd, J = 4.7, 14.0 Hz, 1 H), 1.74 - 1.61 (m, 1 H) |
| 54 | 2-((3,5-difluoro-4 -((2-(trifluoromet hoxy)pyrid-4-yl)o xy)benzyl)oxy)-6, 7,7a,8,10,11-hexa hydro-4H-pyrimid inyl[6',1':2,3]pyri midine[6,1-c][1,4] oxazin-4-one | 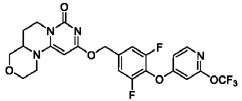 | B | 527 | CDCl₃: δ 8.17 (d, J = 5.8 Hz, 1 H), 7.08 (d, J = 8.2 Hz, 2 H), 6.84 - 6.76 (m, 1 H), 6.46 (br. s., 1 H), 5.47 - 5.33 (m, 2 H), 5.19 (s, 1 H), 4.37 - 4.24 (m, 1 H), 3.99 (d, J = 11.5 Hz, 1 H), 3.94 - 3.86 (m, 1 H), 3.75 - 3.55 (m, 2 H), 3.54 - 3.41 (m, 2 H), 3.32 -3.17 (m, 2 H), 2.11 (td, J = 4.8, 9.1 Hz, 1 H), 1.77 - 1.62 (m, 1 H) |
| 55 | 2-(((3,5-difluoro-4-((2-(trifluorome thyl)pyrimidin-5-yl)yl)benzyl)oxy)-6,7,7a,8,10,11-hex ahydro-4H-pyrimi dinyl[6',1':2,3]pyr imidine[6,1-c][1,4 ]oxazin-4-one | 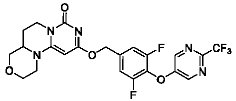 | B | 512 | CDCl₃: δ 8.53 (s, 2 H), 7.12 (d, J = 8.5 Hz, 2 H), 5.40 - 5.37 (m, 2 H), 5.19 (s, 1 H), 4.33 - 4.26 (m, 1 H), 4.00 (dd, J = 2.9, 11.7 Hz, 1 H), 3.90 (dd, J = 2.9, 11.4 Hz, 1 H), 3.74 - 3.66 (m, 1 H), 3.60 (dt, J = 2.3, 11.9 Hz, 1 H), 3.53 - 3.43 (m, 2 H), 3.29 - 3.22 (m, 2 H), 2.12 (td, J = 4.7, 9.2 Hz, 1 H), 1.71 - 1.65 (m, 1 H) |
| 56 | 2-(4-(4-chloro-3-( trifluoromethyl)ph enoxy)phenyletho xy)-6,7,7a,8,10,11 -hexahydro-4H-py rimidinyl[6',1':2,3 ]pyrimidinyl[6,1-c ][1,4]oxazin-4-on e | 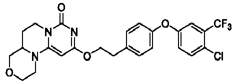 | B | 522 | CDCl₃: δ 7.40 (d, J = 8.8 Hz, 1 H), 7.28 (d, J = 2.7 Hz, 1 H), 7.23 (s, 2 H), 7.03 (dd, J = 2.7, 8.8 Hz, 1 H), 6.93 (d, J = 8.5 Hz, 2 H), 5.07 (s, 1 H), 4.53 (t, J = 6.9 Hz, 2 H), 4.25 (td, J = 4.8, 13.7 Hz, 1 H), 3.97 (dd, J = 3.3, 11.5 Hz, 1 H), 3.88 (dd, J = 3.0, 11.3 Hz, 1 H), 3.70 (ddd, J = 4.1, 10.2, 13.9 Hz, 1 H), 3.58 (dt, J = 2.7, 12.0 Hz, 1 H), 3.49 - 3.36 (m, 2 H), 3.28 - 3.13 (m, 2 H), 2.99 (t, J = 6.9 Hz, 2 H), 2.14 - 2.03 (m, 1 H), 1.67 - 1.55 (m, 1 H) |
| 57 | (7aS,10S)-1-fluor o-2-((3-fluoro-4-(( 2-(trifluoromethyl )pyrid-4-yl)oxy)b enzyl)oxy)-6,7,10, 11-tetrahydro-4H, 8H-7a,10-methylp yrimidinyl[6',1':2, 3]pyrimidinyl[6,1 -c][1,4]oxazin-4-o ne | 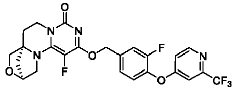 | A | 523 | CDCl₃: δ 8.57 (d, J = 5.9 Hz, 1 H), 7.36 (d, J = 10.8 Hz, 1 H), 7.30 (d, J = 8.8 Hz, 1 H), 7.23 (d, J = 1.5 Hz, 1 H), 7.21 - 7.15 (m, 1 H), 6.98 - 6.91 (m, 1 H), 5.47 (s, 2 H), 4.74 (s, 1 H), 4.64 (d, J = 14.2 Hz, 1 H), 4.00 (d, J = 7.3 Hz, 1 H), 3.91 (d, J = 5.9 Hz, 2 H), 3.77 (d, J = 7.3 Hz, 1 H), 3.39 (d, J = 3.4 Hz, 1 H), 2.46 - 2.37 (m, 1 H), 2.33 (dd, J = 4.9, 12.7 Hz, 1 H), 2.25 - 2.18 (m, 1 H), 1.81 (s, 1 H) |
| 58 | (7aS,10S)-1-chlor o-2-((3-fluoro-4-(( 2-(trifluoromethyl )pyrid-4-yl)oxy)b enzyl)oxy)-6,7,10, 11-tetrahydro-4H, 8H-7a,10-methylp yrimidinyl[6',1':2, 3]pyrimidinyl[6,1 -c][1,4]oxazin-4-o ne | 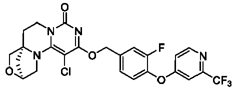 | A | 539 | CDCl₃: δ 8.58 (d, J = 5.4 Hz, 1 H), 7.37 (d, J = 11.2 Hz, 1 H), 7.30 (d, J = 8.3 Hz, 1 H), 7.24 (d, J = 2.0 Hz, 1 H), 7.23 - 7.16 (m, 1 H), 6.98 - 6.93 (m, 1 H), 5.48 (s, 2 H), 4.72 (s, 1 H), 4.28 - 4.19 (m, 2 H), 4.06 (d, J = 10.8 Hz, 1 H), 3.99 - 3.90 (m, 2 H), 3.81 (d, J = 7.8 Hz, 1 H), 2.35 (t, J = 5.9 Hz, 2 H), 2.10 (d, J = 10.3 Hz, 1 H), 1.90 (d, J = 9.8 Hz, 1 H) |
| 59 | (7aS,10S)-2-((3-fl uoro-4-((2-(trifluo romethyl)pyrid-4-yl)oxy)benzyl)oxy )-1-methyl-6,7,10, 11-tetrahydro-4H, 8H-7a,10-methylp yrimidinyl[6',1':2, 3]pyrimidinyl[6,1 -c][1,4]oxazin-4-o ne | 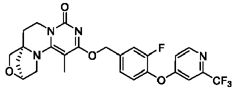 | A | 519 | CDCl₃: δ 8.57 (d, J = 5.4 Hz, 1 H), 7.32 (d, J = 11.2 Hz, 1 H), 7.30 - 7.22 (m, 2 H), 7.22 - 7.14 (m, 1 H), 6.96 (d, J = 3.4 Hz, 1 H), 5.44 (br. s., 2 H), 4.70 (br. s., 1 H), 4.42 (d, J = 13.2 Hz, 1 H), 3.97 (d, J = 9.8 Hz, 1 H), 3.91 - 3.75 (m, 3 H), 3.54 (d, J = 9.3 Hz, 1 H), 2.47 - 2.18 (m, 2 H), 2.05 (s, 3 H), 2.01 - 1.88 (m, 2 H) |
| 60 | (7aS,10S)-1-ethyl -2-((3-fluoro-4-((2 -(trifluoromethyl) pyrid-4-yl)oxy)be nzyl)oxy)-6,7,10,1 1-tetrahydro-4H,8 H-7a,10-methylpy rimidinyl[6',1':2,3 ]pyrimidinyl[6,1-c ][1,4]oxazin-4-on e | 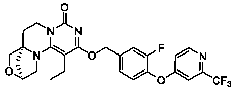 | A | 533 | CDCl₃: δ 8.56 (d, J = 5.4 Hz, 1 H), 7.31 (d, J = 10.8 Hz, 1 H), 7.28 - 7.21 (m, 2 H), 7.21 - 7.14 (m, 1 H), 6.95 (d, J = 5.9 Hz, 1 H), 5.44 (s, 2 H), 4.73 (s, 1 H), 4.41 (td, J = 5.6, 13.8 Hz, 1 H), 3.95 - 3.76 (m, 4 H), 3.58 (d, J = 9.8 Hz, 1 H), 2.52 (d, J = 7.3 Hz, 2 H), 2.40 - 2.30 (m, 1 H), 2.22 (ddd, J = 3.9, 9.5, 13.9 Hz, 1 H), 2.04 - 1.86 (m, 3 H), 1.09 (t, J = 7.3 Hz, 2 H) |
| 61 | (7aS,10S)-2-((4-(( 2-chloropyrid-4-yl )oxy)-3,5-difluoro benzyl)oxy)-1-flu oro-6,7,10,11-tetr ahydro-4H,8H-7a, 10-methylaminop yrimidine[6',1':2,3 ]pyrimidine[6,1-c] [1,4]oxazin-4-one | 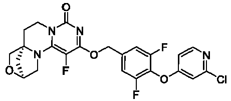 | A | 507 | CDCl₃: δ 8.27 (d, J = 5.4 Hz, 1 H), 7.15 (d, J = 8.3 Hz, 2 H), 6.88 - 6.81 (m, 2 H), 5.46 (s, 2 H), 4.75 (s, 1 H), 4.65 (d, J = 14.2 Hz, 1 H), 4.01 (d, J = 7.3 Hz, 1 H), 3.92 (d, J = 5.9 Hz, 2 H), 3.78 (d, J = 7.8 Hz, 1 H), 3.39 (br. s., 1 H), 2.47 - 2.39 (m, 1 H), 2.34 (dd, J = 3.9, 12.7 Hz, 1 H), 2.23 (d, J = 8.3 Hz, 1 H), 1.81 (d, J = 10.3 Hz, 1 H) |
| 62 | (7aS,10S)-2-((3-fl uoro-4-((2-(trifluo romethyl)pyrid-4-yl)oxy)benzyl)oxy )-1-methoxy-6, 7,1 0,11-tetrahydro-4 H,8H-7a,10-meth ylpyrimidinyl[6',1' :2,3]pyrimidinyl[6 ,1-c][1,4]oxazin-4 -one | 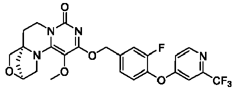 | A | 535 | CDCl₃: δ 8.57 (d, J = 5.9 Hz, 1 H), 7.36 (d, J = 10.8 Hz, 1 H), 7.32 - 7.27 (m, 1 H), 7.24 - 7.15 (m, 2 H), 6.97 - 6.93 (m, 1 H), 5.48 (s, 2 H), 4.71 (br. s., 1 H), 4.53 (d, J = 14.2 Hz, 1 H), 4.00 - 3.89 (m, 3 H), 3.77 (d, J = 7.3 Hz, 1 H), 3.64 (s, 3 H), 3.50 (br. s., 1 H), 2.41 - 2.24 (m, 2 H), 2.15 (d, J = 9.8 Hz, 1 H), 1.81 (s, 1 H) |
| 63 | (7aS,10S)-2-(((3,5 -difluoro-4-((2-(tri fluoromethoxy)py rid-4-yl)oxy)benz yl)oxy)-1-methox y-6,7,10,11-tetrah ydro-4H,8H-7a,10 -methanopyrimido [6',1':2,3]pyrimid o[6,1-c][1,4]oxazi n-4-one | 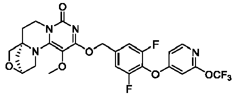 | A | 569 | CDCl₃: δ 8.21 (d, J = 5.8 Hz, 1 H), 7.17 - 7.13 (m, 2 H), 6.82 (dd, J = 2.2, 5.8 Hz, 1 H), 6.50 (d, J = 2.2 Hz, 1 H), 5.52 - 5.40 (m, 2 H), 4.73 (d, J = 1.4 Hz, 1 H), 4.55 (td, J = 4.4, 14.3 Hz, 1 H), 4.03 - 3.89 (m, 3 H), 3.77 (d, J = 7.4 Hz, 1 H), 3.69 - 3.62 (m, 3 H), 3.50 (ddd, J = 4.3, 11.7, 14.2 Hz, 1 H), 2.31(d, J = 4.7 Hz, 2 H), 2.19-2.14 (m, 1 H), 1.81 (d, J = 10.2 Hz, 1 H) |
| 64 | 2-((3-fluoro-4-((2-(trifluoromethyl)p yrid-4-yl)oxy)ben zyl)oxy)-4-oxo-6, 7,7a,8,9,10-hexah ydro-4H-pyrimidi nyl[1,6-a]pyrrolo[ 1,2-c]pyrimidine-1-nitrile | 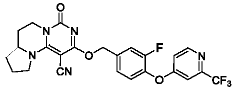 | A | 502 | CDCl₃: δ 8.58 (d, J = 5.8 Hz, 1 H), 7.41 - 7.29 (m, 2 H), 7.25 (d, J = 2.5 Hz, 1 H), 7.23 - 7.15 (m, 1 H), 6.95 (dd, J = 2.3, 5.6 Hz, 1 H), 5.49 (s, 2 H), 4.70 - 4.50 (m, 1 H), 4.31 (ddd, J = 1.9, 9.1, 11.0 Hz, 1 H), 4.05 (dt, J = 7.6, 10.4 Hz, 1 H), 3.74 - 3.57 (m, 1 H), 3.53 - 3.25 (m, 1 H), 2.52 - 2.38 (m, 1 H), 2.35 - 2.12 (m, 2 H), 2.11 - 1.89 (m, 1 H), 1.66 - 1.55 (m, 2 H) |

### Biological testing and data

The compounds of the present invention are Lp-PLA₂ inhibitors, which can be used for treating and preventing Lp-PLA₂-mediated diseases. The biological activity of the compounds of the present invention can be determined using any suitable test for determining the activity of the compounds as the Lp-PLA₂ inhibitors, as well as tissue and in vivo models.

Biological activity data of each compound is reported as the mean value of at least one experiment or multiple experiments. It should be understood that the data described in the present invention may vary reasonably depending on the specific conditions and methods used by the person conducting the experiments.
Determination of lipoprotein-related phospholipase A2 (Lp-PLA₂) in human plasma.

Human plasma determination utilizes a thioester analogue of phosphatidylcholine (PAF), where hydrolysis leads to the formation of phospholipids containing free thiol groups. The amount of the thiol groups is continuously determined by reacting with CPM (7-diethylamino-3-(4'-maleimidophenyl)-4-methylcoumarin), which is maleimide with increased fluorescence after Michael addition of the thiol groups. This determination can detect the activity of Lp-PLA₂ in human plasma, for example, determined by the specific inhibition of the Lp-PLA₂ inhibitors.

Thio-PAF determination, as quenched 75 µL determination, is carried out. A compound source plate is prepared by preparing a 1:3 (volume) series of diluents of each compound in pure DMSO on a 96-well microplate. A Rainin multi-channel pipette is used to transfer 3 µL of a compound on the compound source plate into a 96-well microplate where 57 µL of a determination buffer is pre-added, and the compound on the source plate is diluted by 20 folds. The above determination buffer contains 50 mM HEPES (pH 7.4), 150 mM NaCl, and 1 mM CHAPS. A Rainin multi-channel pipette is used to transfer 1 µL of the compound subjected to 20-fold dilution into a 96-well Greiner 655076 (black) microplate where 40 µL of equally-divided and thawed mixed human plasma is pre-added. The plate is oscillated for 20 seconds in an Elisa plate oscillator to be mixed evenly. After 30 minutes of pre-incubation at the room temperature, a Rainin multi-channel pipette is used to add 10 µL of a substrate solution to a 96-well Greiner 655076 (black) microplate, and the substrate solution contains 2.5 mM 2-thio-PAF [from ethanol mother liquor], 32 µM CPM [from DMSO mother liquor] and 3.2 mM NEM (N-ethylmaleimide) [newly prepared in DMSO for each experiment] in a determination buffer composed of 50 mM HEPES (pH 7.4), 150 mM NaCl, and 1 mM CHAPS. After 2 minutes, 25 µL of a 5% trifluoroacetic acid (TFA) aqueous solution is used for quenching the reaction. The plate is centrifuged at 2000 rpm for 1 minute. A Biotek Synergy H1 (H1MF) Elisa reader is used to read the plate at ex:380/em:485. GraphPad Prism 6.0 and Excel are used for IC₅₀ data, curve, and QC analysis.

### Activities determined in examples

| **Example** | **IC₅₀(nM)** | **Example** | **IC₅₀(nM)** | **Example** | **IC₅₀(nM)** |
|---|---|---|---|---|---|
| **1** | 5.0 | **2** | 6.3 | **3** | 7.7 |
| **4** | 11.2 | **5** | 4.5 | **6** | 5.0 |
| **7** | 3.6 | **8** | 8.1 | **9** | 6.3 |
| **10** | 10.8 | **11** | 8.9 | **12** | 8.3 |
| **13** | 8.3 | **14** | 8.8 | **15** | 27.6 |
| **16** | 9.2 | **17** | 21.5 | **18** | 11.0 |
| **19** | 37.0 | **20** | 37.5 | **21** | 24.2 |
| **22** | 120.0 | **23** | 4.9 | **24** | 5.1 |
| **25** | 6.5 | **26** | 6.6 | **27** | 4.6 |
| **28** | 13.3 | **29** | 54.1 | **31** | 97.1 |
| **32** | 45.7 | **33** | 24.7 | **34** | 9.9 |
| **35** | 23.2 | **36** | 17.4 | **37** | 34.0 |
| **38** | 8.1 | **39** | 33.1 | **40** | 10.7 |
| **41** | 234.6 | **42** | 24.4 | **43** | 11.9 |
| **44** | 14.9 | **45** | 66.5 | **46** | 220.5 |
| **47** | 14.6 | **48** | 5.1 | **49** | 1.6 |
| **50** | 12.9 | **51** | 6.6 | **52** | 10.6 |
| **53** | 7.3 | **54** | 5.3 | **55** | 13.7 |
| **56** | 20.1 | **57** | 3.3 | **58** | 3.4 |
| **59** | 5.1 | **60** | 12.2 | **61** | 7.0 |
| **62** | 5.2 | **63** | 5.5 | **64** | 5.8 |

## Claims

1. A compound as represented by formula I, a cis-trans isomer thereof, an enantiomer thereof, a diastereomer thereof, a racemate thereof, a solvate thereof, a hydrate thereof or a pharmaceutically-acceptable salt thereof or a prodrug thereof, 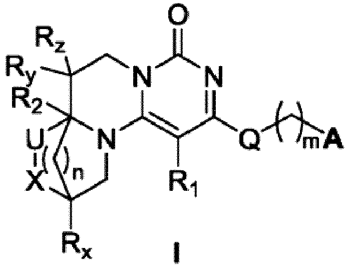 wherein
n is 0, 1 or 2, when n is 0, R₂ is H, methyl or ethyl, and when n is 1 or 2, R₂ is absent;
R₁ is H, halogen, cyano, amino, C₁₋₆ alkyl, C₁₋₆ alkoxy, C₃₋₈ cycloalkyl or 3- to 8-membered heterocyclyl, and R₁ can be substituted by one or more of the following substituents: halogen, cyano, C₁₋₆ alkoxy, C₃₋₈ cycloalkyl, 3- to 8-membered heterocyclyl or 3- to 8-membered heteroaryl;
m is 1 or 2;
Rₓ is H, halogen, hydroxyl, carboxyl, cyano, C₁₋₆ alkyl, C₁₋₆ alkoxy, C₃₋₈ cycloalkyl, 3-to 8-membered heterocyclyl, C₆₋₁₀ aryl, 3- to 8-membered heteroaryl, -C(O)NR_{b}R_{c}, or -S(O)₂NR_{b}R_{c}, and Rₓ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
R_{y} and R_{z} are independently H, halogen, cyano, hydroxyl, amino, -C(O)NR_{b}R_{c}, -S(O)₂NR_{b}R_{c}, C₁₋₆ alkyl, C₃₋₈ cycloalkyl, or 3- to 8-membered heterocyclyl, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl;
or R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ;
Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
Q is -O-, -S- or -NR_{b}-;
X is -O-, -CH₂-, -NR_{c}- or -OCH₂-;
R_{b} is H, C₁₋₆ alkyl, C₃₋₈ cycloalkyl or 3- to 8-membered heterocyclyl;
R_{c} is L, L-C(O)-, L-CH₂- or L-S(O)2-,
wherein L is H, C₁₋₆ alkyl, C₃₋₆ cycloalkyl, 3- to 8-membered heterocyclyl, aryl or 3- to 8-membered heteroaryl, and L can be substituted by one or more of the following groups: halogen, hydroxyl, C₁₋₆ alkoxy, cyano, C₃₋₈ cycloalkyl, 3- to 8-membered heterocyclyl, aryl or 3- to 8-membered heteroaryl;
U is -CH₂-, -C(O)- or absent, and U can be substituted by one or more of the following substituents: halogen, hydroxyl, C₁₋₆ alkyl, C₁₋₆ alkoxy, cyano, C₃₋₈ cycloalkyl, 3- to 8-membered heterocyclyl, aryl or 3- to 8-membered heteroaryl;
A is 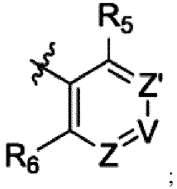
Z is N or CR₃;
Z' is N or CR₄;
R₃, R₄, R₅ and R₆ are independently H, CN, halogen, or C₁₋₃ haloalkyl;
V is N or CR₉, wherein R₉ is H, CN, halogen, C₁₋₃ alkyl, C₁₋₃ haloalkyl or -O-W; and
W is 5- or 6-membered heteroaryl or phenyl, and can be optionally substituted by one or more of the following substituents: halogen, cyano, C₁₋₆ alkyl, C₁₋₃ alkoxy, C₁₋₃ haloalkyl and C₁₋₃ haloalkoxy.

2. A compound as represented by formula II, a cis-trans isomer thereof, an enantiomer thereof, a diastereomer thereof, a racemate thereof, a solvate thereof, a hydrate thereof or a pharmaceutically-acceptable salt thereof or a prodrug thereof, 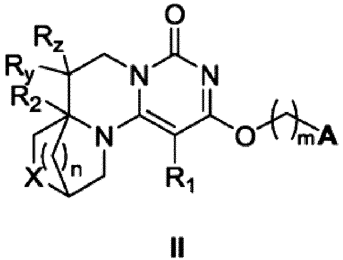 wherein
n is 0, 1 or 2, when n is 0, R₂ is H, methyl or ethyl, and when n is 1 or 2, R₂ is absent;
R₁ is H, halogen, cyano, C₁₋₆ alkyl or C₁₋₆ alkoxy;
m is 1 or 2;
X is -O-, -CH₂- or absent;
R_{y} and R_{z} are independently H, halogen, cyano, hydroxyl, amino, C₁₋₆ alkyl, C₃₋₈ cycloalkyl, or 3- to 8-membered heterocyclyl, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl;
or R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ;
Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
A is 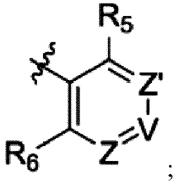
Z is N or CR₃;
Z' is N or CR₄;
R₃, R₄, R₅ and R₆ are independently H, CN, halogen, or C₁₋₃ haloalkyl;
V is N or CR₉, wherein R₉ is H, CN, halogen, C₁₋₃ alkyl, C₁₋₃ haloalkyl or -O-W; and
W is 5- or 6-membered heteroaryl or phenyl, and can be optionally substituted by one or more of the following substituents: halogen, cyano, C₁₋₆ alkyl, C₁₋₃ alkoxy, C₁₋₃ haloalkyl and C₁₋₃ haloalkoxy.

3. The compound according to claim 2, wherein n is 0, R₂ is H or methyl, and m is 1.

4. The compound according to claim 2, wherein n is 1 or 2, R₂ is absent, and m is 1.

5. Compounds as represented by formulas III to VI, cis-trans isomers thereof, enantiomers thereof, diastereomers thereof, racemates thereof, solvates thereof, hydrates thereof or pharmaceutically-acceptable salts thereof or prodrugs thereof, 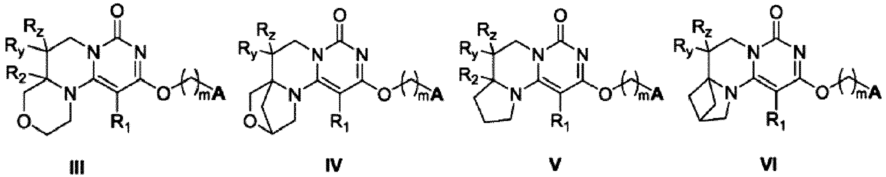 wherein
R₁ is H, halogen, cyano, C₁₋₃ alkyl or C₁₋₆ alkoxy;
R₂ is H or methyl;
m is 1 or 2;
R_{y} and R_{z} are independently H, halogen, cyano, hydroxyl, amino, C₁₋₆ alkyl, C₃₋₈ cycloalkyl, or 3- to 8-membered heterocyclyl, and R_{y} and R_{z} can be both substituted by one or more of the following substituents: halogen, hydroxyl, carboxyl, cyano, C₁₋₆ alkyl, C₆₋₁₀ aryl, and 3- to 8-membered heteroaryl;
or R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ;
Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl;
A is 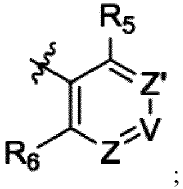
Z is N or CR₃;
Z' is N or CR₄;
R₃, R₄, R₅ and R₆ are independently H, CN, halogen, or C₁₋₃ haloalkyl;
V is N or CR₉, wherein R₉ is H, CN, halogen, C₁₋₃ alkyl, C₁₋₃ haloalkyl or -O-W; and
W is 5- or 6-membered heteroaryl or phenyl, and can be optionally substituted by one or more of the following substituents: halogen, cyano, C₁₋₆ alkyl, C₁₋₃ alkoxy, C₁₋₃ haloalkyl and C₁₋₃ haloalkoxy.

6. The compound or the pharmaceutically-acceptable salt thereof or the prodrug thereof according to any one of claims 1 to 5, wherein m is 1, R_{y} and R_{z} are independently H, halogen, cyano, hydroxyl or amino, and R_{y} and R_{z} can be both substituted by one or more of the following substituents: halogen, hydroxyl, carboxyl, cyano, C₁₋₆ alkyl, C₆₋₁₀ aryl and 3- to 8-membered heteroaryl.

7. The compound or the pharmaceutically-acceptable salt thereof or the prodrug thereof according to any one of claims 1 to 5, wherein m is 1, R_{y} and R_{z} form a 3- to 6-membered saturated ring together with carbon atoms to which they are connected, and the ring is a full-carbon ring or a heterocyclic ring containing one or more atoms selected from N, O and S, and is optionally substituted by one or more Rₘ; wherein
Rₘ is H, halogen, hydroxyl, carboxyl, cyano, C₁₋₆ alkyl, or C₁₋₆ alkoxy, and Rₘ can be substituted by one or more of the following substituents: halogen, hydroxyl, C₁₋₆ alkoxy, cyano, C₃₋₈ cycloalkyl, 3- to 8-membered heterocyclyl, aryl or 3- to 8-membered heteroaryl.

8. The compound or the pharmaceutically-acceptable salt thereof or the prodrug thereof according to any one of claims 1 to 6, wherein R_{y} and R_{z} are independently H or halogen.

9. The compound or the pharmaceutically-acceptable salt thereof or the prodrug thereof according to any one of claims 1 to 8, wherein A is 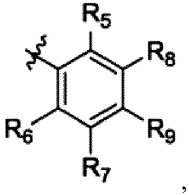 and
R₅, R₆, R₇, R₈ and R₉ are independently H, F or CN.

10. The compound or the pharmaceutically-acceptable salt thereof or the prodrug thereof according to any one of claims 1 to 8, wherein A is 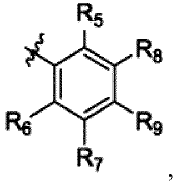
R₅, R₆, R₇ and R₈ are independently H, F or CN;
R₉ is -O-W; and
W is 5- or 6-membered heteroaryl or phenyl, and can be optionally substituted by one or more of the following substituents: C₁₋₃ haloalkyl, C₁₋₃ haloalkoxy, CN, halogen and C₁₋₆ alkyl.

11. The compound or the pharmaceutically-acceptable salt thereof or the prodrug thereof according to any one of claims 1 to 8, wherein A is 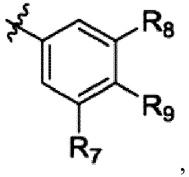
R₇ and R₈ are independently H, F or CN;
R₉ is -O-W; and
W is pyridyl, pyrimidinyl, pyrazolyl, or phenyl, and can be optionally substituted by one or more substituents independently selected from: halogen, CN, CF₃, -OCF₃, CHF₂ and CH₃.

12. The compound or the pharmaceutically-acceptable salt thereof or the prodrug thereof according to any one of claims 1 to 8, wherein A is selected from the following groups:

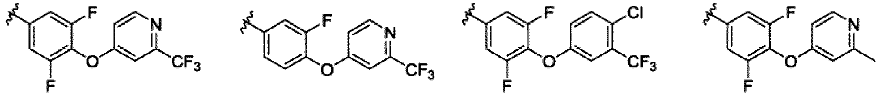

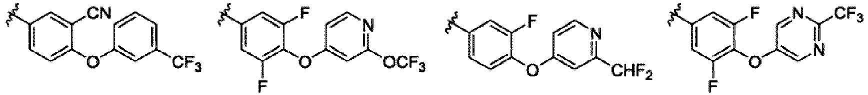

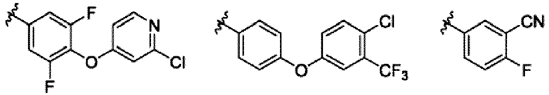

13. The compound or the pharmaceutically-acceptable salt thereof or the prodrug thereof according to any one of claims 1 to 12, having a structure as shown in any one of
| Example | Structure | Example | Structure |
|---|---|---|---|
| 1 | 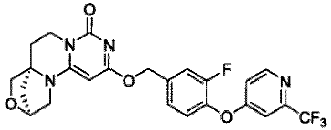 | 2 | 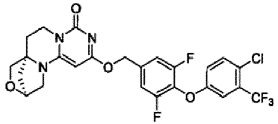 |
| 3 | 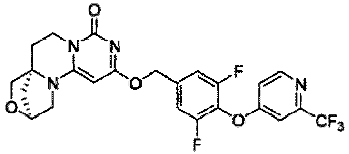 | 4 | 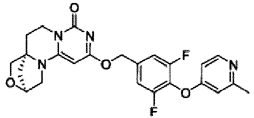 |
| 5 | 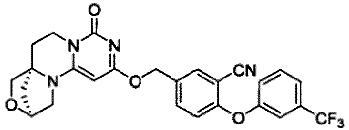 | 6 | 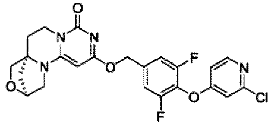 |
| 7 | 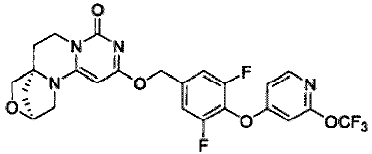 | 8 | 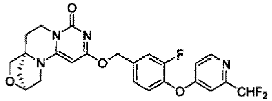 |
| 9 | 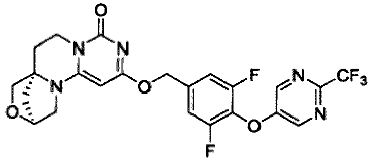 | 10 | 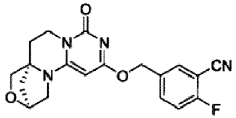 |
| 11 | 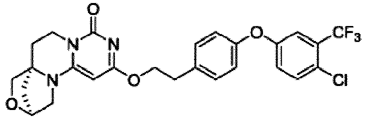 | 12 | 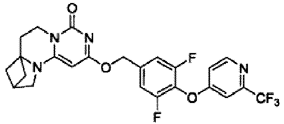 |
| 13 | 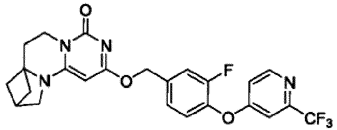 | 14 | 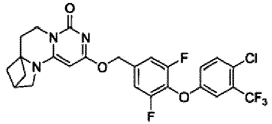 |
| 15 | 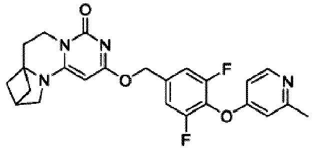 | 16 | 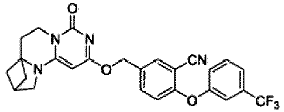 |
| 17 | 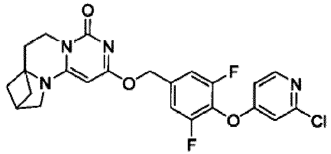 | 18 | 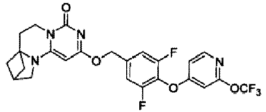 |
| 19 | 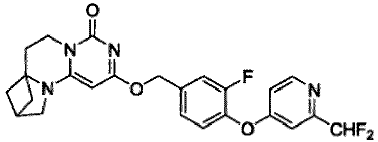 | 20 | 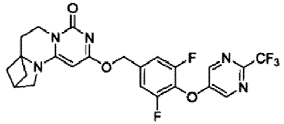 |
| 21 | 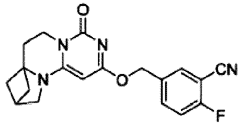 | 22 | 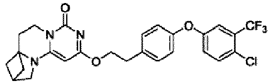 |
| 23 | 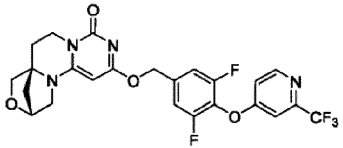 | 24 | 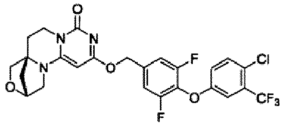 |
| 25 | 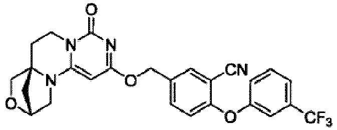 | 26 | 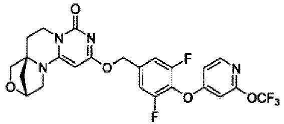 |
| 27 | 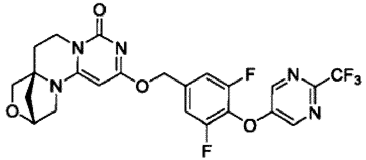 | 28 | 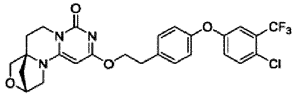 |
| 29 | 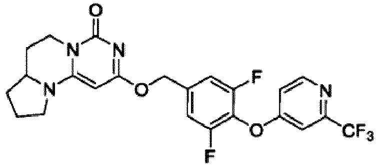 | 30 | 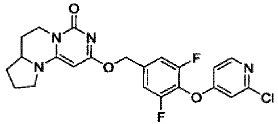 |
| 31 | 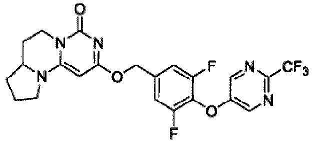 | 32 | 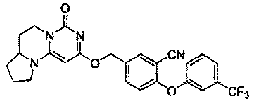 |
| 33 | 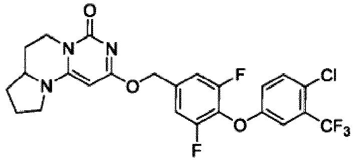 | 34 | 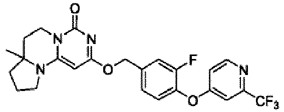 |
| 35 | 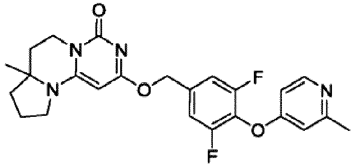 | 36 | 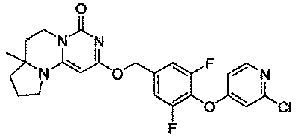 |
| 37 | 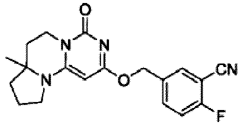 | 38 | 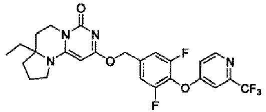 |
| 39 | 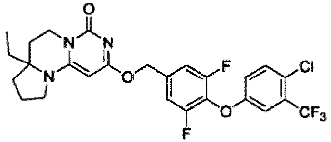 | 40 | 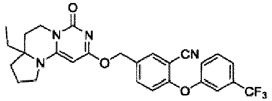 |
| 41 | 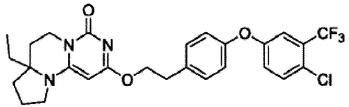 | 42 | 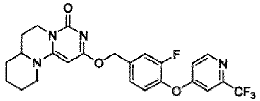 |
| 43 | 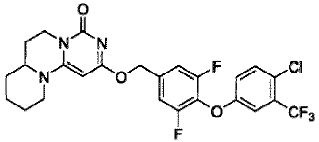 | 44 | 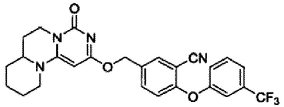 |
| 45 | 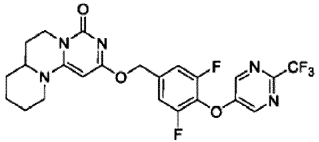 | 46 | 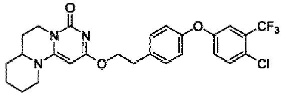 |
| 47 | 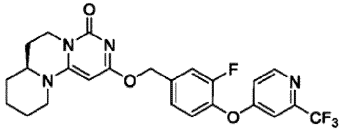 | 48 | 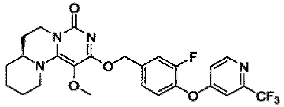 |
| 49 | 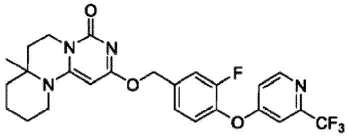 | 50 | 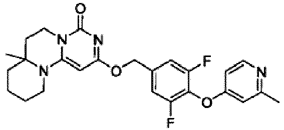 |
| 51 | 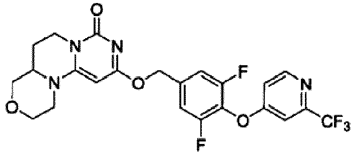 | 52 | 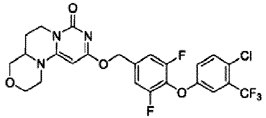 |
| 53 | 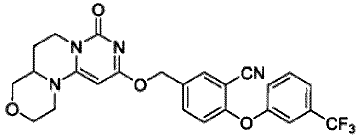 | 54 | 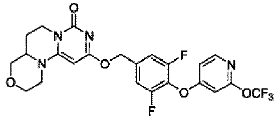 |
| 55 | 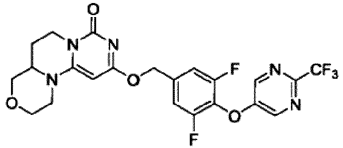 | 56 | 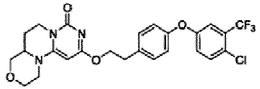 |
| 57 | 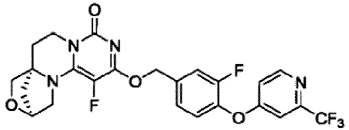 | 58 | 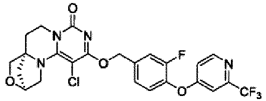 |
| 59 | 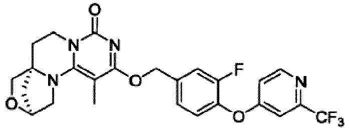 | 60 | 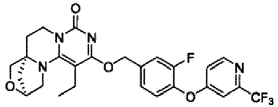 |
| 61 | 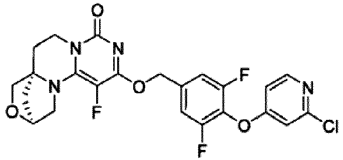 | 62 | 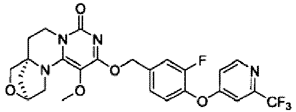 |
| 63 | 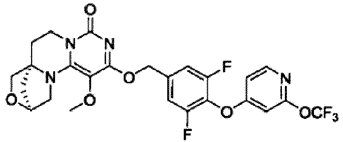 | 64 | 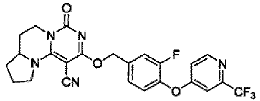 |

14. A composition, comprising the compound or the pharmaceutically-acceptable salt thereof according to any one of claims 1 to 13 and a pharmaceutically-acceptable excipient.

15. Use of the compound according to any one of claims 1 to 13 or the composition according to claim 14 in the manufacture of a medication.

16. Use of the compound according to any one of claims 1 to 13 or the composition according to claim 14 in the manufacture of a medication for treating or preventing the following diseases: diabetic complications, neuroinflammation-related diseases or/and atherosclerosis.

17. A method for treating or preventing diabetic complications, neuroinflammation-related diseases or/and atherosclerosis, comprising administering an effective dosage of the compound according to any one of claims 1 to 13 or the composition according to claim 14 to a patient.

18. The use according to claim 16, wherein the diabetic complications are diabetic retinopathy/diabetic macular edema, diabetic nephropathy, diabetic neuropathy, diabetic peripheral neuropathic pain or/and diabetic foot, and the neuroinflammation-related diseases are Alzheimer's disease, multiple sclerosis, amyotrophic lateral sclerosis or/and Parkinson's disease.

19. The method according to claim 17, wherein the diabetic complications are diabetic retinopathy/diabetic macular edema, diabetic nephropathy, diabetic neuropathy, diabetic peripheral neuropathic pain or/and diabetic foot, and the neuroinflammation-related diseases are Alzheimer's disease, multiple sclerosis, amyotrophic lateral sclerosis or/and Parkinson's disease.
